# EUROPEAN PATENT SPECIFICATION

(11) **EP 1 948 801 B1**
(45) Date of publication and mention of the grant of the patent: **07.11.2012**
(21) Application number: 06829969.2
(22) Date of filing: 09.11.2006
(51) Int. Cl.: C12N 15/60, C12N 15/82, C12N 9/88, A01H 5/00, A01H 5/10

(54) **HERBICIDE-RESISTANT SUNFLOWER PLANTS WITH A NOVEL MUTATION IN THE GENE ENCODING THE LARGE SUBUNIT OF ACETOHYDROXYACID SYNTHASE, ISOLATED POLYNUCLEOTIDES, AND METHODS OF USE**
HERBIZIDRESISTENTE SONNENBLUMENPFLANZEN MIT NEUER MUTATION IN DEM FÜR DIE GROSSE UNTEREINHEIT DER ACETOHYDROXYSÄURE-SYNTHASE CODIERENDEN GEN, ISOLIERTE POLYNUKLEOTIDE UND VERWENDUNGSVERFAHREN
TOURNESOLS RESISTANT AUX HERBICIDES PRESENTANT UNE MUTATION NOVATRICE DANS LE GENE CODANT POUR LA GRANDE SOUS-UNITE DE L ACETOHYDROXYACIDE SYNTHASE, DES POLYNUCLEOTIDES ISOLES, ET PROCEDES D UTILISATION

(30) Priority: 09.11.2005 US 735045 P
(43) Date of publication of application: 30.07.2008
(73) Proprietor: BASF Agrochemical Products, B.V., 6811 CA Arnhem (NL); Advanta Seeds B.V., 4421 AJ Kapelle (NL)
(72) Inventor: LEON, Alberto Javier, BSAS 7600 Mar del Plata (AR); MORATA, Monica Mariel, BSBS 7600 Mar del Plata (AR); OLUNGU, Christine, B-1170 Brussels (BE)
(74) Representative: Reitstötter - Kinzebach
(86) International application number: PCT/EP2006/068321
(87) International publication number: WO 2007/054555

(56) References cited:
- WO-A-03/012115
- WO-A-2005/093093
- US-A1- 2003 180 929
- KOLKMAN J M ET AL: "Acetohydroxyacid synthase mutations conferring resistance to imidazolinone or sulfonylurea herbicides in sunflower" THEORETICAL AND APPLIED GENETICS, SPRINGER, BERLIN, DE, vol. 109, no. 6, October 2004 (2004-10), pages 1147-1159, XP002417954 ISSN: 0040-5752 cited in the application
- CHANG A K ET AL: "Herbicide-resistant forms of Arbidopsis thaliana acetohydroxyacid synthase: characterisation of the catalytic properties and sensitivity to inhibitors of four defined mutants" CA, 1998, XP002432637 cited in the application
- WHITE A D ET AL: "ISOLATION OF ACETOLACTATET SYNTHASE HOMOLOGS IN COMMON SUNFLOWERS" WEED SCIENCE, WEED SCIENCE SOCIETY OF AMERICA, CHAMPAIGN, IL, US, vol. 51, no. 6, November 2003 (2003-11), pages 845-853, XP009058786 ISSN: 0043-1745 cited in the application
- CHONG C-K ET AL: "Amino acid residues conferring herbicide tolerance in tobacco acetolactate synthase" BIOCHEMICAL AND BIOPHYSICAL RESEARCH COMMUNICATIONS, ACADEMIC PRESS INC. ORLANDO, FL, US, vol. 279, no. 2, 20 December 2000 (2000-12-20), pages 462-467, XP002224077 ISSN: 0006-291X
- TAN S ET AL: "IMIDAZOLINONE-TOLERANT CROPS: HISTORY, CURRENT STATUS AND FUTURE" PEST MANAGEMENT SCIENCE, WILEY & SONS, BOGNOR REGIS, GB, vol. 61, no. 3, 31 December 2004 (2004-12-31), pages 246-257, XP009058795 ISSN: 1526-498X

## Description

### FIELD OF THE INVENTION

This invention relates to the field of agricultural biotechnology, particularly to herbicide-resistant sunflower plants and novel polynucleotide sequences that encode wild-type and herbicide-resistant sunflower acetohydroxyacid synthase large subunit proteins.

### BACKGROUND OF THE INVENTION

Acetohydroxyacid synthase (AHAS; EC 4.1.3.18, also known as acetolactate synthase or ALS), is the first enzyme that catalyzes the biochemical synthesis of the branched chain amino acids valine, leucine and isoleucine (Singh (1999) "Biosynthesis of valine, leucine and isoleucine," in Plant Amino Acid, Singh, B.K., ed., Marcel Dekker Inc. New York, New York, pp. 227-247). AHAS is the site of action of five structurally diverse herbicide families including the sulfonylureas (LaRossa and Falco (1984) Trends Biotechnol. 2:158-161), the imidazolinones (Shaner et al. (1984) Plant Physiol. 76:545-546), the triazolopyrimidines (Subramanian and Gerwick (1989) "Inhibition of acetolactate synthase by triazolopyrimidines," in Biocatalysis in Agricultural Biotechnology, Whitaker, J.R. and Sonnet, P.E.. eds., ACS Symposium Series, American Chemical Society, Washington, D.C., pp. 277-288), and the pyrimidyloxybenzoates (Subramanian et al. (1990) Plant Physiol. 94: 239-244). Imidazolinone and sulfonylurea herbicides are widely used in modem agriculture due to their effectiveness at very low application rates and relative non-toxicity in animals. By inhibiting AHAS activity, these families of herbicides prevent further growth and development of susceptible plants including many weed species. Several examples of commercially available imidazolinone herbicides are PURSUIT® (imazethapyr), SCEPTER® (imazaquin) and ARSENAL® (imazapyr). Examples of sulfonylurea herbicides are chlorsulfuron, metsulfuron methyl, sulfometuron methyl, chlorimuron ethyl, thifensulfuron methyl, tribenuron methyl, bensulfuron methyl, nicosulfuron, ethametsulfuron methyl, rimsulfuron, triflusulfuron methyl, triasulfuron, primisulfuron methyl, cinosulfuron, amidosulfiuon, fluzasulfuron, imazosulfuron, pyrazosulfuron ethyl and halosulfuron.

Due to their high effectiveness and low-toxicity, imidazolinone herbicides are favored for application by spraying over the top of a wide area of vegetation. The ability to spray a herbicide over the top of a wide range of vegetation decreases the costs associated with plant establishment and maintenance, and decreases the need for site preparation prior to use of such chemicals. Spraying over the top of a desired tolerant species also results in the ability to achieve maximum yield potential of the desired species due to the absence of competitive species. However, the ability to use such spray-over techniques is dependent upon the presence of imidazolinone-resistant species of the desired vegetation in the spray over area.

Among the major agricultural crops, some leguminous species such as soybean are naturally resistant to imidazolinone herbicides due to their ability to rapidly metabolize the herbicide compounds (Shaner and Robinson (1985) Weed Sci. 33:469-471). Other crops such as corn (Newhouse et al. (1992) Plant Physiol. 100:882-886) and rice (Barrett et al. (1989) Crop Safeners for Herbicides, Academic Press, New York, pp. 195-220) are somewhat susceptible to imidazolinone herbicides. The differential sensitivity to the imidazolinone herbicides is dependent on the chemical nature of the particular herbicide and differential metabolism of the compound from a toxic to a non-toxic form in each plant *(*Shaner et al. (1984) Plant Physiol. 76:545-546; Brown et al., (1987) Pestic. Biochem. Physiol. 27:24-29). Other plant physiological differences such as absorption and translocation also play an important role in sensitivity (Shaner and Robinson (1985) Weed Sci. 33:469-471).

Plants resistant to imidazolinones, sulfonylureas, triazolopyrimidines, and pyrimidyloxybenzoates have been successfully produced using seed, microspore, pollen, and callus mutagenesis in *Zea mays, Arabidopsis thaliana, Brassica napus* (*i*.*e*., canola) *Glycine max, Nicotiana tabacum,* sugarbeet (*Beta vulgaris)* and *Oryza sativa* (Sebastian et al. (1989) Crop Sci. 29:1403-1408; Swanson et al., 1989 Theor. Appl. Genet. 78:525-530; Newhouse et al. (1991) Theor. Appl. Genet. 83:65-70; Sathasivan et al. (1991) Plant Physiol. 97:1044-1050; Mourand et al. (1993) J. Heredity 84:91-96; Wright and Penner (1998) Theor. Appl. Genet. 96:612-620; U.S. Patent No. 5,545,822). In all cases, a single, partially dominant nuclear gene conferred resistance. Four imidazolinone resistant wheat plants were also previously isolated following seed mutagenesis of *Triticum aestivum* L. cv. Fidel (Newhouse et al. (1992) Plant Physiol. 100:882-886). Inheritance studies confirmed that a single, partially dominant gene conferred resistance. Based on allelic studies, the authors concluded that the mutations in the four identified lines were located at the same locus. One of the Fidel cultivar resistance genes was designated FS-4 (Newhouse et al. (1992) Plant Physiol. 100:882-886).

Naturally occurring plant populations that were discovered to be resistant to imidazolinone and/or sulfonylurea herbicides have also been used to develop herbicide-resistant sunflower breeding lines. Recently, two sunflower lines that are resistant to a sulfonylurea herbicide were developed using germplasm originating from a wild population of common sunflower *(Helianthus annuus)* as the source of the herbicide-resistance trait (Miller and Al-Khatib (2004) Crop Sci. 44:1037-1038). Previously, White et al. ((2002) Weed Sci. 50:432-437) had reported that individuals from a wild population of common sunflower from South Dakota, U.S.A. were cross-resistant to an imidazolinone and a sulfonylurea herbicide. Analysis of a portion of the coding region of the acetohydroxyacid synthase large subunit (AHASL) genes of individuals from this population revealed a point mutation that results in an Ala-to-Val amino acid substitution in the sunflower AHASL protein that corresponds to Ala₂₀₅ in the wild-type *Arabidopsis thaliana* AHASL protein (White et al. (2003) Weed Sci. 51:845-853).

Computer-based modeling of the three dimensional conformation of the AHAS-inhibitor complex predicts several amino acids in the proposed inhibitor binding pocket as sites where induced mutations would likely confer selective resistance to imidazolinones *(*Ott et al. (1996) J. Mol. Biol. 263:359-368). Wheat plants produced with some of these rationally designed mutations in the proposed binding sites of the AHAS enzyme have in fact exhibited specific resistance to a single class of herbicides *(*Ott et al. (1996) J. Mol. Biol. 263:359-368).

Plant resistance to imidazolinone herbicides has also been reported in a number of patents. U.S. Patent Nos. 4,761,373, 5,331,107, 5,304,732, 6,211,438, 6,211,439 and 6,222,100 generally describe the use of an altered AHAS gene to elicit herbicide resistance in plants, and specifically discloses certain imidazolinone resistant corn lines. U.S. Patent No. 5,013,659 discloses plants exhibiting herbicide resistance due to mutations in at least one amino acid in one or more conserved regions. The mutations described therein encode either cross-resistance for imidazolinones and sulfonylureas or sulfonylurea-specific resistance, but imidazolinone-specific resistance is not described. U.S. Patent No. 5,731,180 and U.S. Patent No. 5,767,361 discuss an isolated gene having a single amino acid substitution in a wild-type monocot AHAS amino acid sequence that results in imidazolinone-specific resistance. In addition, rice plants that are resistant to herbicides that interfere with AHAS have been developed by mutation breeding and also by the selection of herbicide-resistant plants from a pool of rice plants produced by anther culture. *See,* U.S. Patent Nos. 5,545,822, 5,736,629, 5,773,703, 5,773,704, 5,952,553 and 6,274,796.

In plants, as in all other organisms examined, the AHAS enzyme is comprised of two subunits: a large subunit (catalytic role) and a small subunit (regulatory role) (Duggleby and Pang (2000) J. Biochem. Mol. Biol. 33:1-36). The AHAS large subunit (also referred to herein as AHASL) may be encoded by a single gene as in the case of *Arabidopsis,* rice, and sugar beet or by multiple gene family members as in maize, canola, and cotton. Specific, single-nucleotide substitutions in the large subunit confer upon the enzyme a degree of insensitivity to one or more classes of herbicides (Chang and Duggleby (1998) Biochem J. 333:765-777).

For example, bread wheat, *Triticum aestivum* L., contains three homoeologous acetohydroxyacid synthase large subunit genes. Each of the genes exhibit significant expression based on herbicide response and biochemical data from mutants in each of the three genes (Ascenzi et al. (2003) International Society of Plant Molecular Biologists Congress, Barcelona, Spain, Ref. No. S10-17). The coding sequences of all three genes share extensive homology at the nucleotide level (WO 03/014357). Through sequencing the *AHASL* genes from several varieties of *Triticum aestivum,* the molecular basis of herbicide tolerance in most IMI-tolerant (imidazolinone-tolerant) lines was found to be the mutation S653(*At*)N, indicating a serine to asparagine substitution at a position equivalent to the serine at amino acid 653 in *Arabidopsis thaliana* (WO 03/01436; WO 03/014357). This mutation is due to a single nucleotide polymorphism (SNP) in the DNA sequence encoding the AHASL protein.

Multiple *AHASL* genes are also know to occur in dicotyledonous plants species. Recently, Kolkman et al. ((2004) Theor. Appl. Genet. 109: 1147-1159) reported the identification, cloning, and sequencing for three *AHASL* genes *(AHASL1, AHASL2,* and *AHASL3)* from herbicide-resistant and wild type genotypes of sunflower *(Helianthus annuus* L.). Kolkman *et al.* reported that the herbicide-resistance was due either to the Pro197Leu (using the *Arabidopsis* AHASL amino acid position nomenclature) substitution or the Ala205Val substitution in the AHASL1 protein and that each of these substitutions provided resistance to both imidazolinone and sulfonylurea herbicides.

Given their high effectiveness and low-toxicity, imidazolinone herbicides are favored for agricultural use. However, the ability to use imidazolinone herbicides in a particular crop production system depends upon the availability of imidazolinone-resistant varieties of the crop plant of interest. To produce such imidazolinone-resistant varieties, plant breeders need to develop breeding lines with the imidazolinone-resistance trait. Thus, additional imidazolinone-resistant breeding lines and varieties of crop plants, as well as methods and compositions for the production and use of imidazolinone-resistant breeding lines and varieties, are needed.

### SUMMARY OF THE INVENTION

The present invention provides sunflower plants having increased resistance to imidazolinone herbicides, when compared to a wild-type sunflower plant. The herbicide-resistant sunflower plants of the invention comprise at least one copy of a gene or polynucleotide that encodes a herbicide-resistant acetohydroxyacid synthase large subunit (AHASL). Such a herbicide-resistant AHASL protein comprises a isoleucine at amino acid position 188 or equivalent position. The herbicide-resistant sunflower plant of the invention can contain one, two, three, four, five, six, or more copies of a gene or polynucleotide encoding a herbicide-resistant AHASL protein of the invention. The sunflower plants of the invention also include seeds and progeny plants that comprise at least one copy of a gene or polynucleotide encoding a herbicide-resistant AHASL protein of the invention.

Herbicide-resistant sunflower plants that are from the sunflower line that has been designated as MUT9 are described herein. A sample of seeds of the MUT9 line has been deposited with the American Type Culture Collection (ATCC) as ATCC Patent Deposit No. PTA-6325. Such MUT9 sunflower plants comprise in their genomes an AHASL1 gene that comprises the nucleotide sequence set forth in SEQ ID NO: 1 and that encodes the AHASL1 protein comprising, the amino acid sequence set forth in SEQ ID NO: 2. When compared to the amino acid sequence of the AHASL1 protein (SEQ ID NO: 4) that is encoded by an AHASL1 gene (SEQ ID NO: 3) from a wild-type sunflower plant, the amino acid sequence set forth in SEQ ID NO: 2 has a single amino acid difference from the wild-type amino acid sequence. In the amino acid sequence set forth in SEQ ID NO: 2, there is an isoleucine at amino acid position 102. This position corresponds to position 188 in the full-length sunflower AHASL1 protein encoded by the nucleotide sequence set froth in SEQ ID NO: 24 (Acession No. AY541451). In the wild-type AHASL1 amino acid sequence (SEQ ID NO: 4), this equivalent amino acid position has a threonine. Unless otherwise indicated, the amino acid positions referred to herein for sunflower AHASL proteins correspond to the amino acid positions of the full-length amino acid sequence set forth in SEQ ID NO: 24.

The present invention further provides isolated polynucleotides and isolated polypeptides for sunflower *(Helianthus annuus)* AHASL proteins. The polynucleotides of the invention encompass nucleotide sequences that encode herbicide-resistant and wild-type AHASL proteins, including, but not limited to, the proteins encoded by the sunflower *AHASL1, AHASL2,* and *AHASL3* genes. The herbicide-resistant sunflower AHASL proteins of the invention are imidazolinone-resistant AHASL proteins that comprise an amino acid other than threonine at position 188 of a full-length sunflower AHASL1 protein or equivalent position. In the herbicide-resistant sunflower AHASL proteins of the invention the amino acid at position 188 or equivalent position is an isoleucine. The polynucleotides of the invention encompass the nucleotide sequence set forth in SEQ ID NO: 1, nucleotide sequences encoding the amino acid sequences set forth in SEQ ID NO: 2, and fragments and variants of said nucleotide sequences that encode proteins comprising AHAS activity.

The present invention provides expression cassettes for expressing the polynucleotides of the invention in plants, plant cells, and other, non-human host cells. The expression cassettes comprise a promoter expressible in the plant, plant cell, or other host cells of interest operably linked to a polynucleotide of the invention that encodes an imidazolinone herbicide-resistant AHASL protein. If necessary for targeting expression to the chloroplast, the expression cassette can also comprise an operably linked chloroplast-targeting sequence that encodes of a chloroplast transit peptide to direct an expressed AHASL protein to the chloroplast. The expression cassettes of the invention find use in a method for enhancing the herbicide tolerance of a plant and a host cell. The method involves transforming the plant or host cell with an expression cassette of the invention, wherein the expression cassette comprises a promoter that is expressible in the plant or host cell of interest and the promoter is operably linked to a polynucleotide of the invention that encodes an herbicide-resistant AHASL protein of the invention. The method further comprises regenerating a transformed plant from the transformed plant cell.

The present invention provides a method for increasing AHAS activity in a plant comprising transforming a plant cell with a polynucleotide construct comprising a nucleotide sequence operably linked to a promoter that drives expression in a plant cell and regenerating a transformed plant from the transformed plant cell. The nucleotide sequence is selected from those nucleotide sequences that encode the imidazolinone herbicide-resistant AHASL proteins of the invention, particularly the nucleotide sequence set forth in SEQ ID NO: 1, and nucleotide sequences encoding the amino acid sequence set forth in SEQ ID NO: 2, and fragments and variants thereof. A plant produced by this method comprises increased AHAS activity or increased herbicide-resistant AHAS activity, when compared to an untransformed plant.

The present invention provides a method for producing an imidazolinone herbicide-resistant plant comprising transforming a plant cell with a polynucleotide construct comprising a nucleotide sequence operably linked to a promoter that drives expression in a plant cell and regenerating the imidazolinone herbicide-resistant plant from said transformed plant cell. The nucleotide sequence is selected from those nucleotide sequences that encode the herbicide-resistant AHASL proteins of the invention, particularly the nucleotide sequence set forth in SEQ ID NO: 1, the nucleotide sequence encoding the amino acid sequence set forth in SEQ ID NO: 2, and fragments and variants thereof. A herbicide-resistant plant produced by this method comprises enhanced resistance to at least one imidazolinone herbicide when compared to an untransformed plant.

The present invention provides a method for enhancing herbicide-tolerance in a herbicide-tolerant plant. The method finds use in enhancing the resistance of a plant that already is resistant to a level of a herbicide that would kill or significantly injure a wild-type plant. Such a herbicide-tolerant plant can be a herbicide-tolerant plant that has been genetically engineered for herbicide-tolerance or a herbicide-tolerant plant that was developed by means that do not involve recombinant DNA such as, for example, the MUT9 sunflower plants described herein. The method comprises transforming a herbicide-tolerant plant with a polynucleotide construct comprising a nucleotide sequence operably linked to a promoter that drives expression in a plant cell and regenerating a transformed plant from the transformed plant cell. The nucleotide sequence is selected from those nucleotide sequences that encode the herbicide-resistant AHASL proteins of the invention, particularly the nucleotide sequence set forth in SEQ ID NO: 1, nucleotide sequences encoding the amino acid sequence set forth in SEQ ID NO: 2, and fragments and variants thereof.

The present invention provides transformation vectors comprising a selectable marker gene of the invention. The selectable marker gene comprises a promoter that drives expression in a host cell operably linked to a polynucleotide comprising a nucleotide sequence that encodes an herbicide-resistant AHASL protein of the invention. The transformation vector can additionally comprise a gene of interest to be expressed in the host cell and can also, if desired, include a chloroplast-targeting sequence that is operably linked to the polynucleotide of the invention.

The present invention further provides methods for using the transformation vectors of the invention to select for cells transformed with the gene of interest. Such methods involve the transformation of a host cell with the transformation vector, exposing the cell to a level of an imidazolinone herbicide that would kill or inhibit the growth of a non-transformed host cell, and identifying the transformed host cell by its ability to grow in the presence of the herbicide. In one embodiment of the invention, the host cell is a plant cell and the selectable marker gene comprises a promoter that drives expression in a plant cell.

The present invention provides a method for controlling weeds in the vicinity of the herbicide-resistant plants of the invention, the herbicide-resistant sunflower plants described above and plants transformed with the herbicide-resistant AHASL polynucleotides of the invention. Such transformed plants comprise in their genomes at least one expression cassette comprising a promoter that drives gene expression in a plant cell, wherein the promoter is operably linked to an AHASL polynucleotide of the invention. The method comprises applying an effective amount of an imidazolinone herbicide to the weeds and to the herbicide-resistant plant, wherein the herbicide-resistant plant has increased resistance to at least one imidazolinone herbicide when compared to a wild-type or untransformed plant.

The plants of the present invention can be transgenic or non-transgenic. An example of a non-transgenic sunflower plant having increased resistance to imidazolinone and/or sulfonylurea herbicides includes the sunflower plant (MUT9) having ATCC Patent Deposit No. PTA-6325; or mutant, recombinant, or a genetically engineered derivative of the plant having ATCC Patent Deposit No. PTA-6325; or of any progeny of the plant having ATCC Patent Deposit No. PTA-6325; or a plant that is a progeny of any of these plants; or a plant that comprises the herbicide resistance characteristics of the plant having ATCC Patent Deposit No. PTA-6325.

The present invention also provides plants, plant organs, plant tissues, plant cells, seeds, and non-human host cells that are transformed with the at least one polynucleotide, expression cassette, or transformation vector of the invention. Such transformed plants, plant organs, plant tissues, plant cells, seeds, and non-human host cells have enhanced tolerance or resistance to at least one herbicide, at levels of the herbicide that kill or inhibit the growth of an untransformed plant, plant tissue, plant cell, or non-human host cell, respectively. Preferably, the transformed plants, plant tissues, plant cells, and seeds of the invention are *Arabidopsis thaliana* and crop plants.

The present invention further provides isolated polypeptides comprising imidazolinone-resistant sunflower AHASL proteins. The isolated polypeptides comprise the amino acid sequence set forth in SEQ ID NO: 2, the amino acid sequence encoded by nucleotide sequence set forth in SEQ ID NO: 1, and fragments and variants of said amino acid sequences that encode proteins comprising AHAS activity.

### BRIEF DESCRIPTION THE DRAWINGS

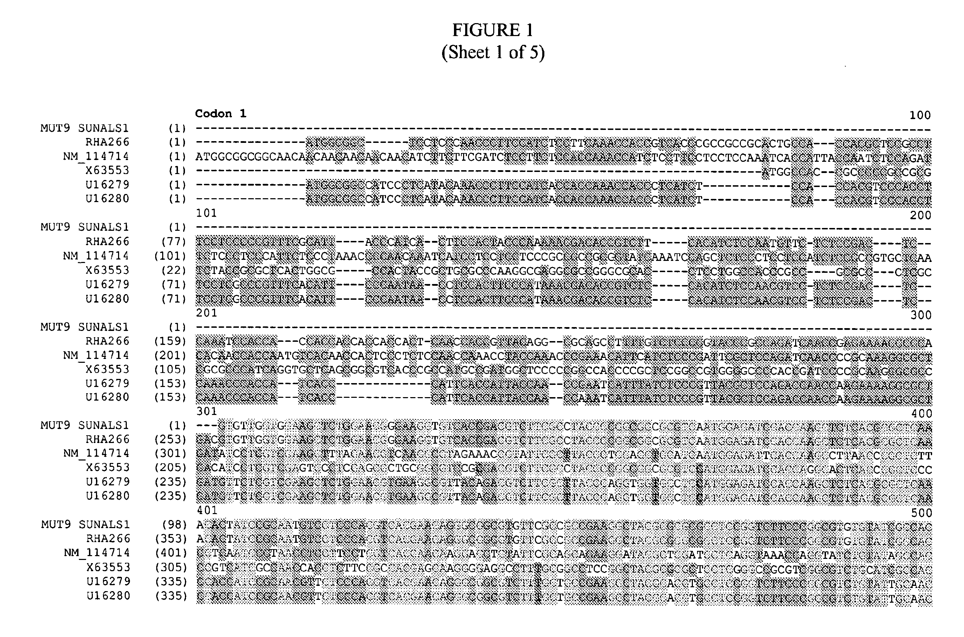

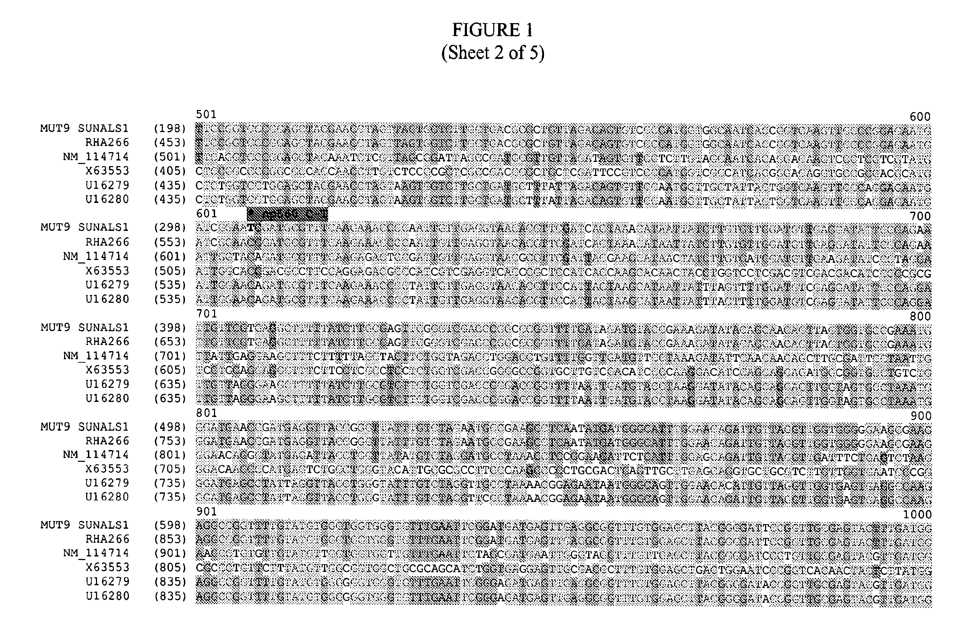

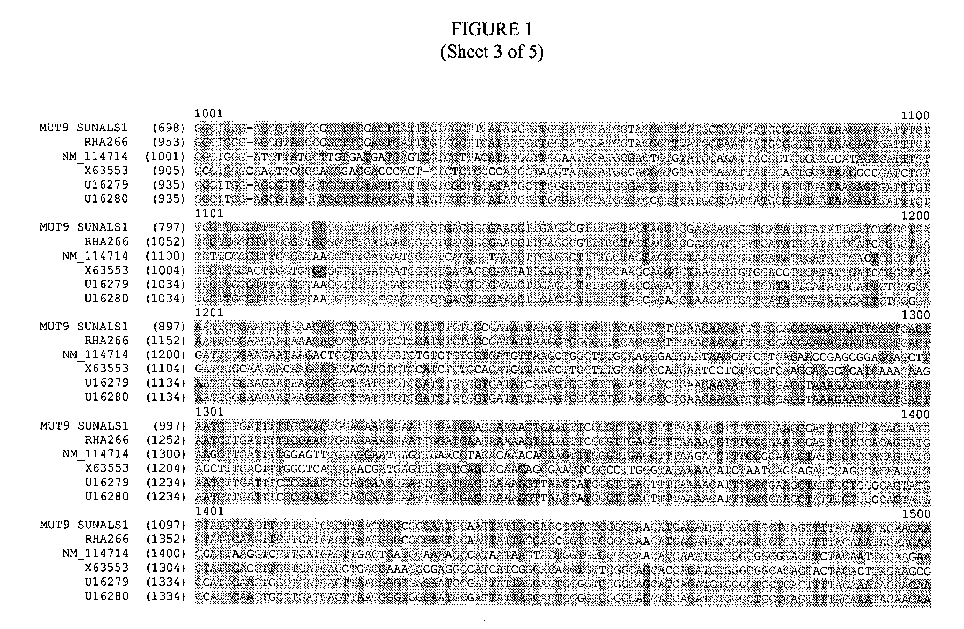

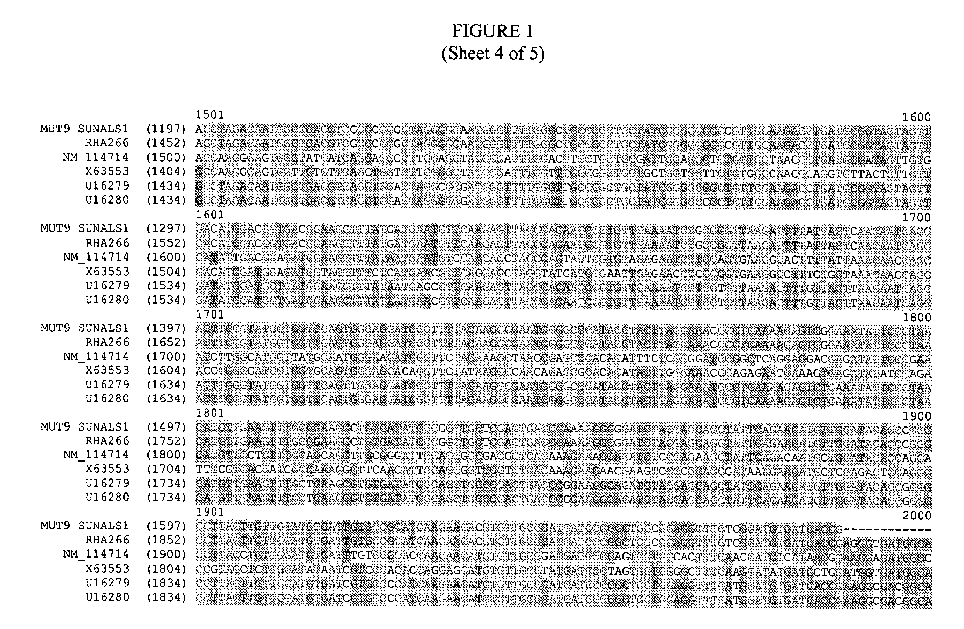

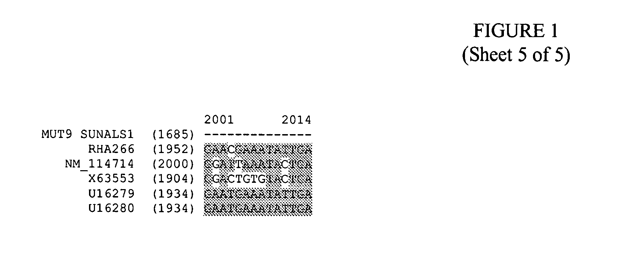

Figure 1 is a nucleotide sequence alignment of the coding sequences of the herbicide-resistant sunflower *AHASL1* gene (MUT9 SUNALS 1; SEQ ID NO: 1), the wild-type sunflower *AHASL1* gene (RHA266; SEQ ID NO: 3), an *Arabidopsis thaliana AHASL* gene (GenBank Accession No. NM_114714; SEQ ID NO: 9), a *Zea mays* AHASL gene (GenBank Accession No. X63553; SEQ ID NO: 25) the herbicide-resistant *AHASL* gene from *Xanthium sp.* (GenBank Accession No. U16279; SEQ ID NO: 5), and the wild-type *AHASL* gene from *Xanthium sp.* (GenBank Accession No. U16280; SEQ ID NO: 7). The site of the mutation in MUT SUNALS1 (SEQ ID NO: 1) is indicated by an asterisk. The mutation is a C-to-T transition at nucleotide position 305 of SEQ ID NO: 1.

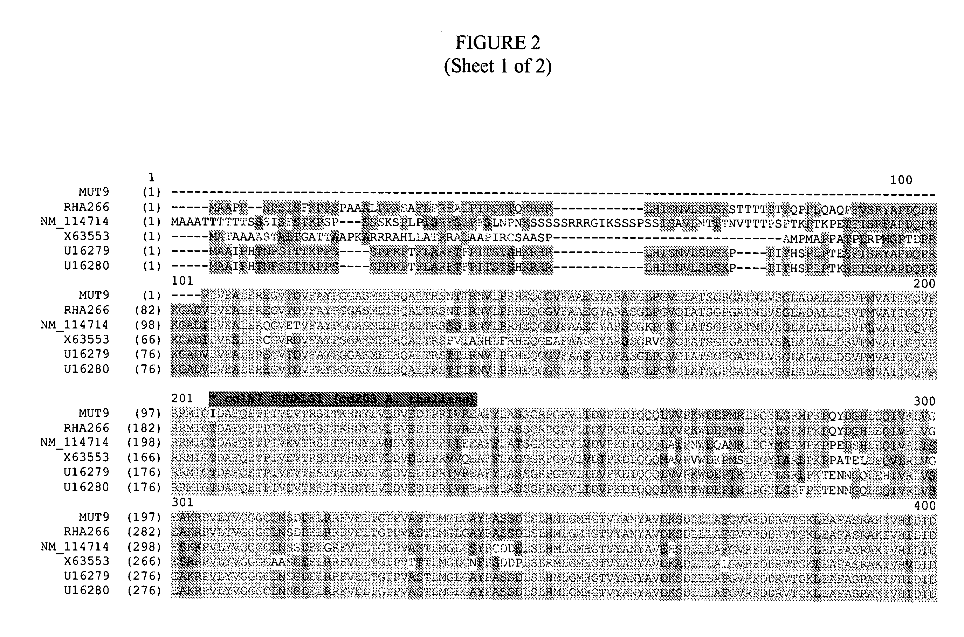

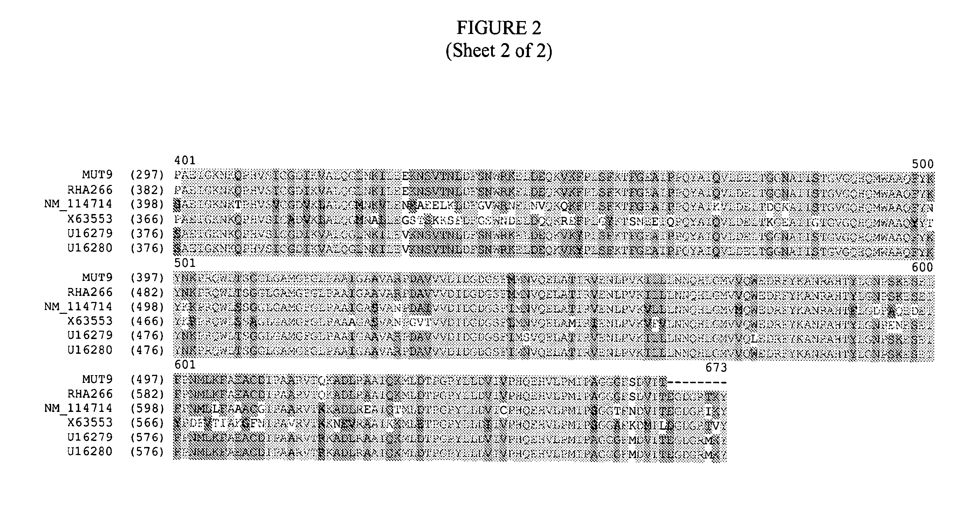

Figure 2 is an amino acid sequence alignment of the herbicide-resistant sunflower AHASL1 protein (MUT9 SUNALS1; SEQ ID NO: 2), the wild-type sunflower AHASL1 protein (RHA266; SEQ ID NO: 4), an *Arabidopsis thaliana* AHASL protein (GenBank Accession No. NM_114714; SEQ ID NO: 10), a *Zea mays* AHASL protein (GenBank Accession No. X63553; SEQ ID NO: 26) the herbicide-resistant AHASL protein from *Xanthiun sp.* (GenBank Accession No. U16279; SEQ ID NO: 6), and the wild-type AHASL protein from *Xanthium sp.* (GenBank Accession No. U16280; SEQ ID NO: 8). The asterisk indicates the site of the single amino acid substitution (Thr-to-Ile) found in the herbicide-resistant sunflower AHASL1 protein. This site of the substitution corresponds to amino acid position 102 in the partial-length AHASL1 amino acid sequence set forth in SEQ ID NO: 2. The equivalent position of this substitution in the full-length sunflower AHASL1 amino acid sequence set forth in SEQ ID NO: 24 is 188.

### SEQUENCE LISTING

The nucleotide and amino acid sequences listed in the accompanying sequence listing are shown using standard letter abbreviations for nucleotide bases, and three-letter code for amino acids. The nucleotide sequences follow the standard convention of beginning at the 5' end of the sequence and proceeding forward (i.e., from left to right in each line) to the 3' end. Only one strand of each nucleic acid sequence is shown, but the complementary strand is understood to be included by any reference to the displayed strand. The amino acid sequences follow the standard convention of beginning at the amino terminus of the sequence and proceeding forward (i.e., from left to right in each line) to the carboxy terminus.
SEQ ID NO:1 sets forth the partial length nucleotide sequence encoding a herbicide-resistant AHASL1 protein from the sunflower line MUT9.
SEQ ID NO: 2 sets forth the partial length amino acid sequence of the herbicide-resistant AHASL1 protein encoded by the nucleotide sequence set forth in SEQ ID NO: 1.
SEQ ID NO: 3 sets forth the partial length nucleotide sequence encoding the wild-type AHASL1 protein from sunflower line RHA266.
SEQ ID NO: 4 sets forth the partial length amino acid sequence of the wild-type AHASL1 protein encoded by the nucleotide sequence set forth SEQ ID NO: 3.
SEQ ID NO: 5 is the nucleotide sequence of GenBank Accession No. U16279.
SEQ ID NO: 6 is the amino acid sequence encoded by the nucleotide sequence of GenBank Accession No. U16279.
SEQ ID NO: 7 is the nucleotide sequence of GenBank Accession No. U16280.
SEQ ID NO: 8 is the amino acid sequence encoded by the nucleotide sequence of GenBank Accession No. U16280.
SEQ ID NO: 9 is the nucleotide sequence of GenBank Accession No. NM_114714.
SEQ ID NO: 10 is the amino acid sequence encoded by the nucleotide sequence of GenBank Accession No. NM_114714.
SEQ ID NO: 11 sets forth the nucleotide sequence of the C2359-FI primer that is described in Example 2.
SEQ ID NO: 12 sets forth the nucleotide sequence of the ALSRI primer that is described in Example 2.
SEQ ID NO: 13 sets forth the nucleotide sequence of the SUNALS1-F1 primer that is described in Example 2.
SEQ ID NO: 14 sets forth the nucleotide sequence of the ALS1-8R primer that is described in Example 2.
SEQ ID NO: 15 sets forth the nucleotide sequence of the ALS-2R primer that is described in Example 2.
SEQ ID NO: 16 sets forth the nucleotide sequence of the ALS-2F primer that is described in Example 2.
SEQ ID NO: 17 sets forth the nucleotide sequence of the ALS-3R primer that is described in Example 2.
SEQ ID NO: 18 sets forth the nucleotide sequence of the ALS-3F primer that is described in Example 2.
SEQ ID NO: 19 sets forth the nucleotide sequence of the ALS-4F primer that is described in Example 2.
SEQ ID NO: 20 sets forth the nucleotide sequence of the ALS-6R primer that is described in Example 2.
SEQ ID NO: 21 sets forth the nucleotide sequence of the ALS-7F primer that is described in Example 2.
SEQ ID NO: 22 sets forth the nucleotide sequence of the ALS-8F primer that is described in Example 2.
SEQ ID NO: 23 is the nucleotide sequence of GenBank Accession No. AY541451.
SEQ ID NO: 24 is the amino acid sequence encoded by the nucleotide sequence of GenBank Accession No. AY41451.
SEQ ID NO: 25 is the nucleotide sequence of GenBank Accession No. X63553.
SEQ ID NO: 26 is the amino acid sequence encoded by the nucleotide sequence of GenBank Accession No. X63553.

### DETAILED DESCRIPTION OF THE INVENTION

The present invention relates to sunflower plants having increased resistance to herbicides when compared to a wild-type sunflower plant. Herbicide-resistant sunflower plants were produced as described hereinbelow by exposing wild-type (with respect to herbicide resistance) sunflower plants to a mutagen, allowing the plants to mature and reproduce, and selecting progeny plants that displayed enhanced resistance to an imidazolinone herbicide, relative to the resistance of a wild-type sunflower plant. A herbicide-resistant sunflower line that is referred to herein as MUT9 is described herein.

From the MUT9 herbicide-resistant sunflower plants and wild-type sunflower plants; the coding region of an acetohydroxyacid synthase large subunit gene (designated as AHASL1) was isolated by polymerase chain reaction (PCR) amplification and sequenced. By comparing the polynucleotide sequences of the herbicide-resistant and wild-type sunflower plants, it was discovered that the coding region of the AHASL1 polynucleotide sequence from the herbicide-resistant sunflower plant differed from the AHASL1 polynucleotide sequence of the wild type plant by a single nucleotide, a C-to-T transition at nucleotide 305 (SEQ ID NO: 1). This C-to-T transition in the AHASL1 polynucleotide sequence results in a threonine-to-isoleucine substitution at amino acid 102 in a conserved region of the predicted amino acid sequence of the herbicide-resistant sunflower AHASL1 protein (SEQ ID NO: 2), relative to the equivalent amino acid position of the wild-type AHASL1 protein from sunflower line RHA266 (amino acid 108 of SEQ ID NO: 4).

Because the nucleotide sequence set forth in SEQ ID NO: 1 does not correspond to a full-length coding region of an AHASL protein, the amino acid sequence encoded thereby that is set forth in SEQ ID NO: 2 is also less than full-length. To facilitate comparison with other sunflower ASHAL amino acid sequences, the amino acid positions of sunflower AHASL proteins referred to herein, unless otherwise indicated or apparent for the context in which such positions appear, correspond to the amino acid positions of the full-length amino acid sequence of the sunflower AHASL1 protein encoded by the nucleotide sequence having GenBank Accession No. AY541451 (SEQ ID NO: 24). Accordingly, the threonine-to-isoleucine substitution at amino acid position 102 of SEQ ID NO: 2 corresponds to amino acid position 188 in the amino acid sequence of SEQ ID NO: 24.

While a variety of amino acid substitutions in wild-type AHASL proteins are known to confer herbicide resistance on a plant expressing such a substituted AHASL protein, an AHASL protein comprising an amino acid substitution at position 188 or equivalent position (e.g., position 203 in the *Arabidopsis* AHASL protein encoded by the nucleotide sequence set forth in GenBank Accession No. X51514) and that confers herbicide resistance on a plant comprising such an AHASL protein has not been previously disclosed. *See,* Example 5 below. *See also,* Hartnett et al. (1990) "Herbicide-resistant plants carrying mutated acetolactate synthase genes,,, In: Managing Resistance to Agrochemicals: Fundamental Research to Practical Strategies, Green et al. (eds.), American Chemical Soc. Symp., Series No. 421, Washington, DC, USA; Simpson (1998) Down to Earth 53(1):26-35; Bruniard (2001) Inheritance of imidazolinone resistance, characterization of cross-resistance pattern, and identification of molecular markers in sunflower (Helianthus annuus L.), Ph.D. Thesis, North Dakota State University, Fargo, ND, USA, pp 1-78.; White et al. (2002) Weed Sci. 50:432-437; and Kolkman et al. (2004) Theor. Appl. Genet. 109: 1147-1159.

Thus, the present invention discloses a novel amino substitution that can be used to produce herbicide-resistant sunflower AHASL proteins, the polynucleotides encoding such proteins, and herbicide-resistant plants, plant tissues, plant cells, and seeds. Because the threonine that is found at amino acid position 188 in wild-type sunflower AHASL1 proteins, or the equivalent position in other AHASL proteins, is within a region of amino acids that is conserved across plant species, it is expected that the substitution of another amino acid, preferably isoleucine, for this same conserved threonine in other AHASL proteins from sunflower (*e*.*g*., sunflower AHASL2 and AHASL3) or from other plant species will result in herbicide-resistant AHASL proteins. Accordingly, such herbicide-resistant AHASL proteins and the polynucleotides encoding them find use in the production of herbicide-resistant plants, plant cells, plant tissues, and seeds by the methods disclosed herein.

The present invention additionally encompasses isolated sunflower *AHASL2* and *AHASL3* polynucleotides that encode herbicide-resistant AHASL2 and AHASL3 proteins, respectively. Such herbicide-resistant AHASL2 and AHASL3 proteins each comprise an amino acid other than threonine at position 188 or equivalent position. Preferably in such herbicide-resistant AHASL2 and AHASL3 proteins, the amino acid at position 188 or equivalent position is isoleucine.

The invention further relates to isolated polynucleotide molecules comprising nucleotide sequences that encode acetohydroxyacid synthase large subunit (AHASL) proteins and to such AHASL proteins. The invention discloses the isolation and nucleotide sequence of a polynucleotide encoding a herbicide-resistant sunflower AHASL1 protein from an herbicide-resistant sunflower plant that was produced by chemical mutagenesis of wild-type sunflower plants. The herbicide-resistant sunflower AHASL1 proteins of the invention possess a threonine-to-isoleucine substitution at position 188 in their respective amino acid sequences, when compared to the corresponding wild-type amino acid sequence. The invention further discloses the isolation and nucleotide sequence of a polynucleotide molecule encoding a wild-type sunflower AHASL1 protein.

The present invention provides isolated polynucleotide molecules that encode AHASL proteins from sunflower *(Helianthus annuus* L.). Specifically, the invention provides isolated polynucleotide molecules comprising: the nucleotide sequence set forth in SEQ ID NO: 1, nucleotide sequences encoding AHASL proteins comprising the amino acid sequence set forth in SEQ ID NO: 2, and fragments and variants of such nucleotide sequences that encode functional AHASL proteins.

The isolated herbicide-resistant AHASL polynucleotide molecules of the invention comprise nucleotide sequences that encode herbicide-resistant AHASL proteins. Such polynucleotide molecules can be used in polynucleotide constructs for the transformation of plants, particularly crop plants, to enhance the resistance of the plants to herbicides, particularly herbicides that are known to inhibit AHAS activity, more particularly imidazolinone herbicides. Such polynucleotide constructs can be used in expression cassettes, expression vectors, transformation vectors, plasmids and the like. The transgenic plants obtained following transformation with such polynucleotide constructs show increased resistance to AHAS-inhibiting herbicides such as, for example, imidazolinone and sulfonylurea herbicides.

Nucleotide sequences that encode AHASL proteins may be included in compositions. In particular, the present invention provides for isolated polynucleotide molecules comprising nucleotide sequences encoding the amino acid sequence shown in SEQ ID NO: 2, and fragments and variants thereof that encode polypeptides comprising AHAS activity. Further provided are polypeptides having an amino acid sequence encoded by a polynucleotide molecule described herein, for example that set forth in SEQ ID NO: 1 , and fragments and variants thereof that encode polypeptides comprising AHAS activity.

The invention encompasses isolated or substantially purified nucleic acid or protein compositions. An "isolated" or "purified" polynucleotide molecule or protein, or biologically active portion thereof, is substantially or essentially free from components that normally accompany or interact with the polynucleotide molecule or protein as found in its naturally occurring environment. Thus, an isolated or purified polynucleotide molecule or protein is substantially free of other cellular material, or culture medium when produced by recombinant techniques, or substantially free of chemical precursors or other chemicals when chemically synthesized. Preferably, an "isolated" nucleic acid is free of sequences (preferably protein encoding sequences) that naturally flank the nucleic acid (i.e., sequences located at the 5' and 3' ends of the nucleic acid) in the genomic DNA of the organism from which the nucleic acid is derived. For example, in various embodiments, the isolated polynucleotide molecule can contain less than about 5 kb, 4 kb, 3 kb, 2 kb, 1 kb, 0.5 kb, or 0.1 kb of nucleotide sequences that naturally flank the polynucleotide molecule in genomic DNA of the cell from which the nucleic acid is derived. A protein that is substantially free of cellular material includes preparations of protein having less than about 30%, 20%, 10%, 5%, or 1% (by dry weight) of contaminating protein. When the protein of the invention or biologically active portion thereof is recombinantly produced, preferably culture medium represents less than about 30%, 20%, 10%, 5%, or 1% (by dry weight) of chemical precursors or non-protein-of interest chemicals.

The present invention provides isolated polypeptides comprising AHASL proteins. The isolated polypeptides comprise an amino acid sequence selected from the group consisting of the amino acid sequence set forth in SEQ ID NO: 2, the amino acid sequences encoded by nucleotide sequence set forth in SEQ ID NO: 1, and functional fragments and variants of said amino acid sequences that encode an AHASL polypeptide comprising AHAS activity. By "functional fragments and variants" is intended fragments and variants of the exemplified polypeptides that comprise AHAS activity.

In certain embodiments of the invention, the methods involve the use of herbicide-tolerant or herbicide-resistant plants. By an "herbicide-tolerant" or "herbicide-resistant" plant, it is intended that a plant that is tolerant or resistant to at least one herbicide at a level that would normally kill, or inhibit the growth of, a normal or wild-type plant. In one embodiment of the invention, the herbicide-tolerant plants of the invention comprise a herbicide-tolerant or herbicide-resistant AHASL protein. By "herbicide-tolerant AHASL protein" or "herbicide-resistant AHASL protein", it is intended that such an AHASL protein displays higher AHAS activity, relative to the AHAS activity of a wild-type AHASL protein, when in the presence of at least one herbicide that is known to interfere with AHAS activity and at a concentration or level of the herbicide that is to known to inhibit the AHAS activity of the wild-type AHASL protein. Furthermore, the AHAS activity of such a herbicide-tolerant or herbicide-resistant AHASL protein may be referred to herein as "herbicide-tolerant" or "herbicide-resistant" AHAS activity.

For the present invention, the terms "herbicide-tolerant" and "herbicide-resistant" are used interchangeable and are intended to have an equivalent meaning and an equivalent scope. Similarly, the terms "herbicide-tolerance" and "herbicide-resistance" are used interchangeable and are intended to have an equivalent meaning and an equivalent scope. Likewise, the terms "imidazolinone-resistant" and "imidazolinone-resistance" are used interchangeable and are intended to be of an equivalent meaning and an equivalent scope as the terms "imidazolinone-tolerant" and "imidazolinone-tolerance", respectively.

The invention encompasses herbicide-resistant AHASL polynucleotides and herbicide-resistant AHASL proteins. By "herbicide-resistant AHASL polynucleotide" is intended a polynucleotide that encodes a protein comprising herbicide-resistant AHAS activity. By "herbicide-resistant AHASL protein" is intended a protein or polypeptide that comprises herbicide-resistant AHAS activity.

Further, it is recognized that a herbicide-tolerant or herbicide-resistant AHASL protein can be introduced into a plant by transforming a plant or ancestor thereof with a nucleotide sequence encoding a herbicide-tolerant or herbicide-resistant AHASL protein. Such herbicide-tolerant or herbicide-resistant AHASL proteins are encoded by the herbicide-tolerant or herbicide-resistant *AHASL* polynucleotides. Alternatively, a herbicide-tolerant or herbicide-resistant AHASL protein may occur in a plant as a result of a naturally occurring or induced mutation in an endogenous *AHASL* gene in the genome of a plant or progenitor thereof.

The present invention provides plants, plant tissues, plant cells, and host cells with increased resistance or tolerance to at least one imidazolinone herbicide. The preferred amount or concentration of the herbicide is an "effective amount" or "effective concentration." By "effective amount" and "effective concentration" is intended an amount and concentration, respectively, that is sufficient to kill or inhibit the growth of a similar, wild-type, plant, plant tissue, plant cell, or host cell, but that said amount does not kill or inhibit as severely the growth of the herbicide-resistant plants, plant tissues, plant cells, and host cells of the present invention. Typically, the effective amount of a herbicide is an amount that is routinely used in agricultural production systems to kill weeds of interest. Such an amount is known to those of ordinary skill in the art, or can be easily determined using methods known in the art.

The herbicides of the present invention are those that interfere with the activity of the AHAS enzyme such that AHAS activity is reduced in the presence of the herbicide. Such herbicides may also referred to herein as "AHAS-inhibiting herbicides" or simply "AHAS inhibitors." As used herein, an "AHAS-inhibiting herbicide" or an "AHAS inhibitor" is not meant to be limited to single herbicide that interferes with the activity of the AHAS enzyme. Thus, unless otherwise stated or evident from the context, an "AHAS-inhibiting herbicide" or an "AHAS inhibitor" can be a one herbicide or a mixture of two, three, four, or more herbicides, each of which interferes with the activity of the AHAS enzyme.

By "similar, wild-type, plant, plant tissue, plant cell or host cell" is intended a plant, plant tissue, plant cell, or host cell, respectively, that lacks the herbicide-resistance characteristics and/or particular polynucleotide of the invention that are disclosed herein. The use of the term "wild-type" is not, therefore, intended to imply that a plant, plant tissue, plant cell, or other host cell lacks recombinant DNA in its genome, and/or does not possess herbicide-resistant characteristics that are different from those disclosed herein.

As used herein unless clearly indicated otherwise, the term "plant" intended to mean a plant at any developmental stage, as well as any part or parts of a plant that may be attached to or separate from a whole intact plant. Such parts of a plant include, but are not limited to, organs, tissues, and cells of a plant. Examples of particular plant parts include a stem, a leaf, a root, an inflorescence, a flower, a floret, a fruit, a pedicle, a peduncle, a stamen, an anther, a stigma, a style, an ovary, a petal, a sepal, a carpel, a root tip, a root cap, a root hair, a leaf hair, a seed hair, a pollen grain, a microspore, a cotyledon, a hypocotyl, an epicotyl, xylem, phloem, parenchyma, endosperm, a companion cell, a guard cell, and any other known organs, tissues, and cells of a plant. Furthermore, it is recognized that a seed is a plant.

The plants of the present invention include both non-transgenic plants and transgenic plants. By "non-transgenic plant" is intended to mean a plant lacking recombinant DNA in its genome. By "transgenic plant" is intended to mean a plant comprising recombinant DNA in its genome. Such a transgenic plant can be produced by introducing recombinant DNA into the genome of the plant. When such recombinant DNA is incorporated into the genome of the transgenic plant, progeny of the plant can also comprise the recombinant DNA. A progeny plant that comprises at least a portion of the recombinant DNA of at least one progenitor transgenic plant is also a transgenic plant.

For the purposes of the present invention unless otherwise expressly indicated or apparent from the context, a "progeny plant" is any plant that is descended from at least one plant of the invention and includes, but is not limited to, first, second, third, fourth, fifth, sixth, seventh, eight, ninth, and tenth generation descendents of the plant of the invention. Preferably, such progeny or descendents comprise increased resistance to at least one herbicide when compared to a wild-type plant. More preferably, such progeny or descendents comprise increased resistance to at least one imidazolinone herbicide when compared to a wild-type plant. Even more preferably, such progeny or descendents comprise the herbicide-resistance characteristics of the plant with ATCC Patent Deposit Number PTA-6325, and/ or a herbicide-resistant AHASL protein comprising an isoleucine at amino acid position 188 or equivalent position.

A herbicide-resistant sunflower line that is referred to herein as MUT9 is described herein. A deposit of at least 2375 seeds from sunflower line RHA266 MUT-9-1-1-2 (referred to herein as "MUT9") with the Patent Depository of the American Type Culture Collection (ATCC), Manassas, VA 20110 USA was made on November 22, 2004 and assigned ATCC Patent Deposit Number PTA-6325. Due to a shortage of seeds of the MUT9 line, less than 2500 seeds of the MUT9 line were initially submitted to the ATCC. On February 4, 2005, Applicants made a supplemental deposit of 125 seeds to the ATCC to reach a total of at least 2500 seeds. The deposit of sunflower line MUT9 was made for a term of at least 30 years and at least 5 years after the most recent request for the furnishing of a sample of the deposit is received by the ATCC. The deposit will be maintained under the terms of the Budapest Treaty on the International Recognition of the Deposit of Microorganisms for the Purposes of Patent Procedure. The deposit of sunflower line MUT9 was made for a term of at least 30 years and at least 5 years after the most recent request for the furnishing of a sample of the deposit is received by the ATCC. Additionally, Applicants have satisfied all the requirements of 37 C.F.R. §§ 1.801-1.809, including providing an indication of the viability of the sample.

The herbicide-resistant sunflower plants of the MUT9 line were produced by a mutation breeding. Wild-type sunflower plants were mutagenized by exposing the plants to a mutagen, particularly a chemical mutagen, more particularly ethyl methanesulfonate (EMS). Any mutagenesis method known in the art may be used to produce the herbicide-resistant sunflower plants of the present invention. Such mutagenesis methods can involve, for example, the use of any one or more of the following mutagens: radiation, such as X-rays, Gamma rays (e.g., cobalt 60 or cesium 137), neutrons, (e.g., product of nuclear fission by uranium 235 in an atomic reactor), Beta radiation (e.g., emitted from radioisotopes such as phosphorus 32 or carbon 14), and ultraviolet radiation (preferably from 2500 to 2900 nm), and chemical mutagens such as base analogues (e.g., 5-bromo-uracil), related compounds (e.g.; 8-ethoxy caffeine), antibiotics (e.g., streptonigrin), alkylating agents (e.g., sulfur mustards, nitrogen mustards, epoxides, ethylenamines, sulfates, sulfonates, sulfones, lactones), azide, hydroxylamine, nitrous acid, or acridines. Herbicide-resistant plants can also be produced by using tissue culture methods to select for plant cells comprising herbicide-resistance mutations and then regenerating herbicide-resistant plants therefrom. See, for example, U.S. Patent Nos. 5,773,702 and 5,859,348. Further details of mutation breeding can be found in "Principals of Cultivar Development" Fehr, 1993 Macmillan Publishing Company.

Analysis of the AHASL1 gene of the sunflower plant of the MUT9 line revealed that the mutation that results in the substitution of a isoleucine for the threonine that is found at amino acid position 188 in the wild-type AHASL1 amino acid sequence for SEQ ID NO: 4. Thus, the present invention discloses that substituting another amino acid for the threonine at position 188 can cause a sunflower plant to have enhanced resistance to a herbicide, particularly an imidazolinone and/or sulfonylurea herbicide. As disclosed in Example 5 below, the threonine at amino acid position 188 occurs within a conserved region of AHASL proteins. Similarly, amino acid substitutions in other conserved regions of AHASL proteins have been disclosed that are known to confer herbicide resistance on a plant that comprises such an AHASL protein. Accordingly, the herbicide-resistant sunflower plants of the invention include, but are not limited to those sunflower plants which comprise in their genomes at least one copy of an AHASL polynucleotide that encodes a herbicide-resistant AHASL protein that comprises a isoleucine at amino acid position 188 or equivalent position.

The sunflower plants of the invention further include plants that comprise, relative to the wild-type AHASL protein, an isoleucine or another amino acid other than threonine at amino acid position 188 or equivalent position and one or more additional amino acid substitutions in the AHASL protein relative to the wild-type AHASL protein, wherein such a sunflower plant has increased resistance to at least one herbicide when compared to a wild-type sunflower plant. Such sunflower plants comprise AHASL proteins that comprise at least one member selected from the group consisting of: a threonine at amino acid position 107 or equivalent position; an alanine, threonine, histidine, leucine, arginine, isoleucine, glutamine, or serine at amino acid position 182 or equivalent position; an aspartate or valine at amino acid position 190 or equivalent position; a leucine at amino acid position 559 or equivalent position; and an asparagine, threonine, or phenylalanine at amino acid position 638 or equivalent position.

The present invention provides AHASL proteins with amino acid substitutions at particular amino acid positions within conserved regions of the sunflower AHASL proteins disclosed herein. Furthermore, those of ordinary skill will recognize that such amino acid positions can vary depending on whether amino acids are added or removed from, for example, the N-terminal end of an amino acid sequence. Thus, the invention encompasses the amino substitutions at the recited position or equivalent position (e.g., "amino acid position 188 or equivalent position"). By "equivalent position" is intended to mean a position that is within the same conserved region as the exemplified amino acid position. Such conserved regions are know in the art (see Table 6 below) or can be determine by multiple sequence alignments as disclosed herein or by methods known in the art.

In addition, the present invention provides AHASL polypeptides comprising amino acid substitutions that are known to confer resistance on a plant to at least one herbicide, particularly an imidazolinone herbicide and/or a sulfonylurea herbicide. Such AHASL polypeptides include, for example, those that comprise at least one member selected from the group consisting of: an isoleucine or another amino acid other than threonine at amino acid position 188 or equivalent position; a threonine at amino acid position 107 or equivalent position; an alanine, threonine, histidine, leucine, arginine, isoleucine, glutamine, or serine at amino acid position 182 or equivalent position; an aspartate or valine at amino acid position 190 or equivalent position; an aspartate or valine at amino acid position 190 or equivalent position; a leucine at amino acid position 559 or equivalent position; and an asparagine, threonine, or phenylalanine at amino acid position 638 or equivalent position. The invention further provides isolated polynucleotides encoding such AHASL polypeptides, as well as expression cassettes, transformation vectors, transformed host cells, transformed plants, and methods comprising such polynucleotides.

The present invention provides methods for enhancing the tolerance or resistance of a plant, plant tissue, plant cell, or other host cell to at least one herbicide that interferes with the activity of the AHAS enzyme. Preferably, such an AHAS-inhibiting herbicide is an imidazolinone herbicide, a sulfonylurea herbicide, a triazolopyrimidine herbicide, a pyrimidinyloxybenzoate herbicide, a sulfonylamino-carbonyltriazolinone herbicide, or mixture thereof. More preferably, such a herbicide is an imidazolinone herbicide, a sulfonylurea herbicide, or mixture thereof. For the present invention, the imidazolinone herbicides include, but are not limited to, PURSUIT® (imazethapyr), GADRE® (imazapic), RAPTOR® (imazamox), SCEPTER® (imazaquin), ASSERT® (imazethabenz), ARSENAL® (imazapyr), a derivative of any of the aforementioned herbicides, and a mixture of two or more of the aforementioned herbicides, for example, imazapyr/imazamox (ODYSSEY®). More specifically, the imidazolinone herbicide can be selected from, but is not limited to, 2- (4-isopropyl-4-methyl-5-oxo-2-imidiazolin-2-yl) -nicotinic acid, [2- (4-isopropyl)-4-] [methyl-5-oxo-2-imidazolin-2-yl)-3-quinolinecarboxylic] acid, [5-ethyl-2- (4-isopropyl-] 4-methyl-5-oxo-2-imidazolin-2-yl) -nicotinic acid, 2- (4-isopropyl-4-methyl-5-oxo-2- imidazolin-2-yl)-5- (methoxymethyl)-nicotinic acid, [2-(4-isopropyl-4-methyl-5-oxo-2-] imidazolin-2-yl)-5-methylnicotinic acid, and a mixture of methyl [6- (4-isopropyl-4-] methyl-5-oxo-2-imidazolin-2-yl) -m-toluate and methyl [2- (4-isopropyl-4-methyl-5-] oxo-2-imidazolin-2-yl) -p-toluate. The use of 5-ethyl-2- (4-isopropyl-4-methyl-5-oxo- 2-imidazolin-2-yl) -nicotinic acid and [2-(4-isopropyl-4-methyl-5-oxo-2-imidazolin-2-] yl)-5- (methoxymethyl)-nicotinic acid is preferred. The use of [2- (4-isopropyl-4-] methyl-5-oxo-2-imidazolin-2-yl)-5-(methoxymethyl)-nicotinic acid is particularly preferred.

For the present invention, the sulfonylurea herbicides include, but are not limited to, chlorsulfuron, metsulfuron methyl, sulfometuron methyl, chlorimuron ethyl, thifensulfuron methyl, tribenuron methyl, bensulfuron methyl, nicosulfuron, ethametsulfuron methyl, rimsulfuron, triflusulfuron methyl, triasulfuron, primisulfuron methyl, cinosulfuron, amidosulfiuon, fluzasulfuron, imazosulfuron, pyrazosulfuron ethyl, halosulfuron, azimsulfuron, cyclosulfuron, ethoxysulfuron, flazasulfuron, flupyrsulfuron methyl, foramsulfuron, iodosulfuron, oxasulfuron, mesosulfuron, prosulfuron, sulfosulfuron, trifloxysulfuron, tritosulfuron, a derivative of any of the aforementioned herbicides, and a mixture of two or more of the aforementioned herbicides. The triazolopyrimidine herbicides of the invention include, but are not limited to, cloransulam, diclosulam, florasulam, flumetsulam, metosulam, and penoxsulam. The pyrimidinyloxybenzoate herbicides of the invention include, but are not limited to, bispyribac, pyrithiobac, pyriminobac, pyribenzoxim and pyriftalid. The sulfonylamino-carbonyltriazolinone herbicides include, but are not limited to, flucarbazone and propoxycarbazone.

It is recognized that pyrimidinyloxybenzoate herbicides are closely related to the pyrimidinylthiobenzoate herbicides and are generalized under the heading of the latter name by the Weed Science Society of America. Thereofore, unless indicated otherwise herein or apparent from the context, pyrimidinylthiobenzoate herbicides of the present invention include, but are not limited to, pyrimidinyloxybenzoate herbicides, pyrimidinylthiobenzoate herbicides, and mixtures thereof. Likewise, unless indicated otherwise herein or apparent from the context, pyrimidinyloxybenzoate herbicides of the present invention include, but are not limited to, pyrimidinyloxybenzoate herbicides, pyrimidinylthiobenzoate herbicides, and mixtures thereof.

The present invention provides methods for enhancing AHAS activity in a plant comprising transforming a plant with a polynucleotide construct comprising a promoter operably linked to an AHASL1 nucleotide sequence of the invention. The methods involve introducing a polynucleotide construct of the invention into at least one plant cell and regenerating a transformed plant therefrom. The methods involve the use of a promoter that is capable of driving gene expression in a plant cell. Preferably, such a promoter is a constitutive promoter or a tissue-preferred promoter. The methods find use in enhancing or increasing the resistance of a plant to at least one herbicide that interferes with the catalytic activity of the AHAS enzyme, particularly an imidazolinone herbicide.

The present invention provides expression cassettes for expressing the polynucleotides of the invention in plants, plant tissues, plant cells, and other host cells. The expression cassettes comprise a promoter expressible in the plant, plant tissue, plant cell, or other host cells of interest operably linked to a polynucleotide of the invention that comprises a nucleotide sequence encoding either a full-length (i.e. including the chloroplast transit peptide) or mature AHASL1 protein (i.e. without the chloroplast transit peptide). If expression is desired in the plastids or chloroplasts of plants or plant cells, the expression cassette may also comprise an operably linked chloroplast-targeting sequence that encodes a chloroplast transit peptide.

The expression cassettes of the invention find use in a method for enhancing the herbicide tolerance of a plant or a host cell. The method involves transforming the plant or host cell with an expression cassette of the invention, wherein the expression cassette comprises a promoter that is expressible in the plant or host cell of interest and the promoter is operably linked to a polynucleotide of the invention that comprises a nucleotide sequence encoding an imidazolinone-resistant AHASL1 protein of the invention.

The use of the term "polynucleotide constructs" herein is not intended to limit the present invention to polynucleotide constructs comprising DNA. Those of ordinary skill in the art will recognize that polynucleotide constructs, particularly polynucleotides and oligonucleotides, comprised of ribonucleotides and combinations of ribonucleotides and deoxyribonucleotides may also be employed in the methods disclosed herein. Thus, the polynucleotide constructs of the present invention encompass all polynucleotide constructs that can be employed in the methods of the present invention for transforming plants including, but not limited to, those comprised of deoxyribonucleotides, ribonucleotides, and combinations thereof. Such deoxyribonucleotides and ribonucleotides include both naturally occurring molecules and synthetic analogues. The polynucleotide constructs of the invention also encompass all forms of polynucleotide constructs including, but not limited to, single-stranded forms, double-stranded forms, hairpins, stem-and-loop structures, and the like. Furthermore, it is understood by those of ordinary skill in the art that each nucleotide sequences disclosed herein also encompasses the complement of that exemplified nucleotide sequence.

Furthermore, it is recognized that the methods of the invention may employ a polynucleotide construct that is capable of directing, in a transformed plant, the expression of at least one protein, or at least one RNA, such as, for example, an antisense RNA that is complementary to at least a portion of an mRNA. Typically such a polynucleotide construct is comprised of a coding sequence for a protein or an RNA operably linked to 5' and 3' transcriptional regulatory regions. Alternatively, it is also recognized that the methods of the invention may employ a polynucleotide construct that is not capable of directing, in a transformed plant, the expression of a protein or an RNA.

Further, it is recognized that, for expression of a polynucleotides of the invention in a host cell of interest, the polynucleotide is typically operably linked to a promoter that is capable of driving gene expression in the host cell of interest. The methods of the invention for expressing the polynucleotides in host cells do not depend on particular promoter. The methods encompass the use of any promoter that is known in the art and that is capable of driving gene expression in the host cell of interest.

The present invention encompasses AHASL polynucleotide molecules and fragments and variants thereof. Polynucleotide molecules that are fragments of these nucleotide sequences are also encompassed by the present invention. By "fragment" is intended a portion of the nucleotide sequence encoding an AHASL protein of the invention. A fragment of an AHASL nucleotide sequence of the invention may encode a biologically active portion of an AHASL protein, or it may be a fragment that can be used as a hybridization probe or PCR primer using methods disclosed below. A biologically active portion of an AHASL protein can be prepared by isolating a portion of one of the AHASL nucleotide sequences of the invention, expressing the encoded portion of the AHASL protein (e.g., by recombinant expression *in vitro),* and assessing the activity of the encoded portion of the AHASL1 protein. Polynucleotide molecules that are fragments of an AHASL nucleotide sequence comprise at least about 15, 20, 50, 75, 100, 200, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550, 1600, 1650, or 1700 nucleotides, or up to the number of nucleotides present in a full-length nucleotide sequence disclosed herein (for example, 1684 and 1706 nucleotides for SEQ ID NOS: 1 and 3, respectively) depending upon the intended use.

A fragment of an AHASL nucleotide sequence that encodes a biologically active portion of an AHASL protein of the invention will encode at least about 15, 25, 30, 50, 75, 100, 125, 150, 175, 200, 250, 300, 350, 400, 450, 500, or 550 contiguous amino acids, or up to the total number of amino acids present in a fidl-length AHASL1 protein of the invention (for example, 561 and 568 amino acids for SEQ ID NOS: 2 and 4, respectively). Fragments of an AHASL1 nucleotide sequence that are useful as hybridization probes for PCR primers generally need not encode a biologically active portion of an AHASL1 protein.

Polynucleotide molecules that are variants of the nucleotide sequences disclosed herein are also encompassed by the present invention. "Variants" of the AHASL nucleotide sequences of the invention include those sequences that encode the AHASL proteins disclosed herein but that differ conservatively because of the degeneracy of the genetic code. These naturally occurring allelic variants can be identified with the use of well-known molecular biology techniques, such as polymerase chain reaction (PCR) and hybridization techniques as outlined below. Variant nucleotide sequences also include synthetically derived nucleotide sequences that have been generated, for example, by using site-directed mutagenesis but which still encode the AHASL I protein disclosed in the present invention as discussed below. Generally, nucleotide sequence variants of the invention will have at least about 75%, 80%, 85%, 90%, 91%, 92%, 93%, 94%, 95%, 96%, 97%, 98%, or 99% identity to a particular nucleotide sequence disclosed herein. A variant AHASL nucleotide sequence will encode an AHASL protein, respectively, that has an amino acid sequence having at least about 75%, 80%, 85%, 90%, 91%, 92%, 93%, 94%, 95%, 96%, 97%, 98%, or 99% identity to the amino acid sequence of an AHASL protein disclosed herein.

In addition, the skilled artisan will further appreciate that changes can be introduced by mutation into the nucleotide sequences of the invention thereby leading to changes in the amino acid sequence of the encoded AHASL proteins without altering the biological activity of the AHASL proteins. Thus, an isolated polynucleotide molecule encoding an AHASL protein having a sequence that differs from that of SEQ ID NO: I can be created by introducing one or more nucleotide substitutions, additions, or deletions into the corresponding nucleotide sequence disclosed herein, such that one or more amino acid substitutions, additions or deletions are introduced into the encoded protein. Mutations can be introduced by standard techniques, such as site-directed mutagenesis and PCR-mediated mutagenesis. Such variant nucleotide sequences are also encompassed by the present invention.

For example, preferably, conservative amino acid substitutions may be made at one or more predicted, preferably nonessential amino acid residues. A "nonessential" amino acid residue is a residue that can be altered from the wild-type sequence of an AHASL protein (e.g., the sequence of SEQ ID NOS: 2 and 4, respectively) without altering the biological activity, whereas an "essential" amino acid residue is required for biological activity. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Such substitutions would not be made for conserved amino acid residues, or for amino acid residues residing within a conserved motif.

The proteins of the invention may be altered in various ways including amino acid substitutions, deletions, truncations, and insertions. Methods for such manipulations are generally known in the art. For example, amino acid sequence variants of the AHASL proteins can be prepared by mutations in the DNA. Methods for mutagenesis and nucleotide sequence alterations are well known in the art. See, for example, Kunkel (1985) Proc. Natl. Acad Sci. USA 82:488-492; Kunkel et al. (1987) Methods in Enzymol. 154:367-382; U.S. Patent No. 4,873,192; Walker and Gaastra, eds. (1983) Techniques in Molecular Biology (MacMillan Publishing Company, New York) and the references cited therein. Guidance as to appropriate amino acid substitutions that do not affect biological activity of the protein of interest may be found in the model of Dayhoff et al. (1978) Atlas of Protein Sequence and Structure (Natl. Biomed. Res. Found., Washington, D.C.). Conservative substitutions, such as exchanging one amino acid with another having similar properties, may be preferable.

Alternatively, variant AHASL nucleotide sequences can be made by introducing mutations randomly along all or part of an AHASL coding sequence, such as by saturation mutagenesis, and the resultant mutants can be screened for AHAS activity to identify mutants that retain AHAS activity, including herbicide-resistant AHAS activity. Following mutagenesis, the encoded protein can be expressed recombinantly, and the activity of the protein can be determined using standard assay techniques.

Thus, the nucleotide sequences of the invention include the sequences disclosed herein as well as fragments and variants thereof. The *AHASL* nucleotide sequences of the invention, and fragments and variants thereof, can be used as probes and/or primers to identify and/or clone *AHASL* homologues in other plants. Such probes can be used to detect transcripts or genomic sequences encoding the same or identical proteins.

In this manner, methods such as PCR, hybridization, and the like can be used to identify such sequences having substantial identity to the sequences of the invention. *See,* for example, Sambrook et al. (1989) Molecular Cloning: Laboratory Manual (2d ed., Cold Spring Harbor Laboratory Press, Plainview, NY) and Innis, et al. (1990) PCR Protocols: A Guide to Methods and Applications (Academic Press, NY). AHASL nucleotide sequences isolated based on their sequence identity to the AHASL1 nucleotide sequences set forth herein or to fragments and variants thereof are encompassed by the present invention.

In a hybridization method, all or part of a known *AHASL* nucleotide sequence can be used to screen cDNA or genomic libraries. Methods for construction of such cDNA and genomic libraries are generally known in the art and are disclosed in Sambrook et al. (1989) Molecular Cloning: A Laboratory Manual (2d ed., Cold Spring Harbor Laboratory Press, Plainview, NY). The so-called hybridization probes may be genomic DNA fragments, cDNA fragments, RNA fragments, or other oligonucleotides, and may be labeled with a detectable group such as ³²P, or any other detectable marker, such as other radioisotopes, a fluorescent compound, an enzyme, or an enzyme co-factor. Probes for hybridization can be made by labeling synthetic oligonucleotides based on the known *AHASL* nucleotide sequence disclosed herein. Degenerate primers designed on the basis of conserved nucleotide or amino acid residues in a known *AHASL* nucleotide sequence or encoded amino acid sequence can additionally be used. The probe typically comprises a region of nucleotide sequence that hybridizes under stringent conditions to at least about 12, preferably about 25, more preferably about 50, 75, 100, 125, 150, 175, 200, 250, 300, 3 50, 400, 500,600, 700, 800, 900, 1000, 1100, 1200, 1300, 1400, 1500, 1600, or 1700 consecutive nucleotides of an *AHASL* nucleotide sequence of the invention or a fragment or variant thereof. Preparation of probes for hybridization is generally known in the art and is disclosed in Sambrook et al. (1989) Molecular Cloning: A Laboratory Manual (2d ed., Cold Spring Harbor Laboratory Press, Plainview, New York).

For example, the entire *AHASL* sequence disclosed herein, or one or more portions thereof, may be used as a probe capable of specifically hybridizing to corresponding *AHASL* sequences and messenger RNAs. Hybridization techniques include hybridization screening of plated DNA libraries (either plaques or colonies; see, for example, Sambrook et al. (1989) Molecular Cloning: A Laboratory Manual (2d ed., Cold Spring Harbor Laboratory Press, Plainview, New York).

Hybridization of such sequences may be carried out under stringent conditions. By "stringent conditions" or "stringent hybridization conditions" is intended conditions under which a probe will hybridize to its target sequence to a detectably greater degree than to other sequences (e.g., at least 2-fold over background). Stringent conditions are sequence-dependent and will be different in different circumstances.

Typically, stringent conditions will be those in which the salt concentration is less than about 1.5 M Na ion, typically about 0.01 to 1.0 M Na ion concentration (or other salts) at pH 7.0 to 8.3 and the temperature is at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1.0 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0,1X SSC at 60 to 65°C. Optionally, wash buffers may comprise about 0.1 % to about 1% SDS. The duration of hybridization is generally less than about 24 hours, usually about 4 to about 12 hours.

Specificity is typically the function of post-hybridization washes, the critical factors being the ionic strength and temperature of the final wash solution. For DNA-DNA hybrids, the Tₘ can be approximated from the equation of Meinkoth and Wahl (1984) Anal. Biochem. 138:267-284: Tₘ = 81.5°C + 16.6 (log M) + 0.41 (%GC) - 0.61 (% form) - 500/L; where M is the molarity of monovalent cations, %GC is the percentage of guanosine and cytosine nucleotides in the DNA, % form is the percentage of formamide in the hybridization solution, and L is the length of the hybrid in base pairs. The Tₘ is the temperature (under defined ionic strength and pH) at which 50% of a complementary target sequence hybridizes to a perfectly matched probe. Tₘ is reduced by about 1 °C for each 1% of mismatching; thus, Tₘ, hybridization, and/or wash conditions can be adjusted to hybridize to sequences of the desired identity. For example, if sequences with ≥90% identity are sought, the Tₘ can be decreased 10°C. Generally, stringent conditions are selected to be about 5°C lower than the thermal melting point (Tₘ) for the specific sequence and its complement at a defined ionic strength and pH. However, severely stringent conditions can utilize a hybridization and/or wash at 1, 2, 3, or 4°C lower than the thermal melting point (Tₘ); moderately stringent conditions can utilize a hybridization and/or wash at 6, 7, 8, 9, or 10°C lower than the thermal melting point (Tₘ); low stringency conditions can utilize a hybridization and/or wash at 11, 12, 13, 14, 15, or 20°C lower than the thermal melting point (Tₘ). Using the equation, hybridization and wash compositions, and desired Tₘ, those of ordinary skill will understand that variations in the stringency of hybridization and/or wash solutions are inherently described. If the desired degree of mismatching results in a Tₘ of less than 45°C (aqueous solution) or 32°C (formamide solution), it is preferred to increase the SSC concentration so that a higher temperature can be used. An extensive guide to the hybridization of nucleic acids is found in Tijssen (1993) Laboratory Techniques in Biochemistry and Molecular Biology— Hybridization with Nucleic Acid Probes, Part I, Chapter 2 (Elsevier, New York); and Ausubel et al., eds. (1995) Current Protocols in Molecular Biology, Chapter 2 (Greene Publishing and Wiley-Interscience, New York). See Sambrook et al. (1989) Molecular Cloning: A Laboratory Manual (2d ed., Cold Spring Harbor Laboratory Press, Plainview, New York).

It is recognized that the polynucleotide molecules and proteins of the invention encompass polynucleotide molecules and proteins comprising a nucleotide or an amino acid sequence that is sufficiently identical to the nucleotide sequence of SEQ ID NO: 1, or to the amino acid sequence of SEQ ID NO: 2. The term "sufficiently identical" is used herein to refer to a first amino acid or nucleotide sequence that contains a sufficient or minimum number of identical or equivalent (e.g., with a similar side chain) amino acid residues or nucleotides to a second amino acid or nucleotide sequence such that the first and second amino acid or nucleotide sequences have a common structural domain and/or common functional activity. Amino acid or nucleotide sequences that contain a common structural domain having at least about 75% identity, more preferably 85%, 95%, or 98% identity are defined herein as sufficiently identical.

To determine the percent identity of two amino acid sequences or of two nucleic acids, the sequences are aligned for optimal comparison purposes. The percent identity between the two sequences is a function of the number of identical positions shared by the sequences (*i.e.*, percent identity = number of identical positions/total number of positions (*e*.*g*., overlapping positions) x 100). In one embodiment, the two sequences are the same length. The percent identity between two sequences can be determined using techniques similar to those described below, with or without allowing gaps. In calculating percent identity, typically exact matches are counted.

The determination of percent identity between two sequences can be accomplished using a mathematical algorithm. A preferred, nonlimiting example of a mathematical algorithm utilized for the comparison of two sequences is the algorithm of Karlin and Altschul (1990) Proc. Natl. Acad. Sci. USA 87:2264, modified as in Karlin and Altschul (1993) Proc. Natl. Acad Sci. USA 90:5873-5877. Such an algorithm is incorporated into the NBLAST and XBLAST programs of Altschul et al. (1990) J. Mol. Biol. 215:403. BLAST nucleotide searches can be performed with the NBLAST program, score = 100, wordlength = 12, to obtain nucleotide sequences homologous to the polynucleotide molecules of the invention. BLAST protein searches can be performed with the XBLAST program, score = 50, wordlength = 3, to obtain amino acid sequences homologous to protein molecules of the invention. To obtain gapped alignments for comparison purposes, Gapped BLAST can be utilized as described in Altschul et al. (1997) Nucleic Acids Res. 25:3389. Alternatively, PSI-Blast can be used to perform an iterated search that detects distant relationships between molecules. *See* Altschul *et al.* (1997) *supra.* When utilizing BLAST, Gapped BLAST, and PSI-Blast programs, the default parameters of the respective programs (e.g., XBLAST and NBLAST) can be used. *See* http://www.ncbi.nlm.nih.gov. Another preferred, non-limiting example of a mathematical algorithm utilized for the comparison of sequences is the algorithm of Myers and Miller (1988) CABIOS 4:11-17. Such an algorithm is incorporated into the ALIGN program (version 2.0), which is part of the GCG sequence alignment software package. When utilizing the ALIGN program for comparing amino acid sequences, a PAM120 weight residue table, a gap length penalty of 12, and a gap penalty of 4 can be used. Alignment may also be performed manually by inspection.

Unless otherwise stated, sequence identity/similarity values provided herein refer to the value obtained using the full-length sequences of the invention and using multiple alignment by mean of the algorithm Clustal W (Nucleic Acid Research, 22(22):4673-4680, 1994) using the program AlignX included in the software package Vector NTI Suite Version 7 (InforMax, Inc., Bethesda, MD, USA) using the default parameters; or any equivalent program thereof. By "equivalent program" is intended any sequence comparison program that, for any two sequences in question, generates an alignment having identical nucleotide or amino acid residue matches and an identical percent sequence identity when compared to the corresponding alignment generated by AlignX in the software package Vector NTI Suite Version 7.

The *AHASL* nucleotide sequences of the invention include mutant forms that encode AHASL proteins comprising herbicide-resistant AHAS activity. Likewise, the proteins of the invention encompass variations and modified forms of naturally occurring proteins. Such variants will continue to possess the desired AHAS activity. Obviously, the mutations that will be made in the DNA encoding the variant must not place the sequence out of reading frame and preferably will not create complementary regions that could produce secondary mRNA structure. See, EP Patent Application Publication No. 75,444.

The deletions, insertions, and substitutions of the protein sequences encompassed herein are not expected to produce radical changes in the characteristics of the protein. However, when it is difficult to predict the exact effect of the substitution, deletion, or insertion in advance of doing so, one skilled in the art will appreciate that the effect will be evaluated by routine screening assays. That is, the activity can be evaluated by AHAS activity assays. *See;* for example, Singh et al. (1988) Anal. Biochem. 171:173-179.

Variant nucleotide sequences and proteins also encompass sequences and proteins derived from a mutagenic and recombinogenic procedure such as DNA shuffling. With such a procedure, one or more different *AHASL* coding sequences can be manipulated to create a new AHASL protein possessing the desired properties. In this manner, libraries of recombinant polynucleotides are generated from a population of related sequence polynucleotides comprising sequence regions that have substantial sequence identity and can be homologously recombined *in vitro* or *in vivo.* For example, using this approach, sequence motifs encoding a domain of interest may be shuffled between the *AHASL* gene of the invention and other known *AHASL* genes to obtain a new gene coding for a protein with an improved property of interest, such as an increased Kₘ in the case of an enzyme. Strategies for such DNA shuffling are known in the art. See, for example, Stemmer (1994) Proc. Natl. Acad. Sci. USA 91:10747-10751; Stemmer (1994) Nature 370:389-391; Crameri et al. (1997) Nature Biotech. 15:436-438; Moore et al. (1997) J. Mol. Biol. 272:336-347; Zhang et al. (1997) Proc. Natl. Acad. Sci. USA 94:4504-4509; Crameri et al. (1998) Nature 391:288-291; and U.S. Patent Nos. 5,605,793 and 5,837,458.

The nucleotide sequences of the invention can be used to isolate corresponding sequences from other organisms, particularly other plants, more particularly other dicots. In this manner, methods such as PCR, hybridization, and the like can be used to identify such sequences based on their sequence homology to the sequences set forth herein. Sequences isolated based on their sequence identity to the entire *AHASL* polynucleotide sequences set forth herein or to fragments thereof are encompassed by the present invention. Thus, isolated polynucleotide sequences that encode for an AHASL protein and which hybridize under stringent conditions to the sequence disclosed herein, or to fragments thereof, are encompassed by the present invention.

In a PCR approach, oligonucleotide primers can be designed for use in PCR reactions to amplify corresponding DNA sequences from cDNA or genomic DNA extracted from any plant of interest. Methods for designing PCR primers and PCR cloning are generally known in the art and are disclosed in Sambrook et al. (1989) Molecular Cloning: A Laboratory Manual (2d ed., Cold Spring Harbor Laboratory Press, Plainview, New York). See also Innis et al., eds. (1990) PCR Protocols: A Guide to Methods and Applications (Academic Press, New York); Innis and Gelfand, eds. (1995) PCR Strategies (Academic Press, New York); and Innis and Gelfand, eds. (1999) PCR Methods Manual (Academic Press, New York). Known methods of PCR include, but are not limited to, methods using paired primers, nested primers, single specific primers, degenerate primers, gene-specific primers, vector-specific primers, partially-mismatched primers, and the like.

The *AHASL* polynucleotide sequences of the invention are provided in expression cassettes for expression in the plant of interest. The cassette will include 5' and 3' regulatory sequences operably linked to an *AHASL* polynucleotide sequence of the invention. By "operably linked" is intended a functional linkage between a promoter and a second sequence, wherein the promoter sequence initiates and mediates transcription of the DNA sequence corresponding to the second sequence. Generally, operably linked means that the nucleic acid sequences being linked are contiguous and, where necessary to join two protein coding regions, contiguous and in the same reading frame. The cassette may additionally contain at least one additional gene to be cotransformed into the organism. Alternatively, the additional gene(s) can be provided on multiple expression cassettes.

Such an expression cassette is provided with a plurality of restriction sites for insertion of the *AHASL* polynucleotide sequence to be under the transcriptional regulation of the regulatory regions. The expression cassette may additionally contain selectable marker genes.

The expression cassette will include in the 5'-3' direction of transcription, a transcriptional and translational initiation region (i.e., a promoter), an *AHASL* polynucleotide sequence of the invention, and a transcriptional and translational termination region (i.e., termination region) functional in plants. The promoter may be native or analogous, or foreign or heterologous, to the plant host and/or to the *AHASL* polynucleotide sequence of the invention. Additionally, the promoter may be the natural sequence or alternatively a synthetic sequence. Where the promoter is "foreign" or "heterologous" to the plant host, it is intended that the promoter is not found in the native plant into which the promoter is introduced. Where the promoter is "foreign" or "heterologous" to the *AHASL* polynucleotide sequence of the invention, it is intended that the promoter is not the native or naturally occurring promoter for the operably linked *AHASL* polynucleotide sequence of the invention. As used herein, a chimeric gene comprises a coding sequence operably linked to a transcription initiation region that is heterologous to the coding sequence.

While it may be preferable to express the *AHASL* polynucleotides of the invention using heterologous promoters, the native promoter sequences may be used. Such constructs would change expression levels of the AHASL protein in the plant or plant cell. Thus, the phenotype of the plant or plant cell is altered.

The termination region may be native with the transcriptional initiation region, may be native with the operably linked *AHASL* sequence of interest, may be native with the plant host, or may be derived from another source (i.e., foreign or heterologous to the promoter, the *AHASL* polynucleotide sequence of interest, the plant host, or any combination thereof). Convenient termination regions are available from the Ti-plasmid *of A. tumefaciens,* such as the octopine synthase and nopaline synthase termination regions. See also Guerineau et al. (1991) Mol. Gen. Genet. 262:141-144; Proudfoot (1991) Cell 64:671-674; Sanfacon et al. (1991) Genes Dev. 5:141-149; Mogen et al. (1990) Plant Cell 2:1261-1272; Munroe et al. (1990) Gene 91:151-158; Ballas et al. (1989) Nucleic Acids Res. 17:7891-7903; and Joshi et al. (1987) Nucleic Acid Res. 15:9627-9639.

Where appropriate, the gene(s) may be optimized for increased expression in the transformed plant. That is, the genes can be synthesized using plant-preferred codons for improved expression. See, for example, Campbell and Gowri (1990) Plant Physiol. 92:1-11 for a discussion of host-preferred codon usage. Methods are available in the art for synthesizing plant-preferred genes. See, for example, U.S. Patent Nos. 5,380,831, and 5,436,391, and Murray et al. (1989) Nucleic Acids Res. 17:477-498.

Additional sequence modifications are known to enhance gene expression in a cellular host. These include elimination of sequences encoding spurious polyadenylation signals, exon-intron splice site signals, transposon-like repeats, and other such well-characterized sequences that may be deleterious to gene expression. The G-C content of the sequence may be adjusted to levels average for a given cellular host, as calculated by reference to known genes expressed in the host cell. When possible, the sequence is modified to avoid predicted hairpin secondary mRNA structures.

Nucleotide sequences for enhancing gene expression can also be used in the plant expression vectors. These include the introns of the maize AdhI, intronl gene (Callis et al. Genes and Development 1:1183-1200, 1987), and leader sequences, (W-sequence) from the Tobacco Mosaic virus (TMV), Maize Chlorotic Mottle Virus and Alfalfa Mosaic Virus (Gallie et al. Nucleic Acid Res. 15:8693-8711, 1987 and Skuzeski et al. Plant Mol. Biol. 15:65-79, 1990). The first intron from the shrunken-1 locus of maize, has been shown to increase expression of genes in chimeric gene constructs. U.S. Pat. Nos. 5,424,412 and 5,593,874 disclose the use of specific introns in gene expression constructs, and Gallie et al. (Plant Physiol. 106:929-939, 1994) also have shown that introns are useful for regulating gene expression on a tissue specific basis. To further enhance or to optimize AHAS large subunit gene expression, the plant expression vectors of the invention may also contain DNA sequences containing matrix attachment regions (MARs). Plant cells transformed with such modified expression systems, then, may exhibit overexpression or constitutive expression of a nucleotide sequence of the invention.

The expression cassettes may additionally contain 5' leader sequences in the expression cassette construct. Such leader sequences can act to enhance translation. Translation leaders are known in the art and include: picornavirus leaders, for example, EMCV leader (Encephalomyocarditis 5' noncoding region) *(*Elroy-Stein et al. (1989) Proc. Natl. Acad. Sci. USA 86:6126-6130); potyvirus leaders, for example, TEV leader (Tobacco Etch Virus) (Gallie et al. (1995) Gene 165(2):233-238), MDMV leader (Maize Dwarf Mosaic Virus) *(*Virology 154:9-20), and human immunoglobulin heavy-chain binding protein (BiP) (Macejak et al. (1991) Nature 353:90-94); untranslated leader from the coat protein mRNA of alfalfa mosaic virus (AMV RNA 4) (Jobling et al. (1987) Nature 325:622-625); tobacco mosaic virus leader (TMV) (Gallie et al. (1989) in Molecular Biology of RNA, ed. Cech (Liss, New York), pp. 237-256); and maize chlorotic mottle virus leader (MCMV) (Lommel et al. (1991) Virology 81:382-385). See also, Della-Cioppa et al. (1987) Plant Physiol. 84:965-968. Other methods known to enhance translation can also be utilized, for example, introns, and the like.

In preparing the expression cassette, the various DNA fragments may be manipulated, so as to provide for the DNA sequences in the proper orientation and, as appropriate, in the proper reading frame. Toward this end, adapters or linkers may be employed to join the DNA fragments or other manipulations may be involved to provide for convenient restriction sites, removal of superfluous DNA, removal of restriction sites, or the like. For this purpose, *in vitro* mutagenesis, primer repair, restriction, annealing, resubstitutions, e.g., transitions and transversions, may be involved.

A number of promoters can be used in the practice of the invention. The promoters can be selected based on the desired outcome. The nucleic acids can be combined with constitutive, tissue-preferred, or other promoters for expression in plants.

Such constitutive promoters include, for example, the core promoter of the Rsyn7 promoter and other constitutive promoters disclosed in WO 99/43838 and U.S. Patent No. 6,072,050; the core CaMV 35S promoter *(*Odell et al. (1985) Nature 313:810-812); rice actin (McElroy et al. (1990) Plant Cell 2:163-171); ubiquitin (Christensen et al. (1989) Plant Mol. Biol. 12:619-632 and Christensen et al. (1992) Plant Mol. Biol. 18:675-689); pEMU (Last et al. (1991) Theor. Appl. Genet. 81:581-588); MAS *(*Velten et al. (1984) EMBO J. 3:2723-2730); ALS promoter (U.S. Patent No. 5,659,026), and the like. Other constitutive promoters include, for example, U.S. Patent Nos. 5,608;149; 5,608,144; 5,604,121; 5,569,597; 5,466,785; 5,399,680; 5,268,463; 5,608,142; and 6,177,611.

Tissue-preferred promoters can be utilized to target enhanced AHASL1 expression within a particular plant tissue. Such tissue-preferred promoters include, but are not limited to, leaf-preferred promoters, root-preferred promoters, seed-preferred promoters, and stem-preferred promoters. Tissue-preferred promoters include Yamamoto et al. (1997) Plant J. 12(2):255-265; Kawamata et al. (1997) Plant Cell Physiol. 38(7):792-803; Hansen et al. (1997) Mol. Gen Genet. 254(3):337-343; Russell et al. (1997) Transgenic Res. 6(2):157-168; Rinehart et al. (1996) Plant Physiol. 112(3): 1331-1341; Van Camp et al. (1996) Plant Physiol. 112(2):525-535; Canevascini et al. (1996) Plant Physiol. 112(2):513-524; Yamamoto et al. (1994) Plant Cell Physiol. 35(5):773-778; Lam (1994) Results Probl. Cell Differ. 20:181-196; Orozco et al. (1993) Plant Mol Biol. 23(6):1129-1138; Matsuoka et al. (1993) Proc Natl. Acad. Sci. USA 90(20):9586-9590; and Guevara-Garcia et al. (1993) Plant J. 4(3):495-505. Such promoters can be modified, if necessary, for weak expression.

In one embodiment, the nucleic acids of interest are targeted to the chloroplast for expression. In this manner, where the nucleic acid of interest is not directly inserted into the chloroplast, the expression cassette will additionally contain a chloroplast-targeting sequence comprising a nucleotide sequence that encodes a chloroplast transit peptide to direct the gene product of interest to the chloroplasts. Such transit peptides are known in the art. With respect to chloroplast-targeting sequences, "operably linked" means that the nucleic acid sequence encoding a transit peptide *(i.e.,* the chloroplast-targeting sequence) is linked to the *AHASL* polynucleotide of the invention such that the two sequences are contiguous and in the same reading frame. See, for example, Von Heijne et al. (1991) Plant Mol. Biol. Rep. 9:104-126; Clark et al. (1989) J. Biol. Chem. 264:17544-17550; Della-Cioppa et al. (1987) Plant Physiol. 84:965-968; Romer et al. (1993) Biochem. Biophys. Res. Commun. 196:1414-1421; and Shah et al. (1986) Science 233:478-481. While the AHASL proteins of the invention include a native chloroplast transit peptide, any chloroplast transit peptide known in the art can be fused to the amino acid sequence of a mature AHASL protein of the invention by operably linking a choloroplast-targeting sequence to the 5'-end of a nucleotide sequence encoding a mature AHASL protein of the invention.

Chloroplast targeting sequences are known in the art and include the chloroplast small subunit of ribulose-1,5-bisphosphate carboxylase (Rubisco) (de Castro Silva Filho et al. (1996) Plant Mol. Biol. 30:769-780; Schnell et al. (1991) J. Biol. Chem. 266(5):3335-3342); 5-(enolpyruvyl)shikimate-3-phosphate synthase (EPSPS) (Archer et al. (1990) J. Bioenerg. Biomemb. 22(6):789-810); tryptophan synthase (Zhao et al. (1995) J. Biol. Chem. 270(11):6081-6087); plastocyanin (Lawrence et al. (1997) J. Biol. Chem. 272(33):20357-20363); chorismate synthase (Schmidt et al. (1993) J. Biol. Chem. 268(36):27447-27457); and the light harvesting chlorophyll a/b binding protein (LHBP) (Lamppa et al. (1988) J. Biol. Chem. 263:14996-14999). See also Von Heijne et al. (1991) Plant Mol. Biol. Rep. 9:104-126; Clark et al. (1989) J. Biol. Chem. 264:17544-17550; Della-Cioppa et al. (1987) Plant Physiol. 84:965-968; Romer et al. (1993) Biochem. Biophys. Res. Commun. 196:1414-1421; and Shah et al. (1986) Science 233:478-481.

Methods for transformation of chloroplasts are known in the art. See, for example, Svab et al. (1990) Proc. Natl. Acad Sci. USA 87:8526-8530; Svab and Maliga (1993) Proc. Natl. Acad Sci. USA 90:913-917; Svab and Maliga (1993) EMBO J. 12:601-606. The method relies on particle gun delivery of DNA containing a selectable marker and targeting of the DNA to the plastid genome through homologous recombination. Additionally, plastid transformation can be accomplished by transactivation of a silent plastid-borne transgene by tissue-preferred expression of a nuclear-encoded and plastid-directed RNA polymerase. Such a system has been reported in McBride et al. (1994) Proc. Natl. Acad. Sci. USA 91:7301-7305.

The nucleic acids of interest to be targeted to the chloroplast may be optimized for expression in the chloroplast to account for differences in codon usage between the plant nucleus and this organelle. In this manner, the nucleic acids of interest may be synthesized using chloroplast-preferred codons. See, for example, U.S. Patent No. 5,380,831.

As disclosed herein, the AHASL nucleotide sequences of the invention find use in enhancing the herbicide tolerance of plants that comprise in their genomes a gene encoding a herbicide-tolerant AHASL protein. Such a gene may be an endogenous gene or a transgene. Additionally, in certain embodiments, the nucleic acid sequences of the present invention can be stacked with any combination of polynucleotide sequences of interest in order to create plants with a desired phenotype. For example, the polynucleotides of the present invention may be stacked with any other polynucleotides encoding polypeptides having pesticidal and/or insecticidal activity, such as, for example, the *Bacillus thuringiensis* toxin proteins (described in U.S. Patent Nos. 5,366,892; 5,747,450; 5,737,514; 5,723,756; 5,593,881; and Geiser et al. (1986) Gene 48:109). The combinations generated can also include multiple copies of any one of the polynucleotides of interest.

It is recognized that with these nucleotide sequences, antisense constructions, complementary to at least a portion of the messenger RNA (mRNA) for the AHASL polynucleotide sequences can be constructed. Antisense nucleotides are constructed to hybridize with the corresponding mRNA. Modifications of the antisense sequences may be made as long as the sequences hybridize to and interfere with expression of the corresponding mRNA. In this manner, antisense constructions having 70%, preferably 80%, more preferably 85% sequence identity to the corresponding antisensed sequences may be used. Furthermore, portions of the antisense nucleotides may be used to disrupt the expression of the target gene. Generally, sequences of at least 50 nucleotides, 100 nucleotides, 200 nucleotides, or greater may be used.

The nucleotide sequences of the present invention may also be used in the sense orientation to suppress the expression of endogenous genes in plants. Methods for suppressing gene expression in plants using nucleotide sequences in the sense orientation are known in the art. The methods generally involve transforming plants with a DNA construct comprising a promoter that drives expression in a plant operably linked to at least a portion of a nucleotide sequence that corresponds to the transcript of the endogenous gene. Typically, such a nucleotide sequence has substantial sequence identity to the sequence of the transcript of the endogenous gene, preferably greater than about 65% sequence identity, more preferably greater than about 85% sequence identity, most preferably greater than about 95% sequence identity. See, U.S. Patent Nos. 5,283,184 and 5,034,323.

While the herbicide-resistant AHASL1 polynucleotides of the invention find use as selectable marker genes for plant transformation, the expression cassettes of the invention can include another selectable marker gene for the selection of transformed cells. Selectable marker genes, including those of the present invention, are utilized for the selection of transformed cells or tissues. Marker genes include, but are not limited to, genes encoding antibiotic resistance, such as those encoding neomycin phosphotransferase II (NEO) and hygromycin phosphotransferase (HPT), as well as genes conferring resistance to herbicidal compounds, such as glufosinate ammonium, bromoxynil, imidazolinones, and 2,4-dichlorophenoxyacetate (2,4-D). See generally, Yarranton (1992) Curr. Opin. Biotech 3:506-511; Christopherson et al. (1992) Proc. Natl. Acad Sci. USA 89:6314-6318; Yao et al. (1992) Cell 71:63-72; Reznikoff(1992) Mol. Microbiol. 6:2419-2422; Barkley et al. (1980) in The Operon, pp. 177-220; Hu et al. (1987) Cell 48:555-566; Brown et al. (1987) Cell 49:603-612; Figge et al. (1988) Cell 52:713-722; Deuschle et al. (1989) Proc. Natl. Acad Aci. USA 86:5400-5404; Fuerst et al. (1989) Proc. Natl. Acad Sci. USA 86:2549-2553; Deuschle et al. (1990) Science 248:480-483; Gossen (1993) Ph.D. Thesis, University of Heidelberg; Reines et al. (1993) Proc. Natl. Acad Sci. USA 90:1917-1921; Labow et al. (1990) Mol. Cell. Biol. 10:3343-3356; Zambretti et al. (1992) Proc. Natl. Acad. Sci. USA 89:3952-3956; Baim et al. (1991) Proc. Natl. Acad. Sci. USA 88:5072-5076; Wyborski et al. (1991) Nucleic Acids Res. 19:4647-4653; Hillenand-Wissman (1989) Topics Mol. Struc. Biol. 10:143-162; Degenkolb et al. (1991) Antimicrob. Agents Chemother. 35:1591-1595*;* Kleinschnidt et al. (1988) Biochemistry 27:1094-1104; Bonin (1993) Ph.D. Thesis, University of Heidelberg; Gossen et al. (1992) Proc. Natl. Acad. Sci. USA 89:5547-5551; Oliva et al. (1992) Antimicrob. Agents Chemother. 36:913-919; Hlavka et al. (1985) Handbook of Experimental Pharmacology, Vol. 78 ( Springer-Verlag, Berlin); Gill et al. (1988) Nature 334:721-724.

The above list of selectable marker genes is not meant to be limiting. Any selectable marker gene can be used in the present invention.

The isolated polynucleotide molecules comprising nucleotide sequence that encode the AHASL proteins of the invention can be used in vectors to transform plants so that the plants created have enhanced resistant to herbicides, particularly imidazolinone herbicides. The isolated AHASL polynucleotide molecules of the invention can be used in vectors alone or in combination with a nucleotide sequence encoding the small subunit of the AHAS (AHASS) enzyme in conferring herbicide resistance in plants. *See,* U.S. Patent No. 6,348,643.

The invention also relates to a plant expression vector comprising a promoter that drives expression in a plant operably linked to an isolated polynucleotide molecule of the invention. The isolated polynucleotide molecule comprises a nucleotide sequence encoding an AHASL protein, particularly an AHASL protein comprising an amino sequence that is set forth in SEQ ID NO: 2, or a functional fragment and variant thereof. The plant expression vector of the invention does not depend on a particular promoter, only that such a promoter is capable of driving gene expression in a plant cell. Preferred promoters include constitutive promoters and tissue-preferred promoters.

The transformation vectors of the invention can be used to produce plants transformed with a gene of interest. The transformation vector will comprise a selectable marker gene of the invention and a gene of interest to be introduced and typically expressed in the transformed plant. Such a selectable marker gene comprises a herbicide-resistant AHASL polynucleotide of the invention operably linked to a promoter that drives expression in a host cell. For use in plants and plant cells, the transformation vector comprises a selectable marker gene comprising a herbicide-resistant AHASL polynucleotide of the invention operably linked to a promoter that drives expression in a plant cell.

The genes of interest vary depending on the desired outcome. For example, various changes in phenotype can be of interest including modifying the fatty acid composition in a plant, altering the amino acid content of a plant, altering a plant's insect and/or pathogen defense mechanisms, and the like. These results can be achieved by providing expression of heterologous products or increased expression of endogenous products in plants. Alternatively, the results can be achieved by providing for a reduction of expression of one or more endogenous products, particularly enzymes or cofactors in the plant. These changes result in a change in phenotype of the transformed plant.

For example, the genes of interest include insect resistance genes such as, for example, *Bacillus thuringiensis* toxin protein genes (U.S. Patent Nos. 5,366,892; 5,747,450; 5,736,514; 5,723,756; 5,593,881; and Geiser et al. (1986) Gene 48:109).

The AHASL proteins or polypeptides of the invention can be purified from, for example, sunflower plants and can be used in compositions. Also, an isolated polynucleotide molecule encoding an AHASL protein of the invention can be used to express an AHASL protein of the invention in a microbe such as *E*. *coli* or a yeast. The expressed AHASL protein can be purified from extracts of *E. coli* or yeast by any method known to those of ordinary skill in the art.

The invention also relates to a method for creating a transgenic plant that is resistant to herbicides, comprising transforming a plant with a plant expression vector comprising a promoter that drives expression in a plant operably linked to an isolated polynucleotide molecule of the invention. The isolated polynucleotide molecule comprises a nucleotide sequence encoding an AHASL protein of the invention, particularly an AHASL protein comprising: an amino sequence that is set forth in SEQ ID NO: 2, an amino acid sequence encoded by SEQ ID NO: 1, or a functional fragment and variant of said amino acid sequences.

The invention also relates to the non-transgenic sunflower plants, transgenic plants produced by the methods of the invention, and progeny and other descendants of such non-transgenic and transgenic plants, which plants exhibit enhanced or increased resistance to imidazolinone herbicides that interfere with the AHAS enzyme.

The AHASL polynucleotides of the invention encoding herbicide-resistant AHASL proteins, find use in methods for enhancing the resistance of herbicide-tolerant plants. In one embodiment of the invention, the herbicide-tolerant plants comprise a herbicide-tolerant or herbicide-resistant AHASL protein. The herbicide-tolerant plants include both plants transformed with a herbicide-tolerant AHASL nucleotide sequences and plants that comprise in their genomes an endogenous gene that encodes a herbicide-tolerant AHASL protein. Nucleotide sequences encoding herbicide-tolerant AHASL proteins and herbicide-tolerant plants comprising an endogenous gene that encodes a herbicide-tolerant AHASL protein include the polynucleotides and plants of the present invention and those that are known in the art. *See,* for example, U.S. Patent Nos. 5,013,659, 5,731,180, 5,767,361, 5,545,822, 5,736,629, 5,773,703, 5,773,704, 5,952,553 and 6,274,796. Such methods for enhancing the resistance of herbicide-tolerant plants comprise transforming a herbicide-tolerant plant with at least one polynucleotide construction comprising a promoter that drives expression in a plant cell that is operably linked to a herbicide-resistant AHASL polynucleotide of the invention, particularly the polynucleotide encoding a herbicide-resistant AHASL protein set forth in SEQ ID NO: 1, polynucleotides encoding the amino acid sequence set forth in SEQ ID NO: 2, and fragments and variants said polynucleotides that encode polypeptides comprising herbicide-resistant AHAS activity.

Numerous plant transformation vectors and methods for transforming plants are available. *See*, for example, An, G. et al. (1986) Plant Pysiol., 81:301-305; Fry, J., et al. (1987) Plant Cell Rep. 6:321-325; Block, M. (1988) Theor. Appl Genet.76:767-774*;* Hinchee, et al. (1990) Stadler. Genet. Symp.203212.203-212; Cousins, et al. (1991) Aust. J. Plant Physiol. 18:481-494; Chee, P. P. and Slightom, J. L. (1992) Gene. 118:255-260; Christou, et al. (1992) Trends. Biotechnol. 10:239-246; D'Halluin, et al. (1992) Bio/Technol. 10:309-314; Dhir, et al. (1992) Plant Physiol. 99:81-88; Casas et al. (1993) Proc. Nat. Acad Sci. USA 90:11212-11216; Christou, P. (1993) In Vitro Cell. Dev. Biol.-Plant; 29P:119-124; Davies, et al. (1993) Plant Cell Rep. 12:180-183; Dong, J. A. and Mchughen, A. (1993) Plant Sci. 91:139-148; Franklin, C. I. and Trieu, T. N. (1993) Plant. Physiol. 102:167; Golovkin, et al. (1993) Plant Sci. 90:41-52; Guo Chin Sci. Bull. 38:2072-2078; Asano, et al. (1994) Plant Cell Rep. 13; Ayeres N. M. and Park, W. D. (1994) Crit. Rev. Plant. Sci. 13:219-239; Barcelo, et al. (1994) Plant. J. 5:583-592; Becker, et al. (1994) Plant. J. 5:299-307; Borkowska et al. (1994) Acta. Physiol Plant. 16:225-230; Christou, P. (1994) Agro. Food. Ind. Hi Tech. 5: 17-27; Eapen et al. (1994) Plant Cell Rep. 13:582-586; Hartman, et al. (1994) Bio-Technology 12: 919923; Ritala, et al. (1994) Plant. Mol. Biol. 24:317-325; and Wan, Y. C. and Lemaux, P. G. (1994) Plant Physiol. 104:3748.

The methods of the invention involve introducing a polynucleotide construct into a plant. By "introducing" is intended presenting to the plant the polynucleotide construct in such a manner that the construct gains access to the interior of a cell of the plant. The methods of the invention do not depend on a particular method for introducing a polynucleotide construct to a plant, only that the polynucleotide construct gains access to the interior of at least one cell of the plant. Methods for introducing polynucleotide constructs into plants are known in the art including, but not limited to, stable transformation methods, transient transformation methods, and virus-mediated methods.

By "stable transformation" is intended that the polynucleotide construct introduced into a plant integrates into the genome of the plant and is capable of being inherited by progeny thereof. By "transient transformation" is intended that a polynucleotide construct introduced into a plant does not integrate into the genome of the plant.

For the transformation of plants and plant cells, the nucleotide sequences of the invention are inserted using standard techniques into any vector known in the art that is suitable for expression of the nucleotide sequences in a plant or plant cell. The selection of the vector depends on the preferred transformation technique and the target plant species to be transformed. In an embodiment of the invention, an AHASL1 nucleotide sequence is operably linked to a plant promoter that is known for high-level expression in a plant cell, and this construct is then introduced into a plant that is susceptible to an imidazolinone herbicide and a transformed plant is regenerated. The transformed plant is tolerant to exposure to a level of an imidazolinone herbicide that would kill or significantly injure an untransformed plant. This method can be applied to any plant species; however, it is most beneficial when applied to crop plants.

Methodologies for constructing plant expression cassettes and introducing foreign nucleic acids into plants are generally known in the art and have been previously described. For example, foreign DNA can be introduced into plants, using tumor-inducing (Ti) plasmid vectors. Other methods utilized for foreign DNA delivery involve the use of PEG mediated protoplast transformation, electroporation, microinjection whiskers, and biolistics or microprojectile bombardment for direct DNA uptake. Such methods are known in the art. (U.S. Pat. No. 5,405,765 to Vasil et al.; Bilang et al. (1991) Gene 100: 247-250; Scheid et al., (1991) Mol. Gen. Genet., 228: 104-112; Guerche et al., (1987) Plant Science 52: 111-116; Neuhause et al., (1987) Theor. Appl Genet. 75: 30-36; Klein et al., (1987) Nature 327: 70-73; Howell et al., (1980) Science 208:1265; Horsch et al., (1985) Science 227: 1229-1231; DeBlock et al., (1989) Plant Physiology 91: 694-701; Methods for Plant Molecular Biology (Weissbach and Weissbach, eds.) Academic Press, Inc. (1988) and Methods in Plant Molecular Biology (Schuler and Zielinski, eds.) Academic Press, Inc. (1989). The method of transformation depends upon the plant cell to be transformed, stability of vectors used, expression level of gene products and other parameters.

Other suitable methods of introducing nucleotide sequences into plant cells and subsequent insertion into the plant genome include microinjection as Crossway et al. (1986) Biotechniques 4:320-334, electroporation as described by Riggs et al. (1986) Proc. Natl. Acad. Sci. USA 83:5602-5606, *Agrobacterium*-mediated transformation as described by Townsend et al., U.S. Patent No. 5,563,055, Zhao et al., U.S. Patent No. 5,981,840, direct gene transfer as described by Paszkowski et al. (1984) EMBO J. 3:2717-2722, and ballistic particle acceleration as described in, for example, Sanford et al., U.S. Patent No. 4,945,050; Tomes et al., U.S. Patent No. 5,879,918; Tomes et al., U.S. Patent No. 5,886,244; Bidney et al., U.S. Patent No. 5,932,782; Tomes et al. (1995) "Direct DNA Transfer into Intact Plant Cells via Microprojectile Bombardment," in Plant Cell, Tissue, and Organ Culture: Fundamental Methods, ed. Gamborg and Phillips (Springer-Verlag, Berlin); McCabe et al. (1988) Biotechnology 6:923-926); and Lec 1 transformation (WO 00/28058). *Also see,* Weissinger et al. (1988) Ann. Rev. Genet. 22:421-477; Sanford et al. (1987) Particulate Science and Technology 5:27-37 (onion); Christou et al. (1988) Plant Physiol. 87:671-674 (soybean); McCabe et al. (1988) Bio/Technology 6:923-926 (soybean); Finer and McMullen (1991) In Vitro Cell Dev. Biol. 27P:175-182 (soybean); Singh et al. (1998) Theor. Appl. Genet. 96:319-324 (soybean); Datta et al. (1990) Biotechnology 8:736-740 (rice); Klein et al. (1988) Proc. Natl. Acad. Sci. USA 85:4305-4309 (maize); Klein et al. (1988) Biotechnology 6:559-563 (maize); Tomes, U.S. Patent No. 5,240,855; Buising et al., U.S. Patent Nos. 5,322,783 and 5,324,646; Tomes et al. (1995) "Direct DNA Transfer into Intact Plant Cells via Microprojectile Bombardment," in Plant Cell, Tissue, and Organ Culture: Fundamental Methods, ed. Gamborg (Springer-Verlag, Berlin) (maize); Klein et al. (1988) Plant Physiol. 91:440-444 (maize); Fromm et al. (1990) Biotechnology 8:833-839 (maize); Hooykaas-Van Slogteren et al. (1984) Nature (London) 311:763-764; Bowen et al., U.S. Patent No. 5,736,369 (cereals); Bytebier et al. (1987) Proc. Natl. Acad Sci. USA 84:5345-5349 (Liliaceae); De Wet et al. (1985) in The Experimental Manipulation of Ovule Tissues, ed. Chapman et al. (Longman, New York), pp. 197-209 (pollen); Kaeppler et al. (1990) Plant Cell Reports 9:415-418 and Kaeppler et al. (1992) Theor. Appl. Genet. 84:560-566 (whisker-mediated transformation); D'Halluin et al. (1992) Plant Cell 4:1495-1505 (electroporation); Li et al. (1993) Plant Cell Reports 12:250-255 and Christou and Ford (1995) Annals of Botany 75:407-413 (rice); Osjoda et al. (1996) Nature Biotechnology 14:745-750 (maize via *Agrobacterium tumefaciens*).

The polynucleotides of the invention may be introduced into plants by contacting plants with a virus or viral nucleic acids. Generally, such methods involve incorporating a polynucleotide construct of the invention within a viral DNA or RNA molecule. It is recognized that the an AHASL protein of the invention may be initially synthesized as part of a viral polyprotein, which later may be processed by proteolysis *in vivo* or *in vitro* to produce the desired recombinant protein. Further, it is recognized that promoters of the invention also encompass promoters utilized for transcription by viral RNA polymerases. Methods for introducing polynucleotide constructs into plants and expressing a protein encoded therein, involving viral DNA or RNA molecules, are known in the art. See, for example, U.S. Patent Nos. 5,889,191, 5,889,190, 5,866,785, 5,589,367 and 5,316,931.

The cells that have been transformed may be grown into plants in accordance with conventional ways. See, for example, McCormick et al. (1986) Plant Cell Reports 5:81-84. These plants may then be grown, and either pollinated with the same transformed strain or different strains, and the resulting hybrid having constitutive expression of the desired phenotypic characteristic identified. Two or more generations may be grown to ensure that expression of the desired phenotypic characteristic is stably maintained and inherited and then seeds harvested to ensure expression of the desired phenotypic characteristic has been achieved. In this manner, the present invention provides transformed seed (also referred to as "transgenic seed") having a polynucleotide construct of the invention, for example, an expression cassette of the invention, stably incorporated into their genome.

The present invention may be used for transformation of any plant species, including, but not limited to, monocots and dicots. Examples of plant species of interest include, but are not limited to, corn or maize (*Zea mays*), *Brassica* sp. (e.g., *B. napus, B. rapa, B. juncea*), particularly those *Brassica* species useful as sources of seed oil, alfalfa *(Medicago sativa*), rice (*Oryza sativa*), rye *(Secale cereale),* sorghum *(Sorghum bicolor, Sorghum vulgare),* millet (e.g.; pearl millet *(Pennisetum glaucum),* proso millet *(Panicum miliaceum*), foxtail millet (*Setaria italica*), finger millet *(Eleusine coracana*)), sunflower (*Helianthus annuus*), safflower *(Carthamus tinctorius*), wheat *(Triticum aestivum, T. Turgidum ssp. durum),* soybean *(Glycine max),* tobacco (*Nicotiana tabacum),* potato *(Solanum tuberosum),* peanuts *(Arachis hypogaea*), cotton *(Gossypium barbadense, Gossypium hirsutum),* sweet potato *(Ipomoea batatus*), cassava (*Manihot esculenta),* coffee (*Coffea* spp.), coconut (*Cocos nucifera*), pineapple *(Ananas comosus),* citrus trees *(Citrus* spp.), cocoa (*Theobroma cacao*), tea *(Camellia sinensis),* banana (*Musa* spp.), avocado (*Persea americana*), fig *(Ficus casica*), guava *(Psidium guajava*), mango (*Mangifera indica*), olive (*Olea europaea*), papaya (*Carica papaya*), cashew *(Anacardium occidentale*), macadamia (*Macadamia integrifolia*), almond *(Prunus amygdalus*), sugar beets (*Beta vulgaris*), sugarcane *(Saccharum* spp.), oats, barley, vegetables, ornamentals, and conifers. Preferably, plants of the present invention are crop plants (for example, sunflower, *Brassica sp.,* cotton, sugar, beet, soybean, peanut, alfalfa, safflower, tobacco, corn, rice, wheat, rye, barley triticale, sorghum, millet, etc.).

The herbicide resistant plants of the invention find use in methods for controlling weeds. Thus, the present invention further provides a method for controlling weeds in the vicinity of a herbicide-resistant plant of the invention. The method comprises applying an effective amount of a herbicide to the weeds and to the herbicide-resistant plant, wherein the plant has increased resistance to at least one imidazolinone herbicide when compared to a wild-type plant. In such a method for controlling weeds, the herbicide-resistant plants of the invention are preferably crop plants, including, but not limited to, sunflower, alfalfa, *Brassica sp.*, soybean, cotton, safflower, peanut, tobacco, tomato, potato, wheat, rice, maize, sorghum, barley, rye, millet, and sorghum.

By providing plants having increased resistance to imidazolinone herbicides, a wide variety of formulations can be employed for protecting plants from weeds, so as to enhance plant growth and reduce competition for nutrients. A herbicide can be used by itself for pre-emergence, post-emergence, pre-planting and at planting control of weeds in areas surrounding the plants described herein or an imidazolinone herbicide formulation can be used that contains other additives. The herbicide can also be used as a seed treatment. That is an effective concentration or an effective amount of the herbicide, or a composition comprising an effective concentration or an effective amount of the herbicide can be applied directly to the seeds prior to or during the sowing of the seeds. Additives found in an imidazolinone herbicide formulation or composition include other herbicides, detergents, adjuvants, spreading agents, sticking agents, stabilizing agents, or the like. The herbicide formulation can be a wet or dry preparation and can include, but is not limited to, flowable powders, emulsifiable concentrates and liquid concentrates. The herbicide and herbicide formulations can be applied in accordance with conventional methods, for example, by spraying, irrigation, dusting, coating, and the like.

The present invention provides non-transgenic and transgenic seeds with increased resistance to at least one imidazolinone herbicide. Such seeds include, for example, non-transgenic sunflower seeds comprising the herbicide-resistance characteristics of the plant with ATCC Patent Deposit Number PTA-6325, and transgenic seeds comprising a polynucleotide molecule of the invention that encodes a herbicide-resistant AHASL1 protein.

The present invention provides methods for producing a herbicide-resistant plant, particularly a herbicide-resistant sunflower plant, through conventional plant breeding involving sexual reproduction. The methods comprise crossing a first plant that is resistant to a herbicide to a second plant that is not resistant to the herbicide. The first plant can be any of the herbicide resistant plants of the present invention including, for example, transgenic plants comprising at least one of the polynucleotides of the present invention that encode a herbicide resistant AHASL and non-transgenic sunflower plants that comprise the herbicide-resistance characteristics of the sunflower plant with ATCC Patent Deposit Number PTA-6325. The second plant can be any plant that is capable of producing viable progeny plants (i.e., seeds) when crossed with the first plant. Typically, but not necessarily, the first and second plants are of the same species. The methods of the invention can further involve one or more generations of backcrossing the progeny plants of the first cross to a plant of the same line or genotype as either the first or second plant. Alternatively, the progeny of the first cross or any subsequent cross can be crossed to a third plant that is of a different line or genotype than either the first or second plant. The methods of the invention can additionally involve selecting plants that comprise the herbicide resistance characteristics of the first plant.

The present invention further provides methods for increasing the herbicide-resistance of a plant, particularly a herbicide-resistant sunflower plant, through conventional plant breeding involving sexual reproduction. The methods comprise crossing a first plant that is resistant to a herbicide to a second plant that may or may not be resistant to the herbicide or may be resistant to different herbicide or herbicides than the first plant. The first plant can be any of the herbicide resistant plants of the present invention including, for example, transgenic plants comprising at least one of the polynucleotides of the present invention that encode a herbicide resistant AHASL and non-transgenic sunflower plants that comprise the herbicide-resistance characteristics of the sunflower plant with ATCC Patent Deposit Number PTA-6325. The second plant can be any plant that is capable of producing viable progeny plants (i.e., seeds) when crossed with the first plant. Typically, but not necessarily, the first and second plants are of the same species. The progeny plants produced by this method of the present invention have increased resistance to a herbicide when compared to either the first or second plant or both. When the first and second plants are resistant to different herbicides, the progeny plants will have the combined herbicide resistance characteristics of the first and second plants. The methods of the invention can further involve one or more generations of backcrossing the progeny plants of the first cross to a plant of the same line or genotype as either the first or second plant. Alternatively, the progeny of the first cross or any subsequent cross can be crossed to a third plant that is of a different line or genotype than either the first or second plant. The methods of the invention can additionally involve selecting plants that comprise the herbicide resistance characteristics of the first plant, the second plant, or both the first and the second plant.

The plants of the present invention can be transgenic or non-transgenic. An example of a non-transgenic sunflower plant having increased resistance to imidazolinone and/or sulfonylurea herbicides includes the sunflower plant (MUT9) having ATCC Patent Deposit No. PTA-6325; or mutant, recombinant, or a genetically engineered derivative of the plant having ATCC Patent Deposit No. PTA-6325; or of any progeny of the plant having ATCC Patent Deposit No. PTA-6325; or a plant that is a progeny of any of these plants; or a plant that comprises the herbicide resistance characteristics of the plant having ATCC Patent Deposit No. PTA-6325.

The present invention also provides plants, plant organs, plant tissues, plant cells, seeds, and non-human host cells that are transformed with the at least one polynucleotide molecule, expression cassette, or transformation vector of the invention. Such transformed plants, plant organs, plant tissues, plant cells, seeds, and non-human host cells have enhanced tolerance or resistance to at least one herbicide, at levels of the herbicide that kill or inhibit the growth of an untransformed plant, plant tissue, plant cell, or non-human host cell, respectively. Preferably, the transformed plants, plant tissues, plant cells, and seeds of the invention are *Arabidopsis thaliana* and crop plants.

The present invention provides methods that involve the use of an AHAS-inhibiting herbicide. In these methods, the AHAS-inhibiting herbicide can be applied by any method known in the art including, but not limited to, seed treatment, soil treatment, and foliar treatment.

Prior to application, the AHAS-inhibiting herbicide can be converted into the customary formulations, for example solutions, emulsions, suspensions, dusts, powders, pastes and granules. The use form depends on the particular intended purpose; in each case, it should ensure a fine and even distribution of the compound according to the invention.

The formulations are prepared in a known manner (see e.g. for review US 3,060,084, EP-A 707 445 (for liquid concentrates), Browning, "Agglomeration", Chemical Engineering, Dec. 4, 1967, 147-48, Perry's Chemical Engineer's Handbook, 4th Ed., McGraw-Hill, New York, 1963, pages 8-57 and et seq. WO 91/13546, US 4,172,714, US 4,144,050, US 3,920,442, US 5,180,587, US 5,232,701, US 5,208,030, GB 2,095,558, US 3,299,566, Klingman, Weed Control as a Science, John Wiley and Sons, Inc., New York, 1961, Hance et al., Weed Control Handbook, 8th Ed., Blackwell Scientific Publications, Oxford, 1989 and Mollet, H., Grubemann, A., Formulation technology, Wiley VCH Verlag GmbH, Weinheim (Germany), 2001, 2. D. A. Knowles, Chemistry and Technology of Agrochemical Formulations, Kluwer Academic Publishers, Dordrecht, 1998 (ISBN 0-7514-0443-8), for example by extending the active compound with auxiliaries suitable for the formulation of agrochemicals, such as solvents and/or carriers, if desired emulsifiers, surfactants and dispersants, preservatives, antifoaming agents, anti-freezing agents, for seed treatment formulation also optionally colorants and/or binders and/or gelling agents.

Examples of suitable solvents are water, aromatic solvents (for example Solvesso products, xylene), paraffins (for example mineral oil fractions), alcohols (for example methanol, butanol, pentanol, benzyl alcohol), ketones (for example cyclohexanone, gamma-butyrolactone), pyrrolidones (NMP, NOP), acetates (glycol diacetate), glycols, fatty acid dimethylamides, fatty acids and fatty acid esters. In principle, solvent mixtures may also be used.

Examples of suitable carriers are ground natural minerals (for example kaolins, clays, talc, chalk) and ground synthetic minerals (for example highly disperse silica, silicates).

Suitable emulsifiers are nonionic and anionic emulsifiers (for example polyoxyethylene fatty alcohol ethers, alkylsulfonates and arylsulfonates).

Examples of dispersants are lignin-sulfite waste liquors and methylcellulose.

Suitable surfactants used are alkali metal, alkaline earth metal and ammonium salts of lignosulfonic acid, naphthalenesulfonic acid, phenolsulfonic acid, dibutylnaphthalenesulfonic acid, alkylarylsulfonates, alkyl sulfates, alkylsulfonates, fatty alcohol sulfates, fatty acids and sulfated fatty alcohol glycol ethers, furthermore condensates of sulfonated naphthalene and naphthalene derivatives with formaldehyde, condensates of naphthalene or of naphthalenesulfonic acid with phenol and formaldehyde, polyoxyethylene octylphenol ether, ethoxylated isooctylphenol, octylphenol, nonylphenol, alkylphenol polyglycol ethers, tributylphenyl polyglycol ether, tristearylphenyl polyglycol ether, alkylaryl polyether alcohols, alcohol and fatty alcohol ethylene oxide condensates, ethoxylated castor oil, polyoxyethylene alkyl ethers, ethoxylated polyoxypropylene, lauryl alcohol polyglycol ether acetal, sorbitol esters, lignosulfite waste liquors and methylcellulose.

Substances which are suitable for the preparation of directly sprayable solutions, emulsions, pastes or oil dispersions are mineral oil fractions of medium to high boiling point, such as kerosene or diesel oil, furthermore coal tar oils and oils of vegetable or animal origin, aliphatic, cyclic and aromatic hydrocarbons, for example toluene, xylene, paraffin, tetrahydronaphthalene, alkylated naphthalenes or their derivatives, methanol, ethanol, propanol, butanol, cyclohexanol, cyclohexanone, isophorone, highly polar solvents, for example dimethyl sulfoxide, N-methylpyrrolidone or water.

Also anti-freezing agents such as glycerin, ethylene glycol, propylene glycol and bactericides such as can be added to the formulation.

Suitable antifoaming agents are for example antifoaming agents based on silicon or magnesium stearate.

Suitable preservatives are for example Dichlorophen und enzylalkoholhemiformal.

Seed Treatment formulations may additionally comprise binders and optionally colorants.

Binders can be added to improve the adhesion of the active materials on the seeds after treatment. Suitable binders are block copolymers EO/PO surfactants but also polyvinylalcoholsl, polyvinylpyrrolidones, polyacrylates, polymethacrylates, polybutenes, polyisobutylenes, polystyrene, polyethyleneanines, polyethyleneamides, polyethyleneimines (Lupasol®, Polymin®), polyethers, polyurethans, polyvinylacetate, tylose and copolymers derived from these polymers.

Optionally, also colorants can be included in the formulation. Suitable colorants or dyes for seed treatment formulations are Rhodamin B, C.I. Pigment Red 112, C.I. Solvent Red 1, pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

An examples of a suitable gelling agent is carrageen (Satiagel^{®})

Powders, materials for spreading, and dustable products can be prepared by mixing or concomitantly grinding the active substances with a solid carrier.

Granules, for example coated granules, impregnated granules and homogeneous granules, can be prepared by binding the active compounds to solid carriers. Examples of solid carriers are mineral earths such as silica gels, silicates, talc, kaolin, attaclay, limestone, lime, chalk, bole, loess, clay, dolomite, diatomaceous earth, calcium sulfate, magnesium sulfate, magnesium oxide, ground synthetic materials, fertilizers, such as, for example, ammonium sulfate, ammonium phosphate, ammonium nitrate, ureas, and products of vegetable origin, such as cereal meal, tree bark meal, wood meal and nutshell meal, cellulose powders and other solid carriers.

In general, the formulations comprise from 0.01 to 95% by weight, preferably from 0.1 to 90% by weight, of the AHAS-inhibiting herbicide. In this case, the AHAS-inhibiting herbicides are employed in a purity of from 90% to 100% by weight, preferably 95% to 100% by weight (according to NMR spectrum). For seed treatment purposes, respective formulations can be diluted 2-10 fold leading to concentrations in the ready to use preparations of 0.01 to 60% by weight active compound by weight, preferably 0.1 to 40% by weight.

The AHAS-inhibiting herbicide can be used as such, in the form of their formulations or the use forms prepared therefrom, for example in the form of directly sprayable solutions, powders, suspensions or dispersions, emulsions, oil dispersions, pastes, dustable products, materials for spreading, or granules, by means of spraying, atomizing, dusting, spreading or pouring. The use forms depend entirely on the intended purposes; they are intended to ensure in each case the finest possible distribution of the AHAS-inhibiting herbicide according to the invention.

Aqueous use forms can be prepared from emulsion concentrates, pastes or wettable powders (sprayable powders, oil dispersions) by adding water. To prepare emulsions, pastes or oil dispersions, the substances, as such or dissolved in an oil or solvent, can be homogenized in water by means of a wetter, tackifier, dispersant or emulsifier. However, it is also possible to prepare concentrates composed of active substance, wetter, tackifier, dispersant or emulsifier and, if appropriate, solvent or oil, and such concentrates are suitable for dilution with water.

The active compound concentrations in the ready-to-use preparations can be varied within relatively wide ranges. In general, they are from 0.0001 to 10%, preferably from 0.01 to 1% per weight.

The AHAS-inhibiting herbicide may also be used successfully in the ultra-low-volume process (ULV), it being possible to apply formulations comprising over 95% by weight of active compound, or even to apply the active compound without additives.

The following are examples of formulations:
1. Products for dilution with water for foliar applications. For seed treatment purposes, such products may be applied to the seed diluted or undiluted.
   A) Water-soluble concentrates (SL, LS)
      Ten parts by weight of the AHAS-inhibiting herbicide are dissolved in 90 parts by weight of water or a water-soluble solvent. As an alternative, wetters or other auxiliaries are added. The AHAS-inhibiting herbicide dissolves upon dilution with water, whereby a formulation with 10 % (w/w) of AHAS-inhibiting herbicide is obtained.
   B) Dispersible concentrates (DC)
      Twenty parts by weight of the AHAS-inhibiting herbicide are dissolved in 70 parts by weight of cyclohexanone with addition of 10 parts by weight of a dispersant, for example polyvinylpyrrolidone. Dilution with water gives a dispersion, whereby a formulation with 20% (w/w) of AHAS-inhibiting herbicide is obtained.
   C) Emulsifiable concentrates (EC)
      Fifteen parts by weight of the AHAS-inhibiting herbicide are dissolved in 7 parts by weight of xylene with addition of calcium dodecylbenzenesulfonate and castor oil ethoxylate (in each case 5 parts by weight). Dilution with water gives an emulsion, whereby a formulation with 15% (w/w) of AHAS-inhibiting herbicide is obtained.
   D) Emulsions (EW, EO, ES)
      Twenty-five parts by weight of the AHAS-inhibiting herbicide are dissolved in 35 parts by weight of xylene with addition of calcium dodecylbenzenesulfonate and castor oil ethoxylate (in each case 5 parts by weight). This mixture is introduced into 30 parts by weight of water by means of an emulsifier machine (e.g. Ultraturrax) and made into a homogeneous emulsion. Dilution with water gives an emulsion, whereby a formulation with 25% (w/w) of AHAS-inhibiting herbicide is obtained.
   E) Suspensions (SC, OD, FS)
      In an agitated ball mill, 20 parts by weight of the AHAS-inhibiting herbicide are comminuted with addition of 10 parts by weight of dispersants, wetters and 70 parts by weight of water or of an organic solvent to give a fine AHAS-inhibiting herbicide suspension. Dilution with water gives a stable suspension of the AHAS-inhibiting herbicide, whereby a formulation with 20% (w/w) of AHAS-inhibiting herbicide is obtained.
   F) Water-dispersible granules and water-soluble granules (WG, SG)
      Fifty parts by weight of the AHAS-inhibiting herbicide are ground finely with addition of 50 parts by weight of dispersants and wetters and made as water-dispersible or water-soluble granules by means of technical appliances (for example extrusion, spray tower, fluidized bed). Dilution with water gives a stable dispersion or solution of the AHAS-inhibiting herbicide, whereby a formulation with 50% (w/w) of AHAS-inhibiting herbicide is obtained.
   G) Water-dispersible powders and water-soluble powders (WP, SP, SS, WS)
      Seventy-five parts by weight of the AHAS-inhibiting herbicide are ground in a rotor-stator mill with addition of 25 parts by weight of dispersants, wetters and silica gel. Dilution with water gives a stable dispersion or solution of the AHAS-inhibiting herbicide, whereby a formulation with 75% (w/w) of AHAS-inhibiting herbicide is obtained.
   I) Gel-Formulation (GF)
      In an agitated ball mill, 20 parts by weight of the AHAS-inhibiting herbicide are comminuted with addition of 10 parts by weight of dispersants, 1 part by weight of a gelling agent wetters and 70 parts by weight of water or of an organic solvent to give a fine AHAS-inhibiting herbicide suspension. Dilution with water gives a stable suspension of the AHAS-inhibiting herbicide, whereby a formulation with 20% (w/w) of AHAS-inhibiting herbicide is obtained. This gel formulation is suitable for us as a seed treatment.
2. Products to be applied undiluted for foliar applications. For seed treatment purposes, such products may be applied to the seed diluted.
   A) Dustable powders (DP, DS)
      Five parts by weight of the AHAS-inhibiting herbicide are ground finely and mixed intimately with 95 parts by weight of finely divided kaolin. This gives a dustable product having 5% (w/w) of AHAS-inhibiting herbicide.
   B) Granules (GR, FG, GG, MG)
      One-half part by weight of the AHAS-inhibiting herbicide is ground finely and associated with 95.5 parts by weight of carriers, whereby a formulation with 0.5% (w/w) of AHAS-inhibiting herbicide is obtained. Current methods are extrusion, spray-drying or the fluidized bed. This gives granules to be applied undiluted for foliar use.

Conventional seed treatment formulations include for example flowable concentrates FS, solutions LS, powders for dry treatment DS, water dispersible powders for slurry treatment WS, water-soluble powders SS and emulsion ES and EC and gel formulation GF. These formulations can be applied to the seed diluted or undiluted. Application to the seeds is carried out before sowing, either directly on the seeds.

In a preferred embodiment a FS formulation is used for seed treatment. Typcially, a FS formulation may comprise 1-800 g/l of active ingredient, 1-200 g/l Surfactant, 0 to 200 g/l antifreezing agent, 0 to 400 g/l of binder, 0 to 200 g/l of a pigment and up to 1 liter of a solvent, preferably water.

The present invention provides non-transgenic and transgenic seeds of the herbicide-resistant plants of the present invention. Such seeds include, for example, non-transgenic sunflower seeds comprising the herbicide-resistance characteristics of the plant with ATCC Patent Deposit Number PTA-6325, and transgenic seeds comprising a polynucleotide molecule of the invention that encodes a herbicide-resistant AHASL1 protein.

For seed treatment, seeds of the herbicide resistant plants according of the present invention are treated with herbicides, preferably herbicides selected from the group consisting of AHAS-inhibiting herbicides such as amidosulfuron, azimsulfuron, bensulfuron, chlorimuron, chlorsulfuron, cinosulfuron, cyclosulfamuron, ethametsulfuron, ethoxysulfuron, flazasulfuron, flupyrsulfuron, foramsulfuron, halosulfuron, imazosulfuron, iodosulfuron, mesosulfuron, metsulfuron, nicosulfuron, oxasulfuron, primisulfuron, prosulfuron, pyrazosulfuron, rimsulfuron, sulfometuron, sulfosulfuron, thifensulfuron, triasulfuron, tribenuron, trifloxysulfuron, triflusulfuron, tritosulfuron, imazamethabenz, imazamox, imazapic, imazapyr, imazaquin, imazethapyr, cloransulam, diclosulam, florasulam, flumetsulam, metosulam, penoxsulam, bispyribac, pyriminobac, propoxycarbazone, flucarbazone, pyribenzoxim, pyriftalid, pyrithiobac, and mixtures thereof, or with a formulation comprising a AHAS-inhibiting herbicide.

The term seed treatment comprises all suitable seed treatment techniques known in the art, such as seed dressing, seed coating, seed dusting, seed soaking, and seed pelleting.

Soil can be treated by the application, in particular into the seed drill: either of a granular formulation containing the AHAS-inhibiting herbicide as a composition/formulation (e.g .a granular formulation, with optionally one or more solid or liquid, agriculturally acceptable carriers and/or optionally with one or more agriculturally acceptable surfactants. This method is advantageously employed, for example, in seedbeds of cereals, maize, cotton, and sunflower.

Seeds can be coated with or contained with a seed treatment formulation comprising at least one ALS inhibitor selected from the group consisting of amidosulfuron, azimsulfuron, bensulfuron, chlorimuron, chlorsulfuron, cinosulfuron, cyclosulfamuron, ethametsulfuron, ethoxysulfuron, flazasulfuron, flupyrsulfuron, foramsulfuron, halosulfuron, imazosulfuron, iodosulfuron, mesosulfuron, metsulfuron, nicosulfuron, oxasulfuron, primisulfuron, prosulfuron, pyrazosulfuron, rimsulfuron, sulfometuron, sulfosulfuron, thifensulfuron, triasulfuron, tribenuron, trifloxysulfuron, triflusulfuron, tritosulfuron, imazamethabenz, imazamox, imazapic, imazapyr, imazaquin, imazethapyr, cloransulam, diclosulam, florasulam, flumetsulam, metosulam, penoxsulam, bispyribac, pyriminobac, propoxycarbazone, flucarbazone, pyribenzoxim, pyriftalid and pyrithiobac.

The term seed embraces seeds and plant propagules of all kinds including but not limited to true seeds, seed pieces, suckers, corms, bulbs, fruit, tubers, grains, cuttings, cut shoots and the like and means in a preferred embodiment true seeds.

The term "coated with and/or containing" generally signifies that the active ingredient is for the most part on the surface of the propagation product at the time of application, although a greater or lesser part of the ingredient may penetrate into the propagation product, depending on the method of application. When the said propagation product is (re)planted, it may absorb the active ingredient.

The seed treatment application with the AHAS-inhibiting herbicide or with a formulation comprising the AHAS-inhibiting herbicide is carried out by spraying or dusting the seeds before sowing of the plants and before emergence of the plants.

In the treatment of seeds, the corresponding formulations are applied by treating the seeds with an effective amount of the AHAS-inhibiting herbicide or a formulation comprising the AHAS-inhibiting herbicide. Herein, the application rates are generally from 0.1 g to 10 kg of the a.i. (or of the mixture of a.i. or of the formulation) per 100 kg of seed, preferably from 1 g to 5 kg per 100 kg of seed, in particular from 1 g to 2.5 kg per 100 kg of seed. For specific crops such as lettuce the rate can be higher.

The present invention provides a method for combating undesired vegetation or controlling weeds comprising contacting the seeds of the resistant plants according to the present invention before sowing and/or after pregermination with an AHAS-inhibiting herbicide. The method can further comprise sowing the seeds, for example, in soil in a field or in a potting medium in greenhouse. The method finds particular use in combating undesired vegetation or controlling weeds in the immediate vicinity of the seed.

The control of undesired vegetation is understood as meaning the killing of weeds and/or otherwise retarding or inhibiting the normal growth of the weeds. Weeds, in the broadest sense, are understood as meaning all those plants which grow in locations where they are undesired.

The weeds include, for example, dicotyledonous and monocotyledonous weeds. Dicotyledonous weeds include, but are not limited to, weeds of the genera: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, and Taraxacum. Monocotyledonous weeds include, but are not limited to, weeds of of the genera: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristyslis, Sagittaria, Eleocharis, Scirpus, Paspalum, Ischaemum, Sphenoclea, Dactyloctenium, Agrostis, Alopecurus, and Apera.

In addition, the weeds can include, for example, crop plants that are growing in an undesired location. For example, a volunteer maize plant that is in a field that predominantly comprises soybean plants can be considered a weed, if the maize plant is undesired in the field of soybean plants.

The articles "a" and "an" are used herein to refer to one or more than one (i.e., to at least one) of the grammatical object of the article. By way of example, "an element" means one or more elements.

As used herein, the word "comprising," or variations such as "comprises" or "comprising," will be understood to imply the inclusion of a stated element, integer or step, or group of elements, integers or steps, but not the exclusion of any other element, integer or step, or group of elements, integers or steps.

The following examples are offered by way of illustration and not by way of limitation.

### EXAMPLE 1: Mutagenesis of Helianthus annuus Line RHA266 and Selection of Imidazolinone-Resistant Plants

In the spring of growing season 1, sunflower plants *(Helianthus annuus*) of the line RHA266 were treated with ethyl methanesulfonate (EMS, also referred to as methanesulfonic acid ethyl ester). EMS is a known mutagen that typically induces G·C-to-A·T transitions in DNA (Jander et al. (2003) Plant Physiol. 131:139-146*).* Two separate experiments were conducted, which differed by planting date and EMS concentrations used. In the first experiment, two concentrations of EMS were used. Plants were treated with a solution comprising 0.5%, or 5%, (*w*/*v*) EMS. For each EMS treatment, 21 rows of seeds were sown outdoors at the Advanta Semillas Biotech Research Station in Balcarce, BsAs, Argentina.

In the second experiment, 42 rows of line RHA266 sunflower seeds were sown outdoors at the Advanta Semillas Biotech Research Station, BsAs, Argentina one week after the seeds were sown in the first experiment. Of these 42 rows, 21 rows were treated with 10% EMS and 21 rows were treated with 15% EMS, as described above.

For each of the experiments, all M₀ plants were bagged prior to flowering in order to ensure that the resulting M1 seeds were the product of self-pollination. The seed heads from each EMS treatment were harvested and threshed in bulk. The following growing season, the mutated M₁ seeds from plants that were treated with 0.5%, 5.0%, 10.0% or 15.0% EMS were sown outdoors with each treatment in a separate plot. Twenty nine days later, when the plants were at the 2-4 leaf pair developmental stage, all of the EMS-treated plants were sprayed with 2X of Sweeper 70DG (100 g a.i./ha). The active ingredient in Sweeper is imazamox. After the herbicide spraying, a total of 14 plants survived and were selected as putative resistant. The distribution of resistant plants per EMS treatment is indicated in Table 1.

**Table 1. Number of M₁ Imidazolinone-Resistant Sunflower Plants Recovered from Each EMS Treatment**

| EMS Concentration (%) | No. of Resistant Plants Recovered |
|---|---|
| 0.5 | 4 |
| 5.0 | 6 |
| 10 | 4 |
| 15 | 0 |

Tissue samples were taken from each individual surviving M₁ plant and DNA from each sample was extracted for PCR amplification and sequencing studies described below in Example 2.

The 14 putative resistant plants (Table 1) were transplanted to pots, placed in the greenhouse, and allowed to grow to maturity. These plants produced seed (M₂), and the seed was harvested. Shortly thereafter, seed of two of these M_{1:2} families were sown in separate pots in the greenhouse.

### EXAMPLE 2: PCR Amplification and Sequencing of Sunflower Polynucleotides Encoding Imidazolinone-Resistant and Wild-Type AHASL1 Proteins

To determine the origin of the imidazolinone tolerance in the sunflower plants of Example, polymerase chain reaction (PCR) amplification of genomic DNA and DNA sequencing were employed to determine the origin of the imidazolinone tolerance described above in Example 1.

From an M₁ plant designated as MUT9, genomic DNA was isolated. Genomic DNA was also isolated from tissue of a wild-type RHA266 plant. The isolated DNA samples from MUT9 and RHA266 were each diluted to a stock concentration of 100ng/µL for use as template DNA for PCR amplifications. The entire coding region of the sunflower *AHASL1* gene was amplified from the MUT9 and RHA266 DNA samples. The specific primers used to obtain each amplicon are set forth in Table 2.

**Table 2. PCR Primers for Amplifying the Coding Region of the Sunflower AHASL1 Gene**

| Region of *AHAS1* | Primer Name | Primer Sequence |
|---|---|---|
| 1^{st} amplicon (1036 bp) | C2359-F1 | AGATCAACCGAGAAAAGGCG (SEQ ID NO: 11) |
| | ALSR1 | CGAAAAATCAAGATTAGTCACC (SEQ ID NO: 12) |
| 2^{nd} amplicon (709 bp) | SUNALS1-F1 | GAGGAAAAGAATTCGGTGACT (SEQ ID NO: 13) |
| | ALS-8R | CGGTGATCACATCCGAGAAA (SEQ ID NO: 14) |

From comparisons of the nucleotide sequences of known *AHASL1, AHASL2,* and *AHASL3* genes, PCR primers were designed to specifically amplify the *AHASL1* gene from sunflower. The following PCR conditions were used in a total reaction volume of 50µl: 1X buffer (Gibco-BRL), 0.2mM dNTPs (Gibco-BRL), 2.5mM MgCl₂ (Gibco-BRL), 0.2µM of each primer, 0.5µL of Platinium Taq (5U/µL) (Gibco-BRL) and 100 ng of genomic DNA. PCR reactions were carried out in a GeneAmp PCR System 9700 (PerkinElmer, Inc., Boston, MA, USA). Cycling conditions were: an initial denaturation step at 94°C for 1 minute followed by a program of 40 cycles consisting of 10 cycles of touch-down PCR (94°C for 30 seconds, 67°C to 58°C for 30 seconds and 72°C for 30 seconds), followed by 30 cycles of the same cycling regime with a fixed annealing temperature of 58°C and a final elongation step of 72°C for 10 minutes. Ten microliters of each resulting PCR product were then analyzed by agarose gel electrophoresis and the remaining 40 µL of the PCR product was purified using the Concert™ Rapid PCR Purification System (Gibco-BRL) prior to sequencing. The purified PCR products were then cycle-sequenced using a BigDye® Terminator v3.1 Cycle Sequencing Kit (Perkin Elmer) following the manufacturer's instructions. In addition to the primers used for the PCR amplifications (Table 2), additional primers set forth in Table 3 were used to complete the sequencing of the entire coding region of the sunflower AHASL1 gene.

**Table 3. Additional Primers for Sequencing the Coding Region of the Sunflower AHASL1 gene**

| Region of *AHAS1* | Primer Name | Primer Sequence |
|---|---|---|
| 1^{st} amplicon (1036 bp) | ALS-2R | CATCGGTTCCGATCATTCCTC (SEQ ID NO: 15) |
| | ALS-2F | GTGTCACCGACGTCTTCGC (SEQ ID NO: 16) |
| | ALS-3R | CTAGACAAATAACCCGGTAACC (SEQ ID NO: 17) |
| | ALS-3F | GCGCTGTTAGACAGTGTCC (SEQ ID NO: 18) |
| | ALSRI | CGAAAAATCAAGATTAGTCACC (SEQ ID NO: 12) |
| | ALS4F | ACAGCAACAGTTAGTGGTG (SEQ ID NO: 19) |
| 2^{nd} amplicon (709 bp) | ALS-6R | CGGCAGATTTTCAACACGGA (SEQ ID NO: 20) |
| | ALS-7F | AGACAATGGCTGACGTCGG (SEQ ID NO: 21) |
| | ALS-8F | TATGGTGGTTCAGTGGGAGG (SEQ ID NO: 22) |

Fluorescent-labeled products from sequencing reactions were resolved on an ABI 377 automatic sequencer (Perkin Elmer). The sunflower AHASL1 sequencing files obtained from each amplicon were assembled using the Vector NTI Suite-Contig Express software, version 7.0 (InforMax, Frederick, MD, USA). The resulting DNA sequences were aligned with AHASL1 polynucleotide sequences of the RHA266 sunflower line and *Xanthium sp.* (Figure 1). The predicted amino acid sequence from the new mutant sunflower AHASL1 gene was aligned with the AHASL1 amino acid sequences of RHA266 and *Xanthium sp.* (Figure 2) using Vector NTI Suite-AlignX software, version 7.0 (InforMax) was used with default parameters. Single nucleotide polymorphisms and amino acid changes were then identified in the nucleotide and amino acids sequence from the MUT9 plant. Relative to the equivalent positions in the wild-type nucleotide (SEQ ID NO: 2) and amino acid (SEQ ID NO: 4) sequences of RHA266, the MUT nucleotide sequence (SEQ ID NO: 1) has a C-to-T transition at nucleotide position 305 and its amino acid sequence (SEQ ID NO: 2) has a threonine-to-isoleucine substitution at amino acid position 102. The site of the C-to-T transition in the MUT9 nucleotide sequence corresponds to nucleotide 563 in the full-length sunflower AHASL coding sequence having GenBank Accession No. AY541451 (SEQ ID NO: 23). The site of the threonine-to-isoleucine substitution in the MUT9 amino acid sequence corresponds to amino acid 188 in the full-length amino acid sequence of the sunflower AHASL1 protein encoded by the nucleotide sequence having GenBank Accession No. AY541451 (SEQ ID NO: 24).

### EXAMPLE 3: Fertile Herbicide-Resistant Sunflower Plants

Twenty M₂ seeds were obtained from the M₁ plant known as MUT9. These M₂ seeds were sown in the greenhouse. Of the six plantlets that resulted from these seeds, two grew into fertile plants. One of the fertile plants yielded 13 seeds and was determined by DNA sequencing to be heterozygous for the mutant *AHASL1* allele described in Example 2. The other fertile plant yield 12 seeds but was homozygous for the wild-type allele at the *AHASL1* gene.

### EXAMPLE 4: The Herbicide-Resistance of MUT9 Sunflower Plants

To evaluate the resistance of MUT9 sunflower plants to an imidazolinone herbicides, RHA266 (wild-type) and homozygous M₅ MUT9 sunflower plants were planted outdoors in Balcarce, Argentina during the growing season in a randomized complete block design (RCBD) field trial with two replications to evaluate the tolerance of MUT9 plants to three rates of Sweeper 70DG: 1X, 2X, and 3X at the six-leaf stage (i.e., 3 pairs of leaves). The active ingredient in Sweeper is imazamox and the 1X dose is 50 g a.i./ha. The results are presented in Table 4.

**Table 4. Imidazolinone Tolerance of MUT9 Sunflower Plants (% Injury')**

| | RATE | | | |
|---|---|---|---|---|
| LINE | 0X | 1X | 2X | 3X |
| RHA266 | 0 | 75 | 75 | 75 |
| MUT9 | 0 | 15 | 31 | 39 |

| | | | | |
|---|---|---|---|---|
| ¹Injury Scale: 0%, no injury; 5-10%, chlorosis, yellow flash; 10-20%, growth rate reduction, shortening of internodes; 20-30%, leaf deformations; 30-45%, necrosis; and >50%, dead plants. | | | | |

Compared to the wild-type RHA266 sunflower plants, the MUT9 sunflower plants had significantly less injury at the 1X, 2X, and 3X does of Sweeper. The results of this trial demonstrate that the MUT9 plants have increased resistance or tolerance to imidazolinone herbicides. In another similar trial, RHA266 plants and MUT9 sunflower plants that were either heterozygous and homozygous for the herbicide-resistant AHASL1 allele were treated 0X, 1X, and 2X rates of Sweeper. The homozygous MUT9 plants displayed a higher level of tolerance than the heterozygous plants. As expected, the RHA266 plants (wild-type) were susceptible to the 1X and 2X rates of Sweeper.

To evaluate the resistance of MUT9 sunflower plants to sulfonylurea herbicides, RHA266 (wild-type) and MUT9 sunflower plants were planted outdoors in Balcarce, Argentina during the growing season in an RCBD field trial with two replications to evaluate the tolerance of homozygous M₅ MUT9 plants to the sulfonylurea herbicides tribenuron methyl (TBM) and thifensulfuron (TFS) at 1X and 2X rates applied at the six-leaf stage. Due to the lack of MUT9 seed, one herbicide was sprayed in each replication. The 1X rates for TBM and TFS are 13.15 g a.i./ha and 4.4 g a.i./ha, respectively. The results are presented in Table 5.

**Table 5. Tolerance of MUT9 Sunflower Plants to the Sulfonylurea Herbicides Tribenuron Methyl (TBM) and Thifensulfuron (TFS) (% Injury¹)**

| | RATE | | | | | | | | |
|---|---|---|---|---|---|---|---|---|---|
| | 0X | | | 1X | | | 2X | | |
| LINE | TBM | TFS | Avg.² | TBM | TFS | Avg. | TBM | TFS | Avg. |
| RHA266 | 0 | 0 | 0 | | 75 | 75 | 45 | 75 | 60 |
| MUT9 | 0 | 0 | 0 | 75 | 75 | 75 | 75 | 75 | 75 |

| | | | | | | | | | |
|---|---|---|---|---|---|---|---|---|---|
| ¹Injury scale same as Table 4. ²Average of TBM and TFS injury values. | | | | | | | | | |

### EXAMPLE 5: Herbicide-Resistant Sunflower AHASL Proteins

The present invention discloses both the nucleotide and amino acid sequences for wild-type and herbicide-resistant sunflower AHASL polypeptides. Plants comprising herbicide-resistant AHASL polypeptides have been previously identified, and a number of conserved regions of AHASL polypeptides that are the sites of amino acids substitutions that confer herbicide resistance have been described. See, Devine and Eberlein (1997) "Physiological, biochemical and molecular aspects of herbicide resistance based on altered target sites". In: Herbicide Activity: Toxicology, Biochemistry and Molecular Biology, Roe et al. (eds.), pp. 159-185, IOS Press, Amsterdam; and Devine and Shukla, (2000) Crop Protection 19:881-889.

Using the AHASL sequences of the invention and methods known to those of ordinary skill in art, one can produce additional polynucleotides encoding herbicide-resistant AHASL polypeptides having one, two, three, or more amino acid substitutions at the identified sites in these conserved regions. Table 6 provides the conserved regions of AHASL proteins, the amino acid substitutions known to confer herbicide resistance within these conserved regions, and the corresponding amino acids in the sunflower AHASL protein set forth in SEQ ID NO: 4.

**Table 6. Mutations in conserved regions of AHASL polypeptides known to confer herbicide-resistance and their equivalent position in sunflower AHASL1**

| Conserved region¹ | Mutation² | Reference | Position in sunflower |
|---|---|---|---|
| VFAYPGGASMEIHQALTRS³ | Ala₁₂₂ to Thr | Bernasconi *et al.⁶* | Ala₁₀₇ |
| | | Wright & Penner¹⁵ | |
| AITGQVPRRMIGT⁴ | Pro₁₉₇ to Ala | Boutsalis *et al.⁷* | Pro₁₈₂ |
| | Pro₁₉₇ to Thr | Guttieri *et al_{.}*⁸ | |
| | Pro₁₉₇ to His | Guttieri *et al.*⁹ | |
| | Pro₁₉₇ to Leu | Guttieri *et al.*⁸ | |
| | | Kolkman *et al.*¹⁶ | |
| | Pro₁₉₇ to Arg | Guttieri *et al*.⁸ | |
| | Pro₁₉₇ to Ile | Boutsalis *et al.*⁷ | |
| | Pro₁₉₇ to Gln | Guttieri *et al.*⁸ | |
| | Pro₁₉₇ to Ser | Guttieri *et al.*⁸ | |
| | Thr₂₀₃ to Ile | Disclosed herein¹⁴ | Thr₁₈₈ |
| AFQETP³ | Ala₂₀₅ to Asp | Hartnett *et al.*¹⁰ | Ala₁₉₀ |
| | Ala₂₀₅ to Val | Simpson¹¹ | |
| | | Kolkman *et al.*¹⁶ | |
| | | White *et al.*¹⁷ | |
| QWED³ | Trp₅₇₄ to Leu | Bruniard¹² | Trp₅₅₉ |
| | | Boutsalis *et al*.⁷ | |
| IPSGG⁵ | Ser₆₅₃ to Asn | Devine & Eberlein¹³ | Ala₆₃₈ |
| | | Lee *et al*.¹⁸ | |
| | Ser₆₅₃ to Thr | Chang & Duggleby¹⁹ | |
| | Ser₆₅₃ to Phe | | |

| | | | |
|---|---|---|---|
| ¹Conserved regions from Devine and Eberlein (1997) "Physiological, biochemical and molecular aspects of herbicide resistance based on altered target sites". In: Herbicide Activity: Toxicology, Biochemistry and Molecular Biology, Roe et al. (eds.), pp. 159-185, IOS Press, Amsterdam and Devine and Shukla, (2000) Crop Protection 19:881-889. ²Amino acid numbering corresponds to the sequence of the *Arabidopsis thaliana* AHASL1 polypeptide. ³Sunflower AHASL1 (SEQ ID NO:4) has the same conserved region. ⁴The region of the sunflower AHASL amino acid sequence set forth in SEQ ID NO: 2 that corresponds to this conserved region includes the site of the T188-to-I substitution and thus has the sequence AITGQVPRRMIGI. The same region of the wild-type sunflower AHASL amino acid sequence that is set forth in SEQ ID NO: 4 is identical to the conserved region indicated in Table 6. ⁵The region of the sunflower AHASL1 (SEQ ID NO:4) corresponding to this conserved region has the sequence IPAGG. ⁶Bernasconi et al. (1995) J. Biol. Chem. 270(29):17381-17385. ⁷Boutsalis et al. (1999) Pestic. Sci. 55:507-516. ⁸Guttieri et al. (1995) Weed Sci. 43:143-178. ⁹Guttieri et al. (1992) Weed Sci. 40:670-678. ¹⁰Hartnett et al. (1990) "Herbicide-resistant plants carrying mutated acetolactate synthase genes," In: Managing Resistance to Agrochemicals: Fundamental Research to Practical Strategies, Green et al. (eds.), American Chemical Soc. Symp., Series No. 421, Washington, DC, USA ¹¹Simpson (1998) Down to Earth 53(1):26-35. ¹²Bruniard (2001) Inheritance of imidazolinone resistance, characterization of cross-resistance pattern, and identification of molecular markers in sunflower (Helianthus annuus L.). Ph.D. Thesis, North Dakota State University, Fargo, ND, USA, pp 1-78. ¹3Devine and Eberlein (1997) "Physiological, biochemical and molecular aspects of herbicide resistance based on altered target sites". In: Herbicide Activity: Toxicology, Biochemistry and Molecular Biology, Roe et al. (eds.), pp. 159-185, IOS Press, Amsterdam ¹⁴ The present invention discloses the amino acid sequence of a herbicide-resistant AHASL1 with the Thr₁₈₈ to Ile substitution (SEQ ID NO: 2) and a polynucleotide sequence encoding this herbicide-resistant AHASL1 (SEQ ID NO: 1). ¹⁵Wright and Penner (1998) Theor. Appl. Genet. 96:612-620. ¹⁶Kolkman et al. (2004) Theor. Appl. Genet. 109: 1147-1159. ¹⁷White et al. (2003) Weed Sci. 51:845-853. ¹⁸Lee et al. (1999) FEBS Lett. 452:341-345. ¹⁹Chang and Duggleby (1998) Biochem J. 333:765-777. | | | |

All publications and patent applications mentioned in the specification are indicative of the level of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced within the scope of the appended claims.

## Claims

1. A sunflower plant comprising in its genome at least one copy of at least one acetohydroxyacid synthase large subunit (AHASL) polynucleotide, wherein said AHASL polynucleotide encodes an imidazolinone herbicide-tolerant AHASL protein comprising an isoleucine substituted at the amino acid position equivalent to position 188 of SEQ ID NO:24, wherein said plant has increased tolerance to an imidazolinone herbicide compared to the tolerance of a wild-type suntlower plant to the imidazolinone herbicide.

2. The sunflower plant of claim 1, wherein said AHASL protein comprises the amino acid sequence set forth in SEQ ID NO:2.

3. The sunflower plant of claim 1 or 2, wherein said AHASL polynucleotide comprises the nucleotide sequence set forth in SEQ ID NO: 1.

4. The suntlower plant of claim 1, wherein said AHASL protein further comprises:
(a) a threonine substituted at the amino acid position equivalent to position 107 of SEQ ID NO:24;
(b) an alanine, threonine, histidine, leucine, arginine, isoleucine, glutamine, or serine substituted at the amino acid position equivalent to position 182 of SEQ ID NO:24;
(c) an aspartate or valine substituted at the amino acid position equivalent to position 190 of SEQ ID NO:24;
(d) a leucine substituted at the amino acid position equivalent to position 559 of SEQ ID NO:24; or
(e) an asparagine, threonine, or phenylalanine substituted at the amino acid position equivalent to position 638 of SEQ ID NO:24.

5. The sunflower plant of any one of claims 1-4, wherein said plant is transgenic or non-transgenic.

6. A seed of the suntlower plant of any one of claims 1-5. wherein said seed comprises in its genome at least one copy of said AHASL polynucleotide.

7. The sunflower plant of claim 1, wherein said plant is selected from the group consisting of:
(a) descendants of any plants of line MUT9, a representative sample of seed of the line having been deposited under ATCC Patent Deposit Number PTA-6325;
(b) mutants, recombinants, or genetically engineered derivatives of any plants of line MUT9; and
(c) descendants of any one of (a)-(b).

8. The sunflower plant of claim 7, wherein said plant is a descendant of a plant of line MUT9, a representative sample of seed of the line having been deposited under ATCC Patent Deposit Number PTA-6325:

9. The sunflower plant of claim 7, wherein said plant is a mutant, recombinant, or genetically engineered derivative of a plant of line MUT9, a representative sample of seed of the line having been deposited under ATCC Patent Deposit Number PTA-6325.

10. The sunflower plant of claim 7, wherein said plant is a descendant of a sunflower of any one of claims 8 or 9.

11. The sunflower plant of any one of claims 7-10, wherein said plant is transgenic or non transgenic.

12. A seed of the sunflower plant of any one of claims 7-11, wherein said seed comprises the at least one copy of the at least one AHASL polynucleotide encoding the imidazolinone herbicide-tolerant AHASL protein.

13. A method of controlling weeds in the vicinity of a sunflower plant, said method comprising applying an effective amount of an imidazolinone herbicide to the weeds and to the sunflower plant, wherein said sunflower plant is a suntlower plant of any one of claims 1-5 and 7-11. or a sunflower plant of line MUT9, a representative sample of seed of the line having been deposited under ATCC Patent Deposit Number PTA-6325.

14. The method of claim 13, wherein said imidazolinone herbicide is selected from the group consisting of: 2-(4-isopropyl-4-methyl-5-oxo-2-imidiazolin-2-yl)-nicotinic acid, 2-(4-isopropyl-4-methyl-5-oxo-2-imidazolin-2-yl)-3-quinolinecarboxylic acid, 5-ethyl-2-(4-isopropyl-4-methyl-5-oxo-2-imidazolin-2-yl)-nicotinic acid, 2-(4-isopropyl-4-methyl-5-oxo-2-imidazolin-2-yl)-5-(methoxymethyl)-nicotinic acid, 2-(4-isopropyl-4-methyl-5-oxo-2-imidazolin-2-yl)-5-methylnicotinic acid, mixtures of methyl 6-(4-isopropyl-4-methyl-5-oxo-2-imidazolin-2-yl)-m-toluate and methyl 2-(4-isopropyl-4-methyl-5-oxo-2-imidazolin-2-yl)-p-toluate, and combinations thereof.

15. An isolated polynucleotide molecule comprising a nucleotide sequence selected from the group consisting of:
(a) the nucleotide sequence set forth in SEQ ID NO: 1;
(b) nucleotide sequences encoding the amino acid sequence set forth in SEQ ID NO:2;
(c) nucleotide sequences having at least 75% sequence identity to the nucleotide sequence set forth in SEQ ID NO:1, wherein said nucleotide sequence encodes a protein comprising an isoleucine substituted at the amino acid position equivalent to position 188 of SEQ ID NO:24, wherein said protein provides imidazolinone herbicide-tolerant acetohydroxyacid synthase (AHAS) activity;
(d) nucleotide sequences encoding a protein having at least 75% sequence identity to the amino acid sequence set forth in SEQ ID NO:2, wherein said protein comprises an isoleucine substituted at the amino acid position equivalent to position 188 of SEQ ID NO:24, wherein said protein provides imidazolinone herbicide-tolerant AHAS activity; and
(e) a nucleotide sequence that is fully complementary to any one of the sequences set forth in (a)-(d).

16. The isolated polynucleotide molecule of claim 15. wherein the nucleotide sequence is set forth in SEQ ID NO:1.

17. The isolated polynucleotide molecule of claim 15, wherein the nucleotide sequence encodes the amino acid sequence set forth in SEQ ID NO:2.

18. The isolated polynucleotide molecule of any one of claims 15-17, wherein said protein encoded by the nucleotide sequence of (c) or (d), further comprises:
(a) a threonine substituted at the amino acid position equivalent to position 107 of SEQ ID NO:24;
(b) an alanine, threonine, histidine, leucine, arginine, isoleucine, glutamine, or serine substituted at the amino acid position equivalent to position 182 of SEQ ID NO:24;
(c) an aspartate or valine substituted at the amino acid position equivalent to position 190 of SEQ ID NO:24;
(d) a leucine substituted at the amino acid position equivalent to position 559 of SEQ ID NO:24; or
(e) an asparagine, threonine, or phenylalanine substituted at the amino acid position equivalent to position 638 of SEQ ID NO:24.

19. An expression cassette comprising a promoter operably linked to the polynucleotide molecule of any one of claims 15-18.

20. The expression cassette of claim 19, wherein said promoter is capable of driving gene expression in a bacterium, a fungal cell, or a plant cell.

21. A non-human host cell transformed with the expression cassette of claim 19 or 20.

22. The host cell of claim 21, wherein said host cell is selected from the group consisting of a bacterium, a fungal cell, and a plant cell.

23. A transformer plant comprising stably incorporated in its genome a polynucleotide construct comprising a nucleotide sequence operably linked to a promoter that drives expression in a plant cell, wherein said nucleotide sequence is selected from the group consisting of:
(a) the nucleotide sequence set forth in SEQ ID NO: 1;
(b) nucleotide sequences encoding the amino acid sequence set forth in SEQ ID NO: 2;
(c) nucleotide sequences having at least 75% sequence identity to the nucleotide sequence set forth in SEQ ID NO:1, wherein said nucleotide sequence encodes a protein comprising an isoleucine substituted at the amino acid position equivalent to position 188 of SEQ ID NO:24, wherein said protein provides imidazolinone herbicide-tolerant AHAS activity; and
(d) nucleotide sequences encoding a protein having at least 75% sequence identity to the amino acid sequence set forth in SEQ ID NO:2, wherein said protein comprises an isoleucine substituted at the amino acid position equivalent to position 188 of SEQ ID NO:24, wherein said protein provides imidazolinone herbicide-tolerant AHAS activity.

24. The transformed plant of claim 23, wherein the nucleotide sequence is as set forth in SEQ ID NO:1.

25. The transformed plant of claim 23, wherein the nucleotide sequence encodes the amino acid sequence set forth in SEQ ID NO:2.

26. The transformed plant of any one of claims 23-25, wherein said promoter is selected from the group consisting of constitutive promoters and tissue-preferred promoters.

27. The transformed plant of any one of claims 23-26, wherein said polynucleotide construct further comprises an operably linked chloroplast-targeting sequence.

28. The transformed plant of any one of claims 23-27, wherein the AHAS activity of said transformed plant is increased relative to an untransformed plant.

29. The transformed plant of any one of claims 23-28, wherein the tolerance of said transformed plant to an imidazolinone herbicide is increased when compared to a wild-type sunflower plant.

30. The transformed plant of claim 29, wherein said imidazolinone herbicide is selected from the group consisting of: 2-(4-isopropyl-4-methyl-5-oxo-2-imidiazolin-2-yl)-nicotinic acid, 2-(4-isopropyl-4-methyl-5-oxo-2-imidazolin-2-yl)-3-quinolinecarboxylic acid, 5-ethyl-2-(4-isopropyl-4-methyl-5-oxo-2-imidazolin-2-yl)-nicotinic acid, 2-(4-isopropyl-4-methyl-5-oxo-2-imidazolin-2-yl)-5-(methoxymethyl)-nicotinic acid, 2-(4-isopropyl-4-methyl-5-oxo-2-imidazolin-2-yl)-5-methylnicotinic acid, mixtures of methyl 6-(4-isopropyl-4-methyl-5-oxo-2-imidazolin-2-yl)-m-toluate and methyl 2-(4-isopropyl-4-methyl-5-oxo-2-imidazolin-2-yl)-p-toluate, and combinations thereof.

31. The transformed plant of any one of claims 23-30, wherein said transformed plant is a dicot or a monocot.

32. The transformed plant of claim 31, wherein said dicot is selected from the group consisting of sunflower, soybean, cotton, *Brassica spp., Arabidopsis thaliana,* tobacco, potato, sugar beet, alfalfa, safflower, and peanut and wherein said monocot is selected from the group consisting of wheat, rice, maize, barley, rye, oats, triticale, millet, and sorghum.

33. A seed of the transformed plant of any one of claims 23-32, wherein said seed comprises said polynucleotide construct.

34. A method for producing an imidazolinone herbicide-tolerant plant, the method comprising transforming a plant cell with a polynucleotide construct comprising a nucleotide sequence operably linked to a promoter that drives expression in a plant cell and regenerating the imidazolinone herbicide-tolerant plant from said transformed plant cell, wherein said nucleotide sequence is selected from the group consisting of:
(a) the nucleotide sequence set forth in SEQ ID NO:1;
(b) nucleotide sequences encoding the amino acid sequence set forth in SEQ ID NO:2;
(c) nucleotide sequences having at least 75% sequence identity to the nucleotide sequence set forth in SEQ ID NO:1, wherein said nucleotide sequence encodes a protein comprising an isoleucine substituted at the amino acid position equivalent to position 188 of SEQ ID NO:24, wherein said protein provides the imidazolinone herbicide-tolerant AHAS activity; and
(d) nucleotide sequences encoding a protein having at least 75% sequence identity to the amino acid sequence set forth in SEQ ID NO:2, wherein said protein comprises an isoleucine substituted at the amino acid position equivalent to position 188 of SEQ ID NO:24, wherein said protein provides the imidazolinone herbicide-tolerant AHAS activity;
wherein said transformed plant has increased tolerance to an imidazolinone herbicide relative to the tolerance of an untransformed plant to said herbicide.

35. The method of claim 34, wherein the nucleotide sequence is as set forth in SEQ ID NO:1.

36. The method of claim 34, wherein the nucleotide sequence encodes the amino acid sequence set forth in SEQ ID NO:2.

37. The method of any one of claims 34-36, wherein said promoter is selected from the group consisting of constitutive promoters and tissue-preferred promoters.

38. The method of any one of claims 34-37, wherein said polynucleotide construct further comprises an operably linked chloroplast-targeting sequence.

39. The method of any one of claims 34-38, wherein the AHAS activity of said transformed plant is increased relative to an untransformed plant.

40. The method of any one of claims 34-39, wherein said imidazolinone herbicide is selected from the group consisting of: 2-(4-isopropyl-4-methyl-5-oxo-2-imidiazolin-2-yl)-nicotinic acid [, 2-(4-isopropyl-4-methyl-5-oxo-2-imidazolin-2-yl)-3-quinolinecarboxylic acid, 5-ethyl-2-(4-isopropyl-4-methyl-5-oxo-2-imidazolin-2-yl)-nicotinic acid, 2-(4-isopropyl-4-methyl-5-oxo-2-imidazolin-2-yl)-5-(methoxymethyl)-nicotinic acid, 2-(4-isopropyl-4-methyl-5-oxo-2-imidazolin-2-yl)-5-methylnicotinic acid, mixtures of methyl 6-(4-isopropyl-4-methyl-5-oxo-2-imidazolin-2-yl)-m-toluate and methyl 2-(4-isopropyl-4-methyl-5-oxo-2-imidazolin-2-yl)-p-toluate, and combinations thereof.

41. The method of any one of claims 34-40, wherein said imidazolinone herbicide-tolerant plant is a dicot or a monocot.

42. The method of claim 41, wherein said dicot is selected from the group consisting of sunflower, soybean, cotton, *Brassica spp., Arabidopsis thaliana,* tobacco, potato, sugar beet, alfalfa, safflower, and peanut and wherein said monocot is selected from the group consisting of wheat, rice, maize, barley, rye, oats, triticale, millet, and sorghum.

43. The method of any one of claims 34-42, wherein said plant cell comprises tolerance to at least one herbicide, prior to said transformation step.

44. A method of controlling weeds in the vicinity of a transformed plant, said method comprising applying an effective amount of an imidazolinone herbicide to the weeds and to the transformed plant, wherein said transformed plant is according to any one of claims 23-32.

45. The method of claim 44, wherein said imidazolinone herbicide is selected from the group consisting of: 2-(4-isopropyl-4-methyl-5-oxo-2-imidiazolin-2-yl)-nicotinic acid, 2-(4-isopropyl-4-methyl-5-oxo-2-imidazolin-2-yl)-3-quinolinecarboxylic acid, 5-ethyl-2-(4-isopropyl-4-methyl-5-oxo-2-imidazolin-2-yl)-nicotinic acid, 2-(4-isopropyl-4-methyl-5-oxo-2-imidazolin-2-yl)-5-(methoxymethyl)-nicotinic acid, 2-(4-isopropyl-4-methyl-5-oxo-2-imidazolin-2-yl)-5-methylnicotinic acid, mixtures of methyl 6-(4-isopropyl-4-methyl-5-oxo-2-imidazolin-2-yl)-m-toluate and methyl 2-(4-isopropyl-4-methyl-5-oxo-2-imidazolin-2-yl)-p-toluate, and combinations thereof.

46. An isolated polypeptide comprising an amino acid sequence selected from the group consisting of:
(a) the amino acid sequence set forth in SEQ ID NO:2;
(b) the amino acid sequence encoded by the nucleotide sequence set forth in SEQ ID NO:1;
(c) amino acid sequences having at least 75% sequence identity to the amino acid sequence set forth in SEQ ID NO:2. wherein said polypeptide comprises an isoleucine substituted at the amino acid position equivalent to position 188 of SEQ ID NO:24, wherein said polypeptide provides imidazolinone herbicide-tolerant AHAS activity; and
(d) the amino acid sequences encoded by nucleotide sequences having at least 75% sequence identity to the nucleotide sequence set forth in SEQ ID NO:1, wherein said nucleotide sequence encodes a protein comprising an isoleucine substituted at the amino acid position equivalent to position 188 of SEQ ID NO:24, wherein said protein provides imidazolinone herbicide-tolerant AHAS activity.

47. The isolated polypeptide of claim 46, wherein the amino acid sequence is encoded by the nucleotide sequence set forth in SEQ ID NO:1.

48. The isolated polypeptide of claim 46, wherein the amino acid sequence is as set forth in SEQ ID NO:2.

49. The seed of any one of claims 6, 12, and 33, wherein said seed is treated with an AHAS-inhibiting herbicide.

50. A method for combating undesired vegetation comprising contacting the seed of any one of claims 6, 12, 33, and 49 before sowing and/or after pregermination with an AHAS-inhibiting herbicide.

## Patentansprüche

1. Sonnenblumenpflanze, die in ihrem Genom mindestens eine Kopie von mindestens einem Polynukleotid der großen Untereinheit der Acetohydroxysäuresynthase (AHASL) umfasst, wobei das AHASL-Polynukleotid für ein imidazolinonherbizidtolerantes AHASL-Protein kodiert, das an der Aminosäureposition, die Position 188 von SEQ ID NO: 24 entspricht, eine Isoleucinsubstitution umfasst, wobei die Pflanze eine im Vergleich zu der Imidazolinonherbizidtoleranz einer Wildtypsonnenblumenpflanze erhöhte Imidazolinonherbizidtoleranz aufweist.

2. Sonnenblumenpflanze nach Anspruch 1, wobei das AHASL-Protein die Aminosäuresequenz gemäß SEQ ID NO: 2 umfasst.

3. Sonnenblumenpflanze nach Anspruch 1 oder 2, wobei das AHASL-Polynukleotid die Nukleotidsequenz gemäß SEQ ID NO: 1 umfasst.

4. Sonnenblumenpflanze nach Anspruch 1, wobei das AHASL-Protein weiterhin Folgendes umfasst:
(a) eine Threoninsubstitution an der Aminosäureposition, die Position 107 von SEQ ID NO: 24 entspricht;
(b) eine Alanin-, Threonin-, Histidin-, Leucin-, Arginin-, Isoleucin-, Glutamin- oder Serinsubstitution an der Aminosäureposition, die Position 182 von SEQ ID NO: 24 entspricht;
(c) eine Asparaginsäure- oder Valinsubstitution an der Aminosäureposition, die Position 190 von SEQ ID NO: 24 entspricht;
(d) eine Leucinsubstitution an der Aminosäureposition, die Position 559 von SEQ ID NO: 24 entspricht; oder
(e) eine Asparagin-, Threonin- oder Phenylalaninsubstitution an der Aminosäureposition, die Position 638 von SEQ ID NO: 24 entspricht.

5. Sonnenblumenpflanze nach einem der Ansprüche 1 - 4, wobei die Pflanze transgen oder nichtransgen ist.

6. Samen der Sonnenblumenpflanze nach einem der Ansprüche 1 - 5, wobei der Samen in seinem Genom mindestens eine Kopie des AHASL-Polynukleotids umfasst.

7. Sonnenblumenpflanze nach Anspruch 1, wobei die Pflanze aus der Gruppe, die aus Folgendem besteht, ausgewählt ist:
(a) Nachkommen von beliebigen Pflanzen der Linie MUT9, wobei eine repräsentative Stichprobe von Samen der Linie unter der ATCC-Patenthinterlegungsnummer PTA-6325 hinterlegt worden ist;
(b) Mutanten, Rekombinanten oder genetisch modifizierten Abkömmlingen von beliebigen Pflanzen der Linie MUT9; und
(c) Nachkommen von beliebigen aus der Reihe (a)-(b).

8. Sonnenblumenpflanze nach Anspruch 7, wobei es sich bei der Pflanze um einen Nachkommen einer Pflanze der Linie MUT9 handelt, wobei eine repräsentative Stichprobe von Samen der Linie unter der ATCC-Patenthinterlegungsnummer PTA-6325 hinterlegt worden ist.

9. Sonnenblumenpflanze nach Anspruch 7, wobei es sich bei der Pflanze um eine Mutante, eine Rekombinante oder einen genetisch modifizierten Abkömmling einer Pflanze der Linie MUT9 handelt, wobei eine repräsentative Stichprobe von Samen der Linie unter der ATCC-Patenthinterlegungsnummer PTA-6325 hinterlegt worden ist.

10. Sonnenblumenpflanze nach Anspruch 7, wobei es sich bei der Pflanze um einen Nachkommen einer Sonnenblume von einem der Ansprüche 8 oder 9 handelt.

11. Sonnenblumenpflanze nach einem der Ansprüche 7 - 10, wobei die Pflanze transgen oder nichtransgen ist.

12. Samen der Sonnenblumenpflanze nach einem der Ansprüche 7-11, wobei der Samen die mindestens eine Kopie des mindestens einen AHASL-Polynukleotids, das für das imidazolinonherbizidtolerante AHASL-Protein kodiert, umfasst.

13. Verfahren zum Bekämpfen von Unkräutern in der Nähe einer Sonnenblumenpflanze, wobei das Verfahren umfasst, dass man auf die Unkräuter und auf die Sonnenblumenpflanze eine wirksame Menge eines Imidazolinonherbizids appliziert, wobei es sich bei der Sonnenblumenpflanze um eine Sonnenblumenpflanze nach einem der Ansprüche 1 - 5 und 7 - 11 oder um eine Sonnenblumenpflanze der Linie MUT9, wobei eine repräsentative Stichprobe von Samen der Linie unter der ATCC-Patenthinterlegungsnummer PTA-6325 hinterlegt worden ist, handelt.

14. Verfahren nach Anspruch 13, wobei das Imidazolinonherbizid aus der Gruppe bestehend aus: 2-(4-Isopropyl-4-methyl-5-oxo-2-imidazolin-2-yl)nikotinsäure, 2-(4-Isopropyl-4-methyl-5-oxo-2-imidazolin-2-yl)-3-chinolincarbonsäure, 5-Ethyl-2-(4-isopropyl-4-methyl-5-oxo-2-imidazolin-2-yl)nikotinsäure, 2-(4-Isopropyl-4-methyl-5-oxo-2-imidazolin-2-yl)-5-(methoxymethyl)nikotinsäure, 2-(4-Isopropyl-4-methyl-5-oxo-2-imidazolin-2-yl)-5-methylnikotinsäure, Mischungen aus Methyl-6-(4-isopropyl-4-methyl-5-oxo-2-imidazolin-2-yl)-m-toluat und Methyl-2-(4-isopropyl-4-methyl-5-oxo-2-imidazolin-2-yl)-p-toluat und Kombinationen davon ausgewählt ist.

15. Isoliertes Polynukleotidmolekül, das eine Nukleotidsequenz umfasst, die aus der Gruppe bestehend aus Folgendem ausgewählt ist:
(a) der Nukleotidsequenz gemäß SEQ ID NO: 1;
(b) Nukleotidsequenzen, die für die Aminosäuresequenz gemäß SEQ ID NO: 2 kodieren;
(c) Nukleotidsequenzen mit mindestens 75% Sequenzidentität zu der Nukleotidsequenz gemäß SEQ ID NO: 1, wobei die Nukleotidsequenz für ein Protein kodiert, das eine Isoleucinsubstitution an der Aminosäureposition, die Position 188 von SEQ ID NO: 24 entspricht, umfasst, wobei das Protein imidazolinonherbizidtolerante Acetohydroxysäuresynthase (AHAS)-Aktivität bereitstellt;
(d) Nukleotidsequenzen, die für ein Protein kodieren, das mindestens 75% Sequenzidentität zu der Aminosäuresequenz gemäß SEQ ID NO: 2 aufweist, wobei das Protein eine Isoleucinsubstitution an der Aminosäureposition, die Position 188 von SEQ ID NO: 24 entspricht, aufweist, wobei das Protein imidazolinonherbizidtolerante AHAS-Aktivität bereitstellt; und
(e) einer Nukleotidsequenz, die zu einer beliebigen der Sequenzen gemäß (a)-(d) vollständig komplementär ist.

16. Isoliertes Polynukleotidmolekül nach Anspruch 15, wobei die Nukleotidsequenz in SEQ ID NO: 1 angegeben ist.

17. Isoliertes Polynukleotidmolekül nach Anspruch 15, wobei die Nukleotidsequenz für die Aminosäuresequenz gemäß SEQ ID NO: 2 kodiert.

18. Isoliertes Polynukleotidmolekül nach einem der Ansprüche 15 - 17, wobei das Protein, das von der Nukleotidsequenz von (c) oder (d) kodiert wird, weiterhin Folgendes umfasst:
(a) eine Threoninsubstitution an der Aminosäureposition, die Position 107 von SEQ ID NO: 24 entspricht;
(b) eine Alanin-, Threonin-, Histidin-, Leucin-, Arginin-, Isoleucin-, Glutamin- oder Serinsubstitution an der Aminosäureposition, die Position 182 von SEQ ID NO: 24 entspricht;
(c) eine Asparaginsäure- oder Valinsubstitution an der Aminosäureposition, die Position 190 von SEQ ID NO: 24 entspricht;
(d) eine Leucinsubstitution an der Aminosäureposition, die Position 559 von SEQ ID NO: 24 entspricht; oder
(e) eine Asparagin-, Threonin- oder Phenylalaninsubstitution an der Aminosäureposition, die Position 638 von SEQ ID NO: 24 entspricht.

19. Expressionskassette, die einen Promoter in operativer Verknüpfung mit dem Polynukleotidmolekül nach einem der Ansprüche 15 - 18 umfasst.

20. Expressionskassette nach Anspruch 19, wobei der Promoter fähig ist, die Genexpression in einem Bakterium, einer Pilzzelle oder einer Pflanzenzelle voranzutreiben.

21. Nichthumane Wirtszelle, die mit der Expressionskassette nach Anspruch 19 oder 20 transformiert ist.

22. Wirtszelle nach Anspruch 21, wobei die Wirtszelle aus der Gruppe bestehend aus einem Bakterium, einer Pilzzelle und einer Pflanzenzelle ausgewählt ist.

23. Transformierte Pflanze, die ein Polynukleotidkonstrukt, das eine Nukleotidsequenz in operativer Verknüpfung mit einem Promoter, der die Expression in einer Pflanzenzelle vorantreibt, umfasst, stabil in ihr Genom eingebaut enthält, wobei die Nukleotidsequenz aus der Gruppe ausgewählt ist, die aus Folgendem besteht:
(a) der Nukleotidsequenz gemäß SEQ ID NO: 1;
(b) Nukleotidsequenzen, die für die Aminosäuresequenz gemäß SEQ ID NO: 2 kodieren;
(c) Nukleotidsequenzen mit mindestens 75% Sequenzidentität zu der Nukleotidsequenz gemäß SEQ ID NO: 1, wobei die Nukleotidsequenz für ein Protein kodiert, das eine Isoleucinsubstitution an der Aminosäureposition, die Position 188 von SEQ ID NO: 24 entspricht, umfasst, wobei das Protein imidazolinonherbizidtolerante AHAS-Aktivität bereitstellt; und
(d) Nukleotidsequenzen, die für ein Protein kodieren, das mindestens 75% Sequenzidentität zu der Aminosäuresequenz gemäß SEQ ID NO: 2 aufweist, wobei das Protein eine Isoleucinsubstitution an der Aminosäureposition, die Position 188 von SEQ ID NO: 24 entspricht, aufweist, wobei das Protein imidazolinonherbizidtolerante AHAS-Aktivität bereitstellt.

24. Transformierte Pflanze nach Anspruch 23, wobei die Nukleotidsequenz wie in SEQ ID NO: 1 angegeben ist.

25. Transformierte Pflanze nach Anspruch 23, wobei die Nukleotidsequenz für die Aminosäuresequenz gemäß SEQ ID NO: 2 kodiert.

26. Transformierte Pflanze nach einem der Ansprüche 23 - 25, wobei der Promoter aus der Gruppe bestehend aus konstitutiven Promotern und Promotern mit Gewebebevorzugung ausgewählt ist.

27. Transformierte Pflanze nach einem der Ansprüche 23 - 26, wobei das Polynukleotidkonstrukt weiterhin eine operativ verknüpfte Chloroplasten-Zielsteuerungssequenz umfasst.

28. Transformierte Pflanze nach einem der Ansprüche 23 - 27, wobei die AHAS-Aktivität der transformierten Pflanze im Vergleich zu einer untransformierten Pflanze erhöht ist.

29. Transformierte Pflanze nach einem der Ansprüche 23 - 28, wobei die Toleranz der transformierten Pflanze gegenüber einem Imidazolinonherbizid im Vergleich zu einer Wildtyp-Sonnenblumenpflanze erhöht ist.

30. Transformierte Pflanze nach Anspruch 29, wobei das Imidazolinonherbizid aus der Gruppe bestehend aus: 2-(4-Isopropyl-4-methyl-5-oxo-2-imidazolin-2-yl)nikotinsäure, 2-(4-Isopropyl-4-methyl-5-oxo-2-imidazolin-2-yl)-3-chinolincarbonsäure, 5-Ethyl-2-(4-isopropyl-4-methyl-5-oxo-2-imidazolin-2-yl)nikotinsäure, 2-(4-Isopropyl-4-methyl-5-oxo-2-imidazolin-2-yl)-5-(methoxymethyl)nikotinsäure, 2-(4-Isopropyl-4-methyl-5-oxo-2-imidazolin-2-yl)-5-methylnikotinsäure, Mischungen aus Methyl-6-(4-isopropyl-4-methyl-5-oxo-2-imidazolin-2-yl)-m-toluat und Methyl-2-(4-isopropyl-4-methyl-5-oxo-2-imidazolin-2-yl)-p-toluat und Kombinationen davon ausgewählt ist.

31. Transformierte Pflanze nach einem der Ansprüche 23 - 30, wobei es sich bei der transformierten Pflanze um eine dikotyle oder eine monokotyle Pflanze handelt.

32. Transformierte Pflanze nach Anspruch 31, wobei die dikotyle Pflanze aus der Gruppe bestehend aus Sonnenblume, Sojabohne, Baumwolle, *Brassica* spp., *Arabidopsis thaliana,* Tabak, Kartoffel, Zuckerrübe, Luzerne, Saflor und Erdnuss ausgewählt ist und wobei die monokotyle Pflanze aus der Gruppe bestehend aus Weizen, Reis, Mais, Gerste, Roggen, Hafer, Triticale, Hirse und Sorghum ausgewählt ist.

33. Samen der transformierten Pflanze nach einem der Ansprüche 23 - 32, wobei der Samen das Polynukleotidkonstrukt umfasst.

34. Verfahren zur Herstellung einer imidazolinonherbizidtoleranten Pflanze, wobei das Verfahren umfasst, dass man eine Pflanzenzelle mit einem Polynukleotidkonstrukt, das eine Nukleotidsequenz in operativer Verknüpfung mit einem Promoter, der die Expression in einer Pflanzenzelle vorantreibt, umfasst, transformiert und die imidazolinonherbizidtolerante Pflanze aus der transformierten Pflanzenzelle regeneriert, wobei die Nukleotidsequenz aus der Gruppe ausgewählt ist, die aus Folgendem besteht:
(a) der Nukleotidsequenz gemäß SEQ ID NO: 1;
(b) Nukleotidsequenzen, die für die Aminosäuresequenz gemäß SEQ ID NO: 2 kodieren;
(c) Nukleotidsequenzen mit mindestens 75% Sequenzidentität zu der Nukleotidsequenz gemäß SEQ ID NO: 1, wobei die Nukleotidsequenz für ein Protein kodiert, das eine Isoleucinsubstitution an der Aminosäureposition, die Position 188 von SEQ ID NO: 24 entspricht, umfasst, wobei das Protein imidazolinonherbizidtolerante AHAS-Aktivität bereitstellt;
(d) Nukleotidsequenzen, die für ein Protein kodieren, das mindestens 75% Sequenzidentität zu der Aminosäuresequenz gemäß SEQ ID NO: 2 aufweist, wobei das Protein eine Isoleucinsubstitution an der Aminosäureposition, die Position 188 von SEQ ID NO: 24 entspricht, umfasst, wobei das Protein imidazolinonherbizidtolerante AHAS-Aktivität bereitstellt;
wobei die transformierte Pflanze eine erhöhte Imidazolinonherbizidtoleranz im Vergleich zu der Toleranz einer untransformierten Pflanze gegenüber diesem Herbizid aufweist.

35. Verfahren nach Anspruch 34, wobei die Nukleotidsequenz wie in SEQ ID NO: 1 angegeben ist.

36. Verfahren nach Anspruch 34, wobei die Nukleotidsequenz für die Aminosäuresequenz gemäß SEQ ID NO: 2 kodiert.

37. Verfahren nach einem der Ansprüche 34 - 36, wobei der Promoter aus der Gruppe bestehend aus konstitutiven Promotern und Promotern mit Gewebebevorzugung ausgewählt ist.

38. Verfahren nach einem der Ansprüche 34 - 37, wobei das Polynukleotidkonstrukt weiterhin eine operativ verknüpfte Chloroplasten-Zielsteuerungssequenz umfasst.

39. Verfahren nach einem der Ansprüche 34 - 38, wobei die AHAS-Aktivität der transformierten Pflanze im Vergleich zu einer untransformierten Pflanze erhöht ist.

40. Verfahren nach einem der Ansprüche 34 - 39, wobei das Imidazolinonherbizid aus der Gruppe bestehend aus: 2-(4-Isopropyl-4-methyl-5-oxo-2-imidazolin-2-yl)nikotinsäure, 2-(4-Isopropyl-4-methyl-5-oxo-2-imidazolin-2-yl)-3-chinolincarbonsäure, 5-Ethyl-2-(4-isopropyl-4-methyl-5-oxo-2-imidazolin-2-yl)nikotinsäure, 2-(4-Isopropyl-4-methyl-5-oxo-2-imidazolin-2-yl)-5-(methoxymethyl)nikotinsäure, 2-(4-Isopropyl-4-methyl-5-oxo-2-imidazolin-2-yl)-5-methylnikotinsäure, Mischungen aus Methyl-6-(4-isopropyl-4-methyl-5-oxo-2-imidazolin-2-yl)-m-toluat und Methyl-2-(4-isopropyl-4-methyl-5-oxo-2-imidazolin-2-yl)-p-toluat und Kombinationen davon ausgewählt ist.

41. Verfahren nach einem der Ansprüche 34 - 40, wobei es sich bei der imidazolinonherbizidtoleranten Pflanze um eine dikotyle oder eine monokotyle Pflanze handelt.

42. Verfahren nach Anspruch 41, wobei die dikotyle Pflanze aus der Gruppe bestehend aus Sonnenblume, Sojabohne, Baumwolle, *Brassica* spp., *Arabidopsis thaliana,* Tabak, Kartoffel, Zuckerrübe, Luzerne, Saflor und Erdnuss ausgewählt ist und wobei die monokotyle Pflanze aus der Gruppe bestehend aus Weizen, Reis, Mais, Gerste, Roggen, Hafer, Triticale, Hirse und Sorghum ausgewählt ist.

43. Verfahren nach einem der Ansprüche 34 - 42, wobei die Pflanzenzelle vor dem Transformationsschritt Toleranz gegenüber mindestens einem Herbizid umfasst.

44. Verfahren zur Bekämpfung von Unkräutern in der Umgebung einer transformierten Pflanze, wobei das Verfahren umfasst, dass man auf die Unkräuter und auf die transformierte Pflanze eine wirksame Menge eines Imidazolinonherbizids appliziert, wobei die transformierte Pflanze gemäß einem der Ansprüche 23 - 32 ist.

45. Verfahren nach Anspruch 44, wobei das Imidazolinonherbizid aus der Gruppe bestehend aus: 2-(4-Isopropyl-4-methyl-5-oxo-2-imidazolin-2-yl)nikotinsäure, 2-(4-Isopropyl-4-methyl-5-oxo-2-imidazolin-2-yl)-3-chinolincarbonsäure, 5-Ethyl-2-(4-isopropyl-4-methyl-5-oxo-2-imidazolin-2-yl)nikotinsäure, 2-(4-Isopropyl-4-methyl-5-oxo-2-imidazolin-2-yl)-5-(methoxymethyl)nikotinsäure, 2-(4-Isopropyl-4-methyl-5-oxo-2-imidazolin-2-yl)-5-methylnikotinsäure, Mischungen aus Methyl-6-(4-isopropyl-4-methyl-5-oxo-2-imidazolin-2-yl)-m-toluat und Methyl-2-(4-isopropyl-4-methyl-5-oxo-2-imidazolin-2-yl)-p-toluat und Kombinationen davon ausgewählt ist.

46. Isoliertes Polypeptid, das eine Aminosäuresequenz umfasst, die aus der Gruppe bestehend aus Folgendem ausgewählt ist:
(a) der Aminosäuresequenz gemäß SEQ ID NO: 2;
(b) der Aminosäuresequenz, die von der Nukleotidsequenz gemäß SEQ ID NO: 1 kodiert wird;
(c) Aminosäuresequenzen mit mindestens 75% Sequenzidentität zu der Aminosäuresequenz gemäß SEQ ID NO: 2, wobei das Polypeptid eine Isoleucinsubstitution an der Aminosäureposition, die Position 188 von SEQ ID NO: 24 entspricht, umfasst, wobei das Polypeptid imidazolinonherbizidtolerante AHAS-Aktivität bereitstellt; und
(d) den Aminosäuresequenzen, die von Nukleotidsequenzen mit mindestens 75% Sequenzidentität zu der Nukleotidsequenz gemäß SEQ ID NO: 1 kodiert werden, wobei die Nukleotidsequenz für ein Protein kodiert, das eine Isoleucinsubstitution an der Aminosäureposition, die Position 188 von SEQ ID NO: 24 entspricht, umfasst, wobei das Protein eine imidazolinonherbizidtolerante AHAS-Aktivität bereitstellt.

47. Isoliertes Polypeptid nach Anspruch 46, wobei die Aminosäuresequenz von der Nukleotidsequenz gemäß SEQ ID NO: 1 kodiert wird.

48. Isoliertes Polypeptid nach Anspruch 46, wobei die Aminosäuresequenz wie in SEQ ID NO: 2 dargestellt ist.

49. Samen nach einem der Ansprüche 6, 12 und 33, wobei der Samen mit einem AHAS-hemmenden Herbizid behandelt ist.

50. Verfahren zum Bekämpfen von unerwünschtem Pflanzenwuchs, bei dem man den Samen nach einem der Ansprüche 6, 12, 33 und 49 vor dem Säen und/oder nach dem Vorkeimen mit einem AHAS-hemmenden Herbizid in Kontakt bringt.

## Revendications

1. Plante de tournesol comprenant dans son génome au moins une copie d'au moins un polynucléotide codant pour la grande sous-unité de l'acétohydroxyacide-synthase (AHASL), ledit polynucléotide AHASL codant pour une protéine AHASL tolérante à l'herbicide imidazolinone comprenant une isoleucine substituée au niveau de la position d'acide aminé équivalente à la position 188 de SEQ ID N° 24, ladite plante ayant une tolérance accrue à un herbicide imidazolinone, par comparaison avec la tolérance d'une plante de tournesol de type sauvage à l'herbicide imidazolinone.

2. Plante de tournesol de la revendication 1, dans laquelle ladite protéine AHASL comprend la séquence d'acides aminés présentée dans SEQ ID N° 2.

3. Plante de tournesol de la revendication 1 ou 2, dans laquelle ledit polynucléotide AHASL comprend la séquence nucléotidique présentée dans SEQ ID N° 1.

4. Plante de tournesol de la revendication 1, dans laquelle ladite protéine AHASL comprend en outre :
(a) une thréonine substituée au niveau de la position d'acide aminé équivalente à la position 107 de SEQ ID N° 24 ;
(b) une alanine, une thréonine, une histidine, une leucine, une arginine, une isoleucine, une glutamine ou une sérine, substituée au niveau de la position d'acide aminé équivalente à la position 182 de SEQ ID N° 24 ;
(c) un aspartate ou une valine, substitué au niveau de la position d'acide aminé équivalente à la position 190 de SEQ ID N° 24 ;
(d) une leucine substituée au niveau de la position d'acide aminé équivalente à la position 559 de SEQ ID N° 24 ; ou
(e) une asparagine, une thréonine ou une phénylalanine substituée au niveau de la position d'acide aminé équivalente à la position 638 de SEQ ID N° 24.

5. Plante de tournesol de l'une quelconque des revendications 1 à 4, ladite plante étant transgénique ou non transgénique.

6. Graine de la plante de tournesol de l'une quelconque des revendications 1 à 5, ladite graine comprenant dans son génome au moins une copie dudit polynucléotide AHASL.

7. Plante de tournesol de la revendication 1, ladite plante étant choisie dans le groupe consistant en :
(a) les descendants de toutes plantes de la lignée MUT9, un échantillon représentatif de la graine de la lignée ayant été déposé sous le numéro de dépôt de brevet ATCC PTA-6325 ;
(b) les mutants, recombinants ou dérivés génétiquement modifiés de toutes plantes de la lignée MUT9 ; et
(c) les descendants de l'un quelconque de (a)-(b).

8. Plante de tournesol de la revendication 7, ladite plante étant un descendant d'une plante de la lignée MUT9, un échantillon représentatif de la graine de la lignée ayant été déposé sous le numéro de dépôt de brevet ATCC PTA-6325.

9. Plante de tournesol de la revendication 7, ladite plante étant un mutant, un recombinant ou une dérivé génétiquement modifié d'une plante de la lignée MUT9, un échantillon représentatif de la graine de la lignée ayant été déposé sous le numéro de dépôt de brevet ATCC PTA-6325.

10. Plante de tournesol de la revendication 7, ladite plante étant un descendant d'un tournesol de l'une quelconque des revendications 8 ou 9.

11. Plante de tournesol de l'une quelconque des revendications 7 à 10, ladite plante étant transgénique ou non transgénique.

12. Graine de la plante de tournesol de l'une quelconque des revendications 7-11, ladite graine comprenant la ou les copies du ou des polynucléotides AHASL codant pour la protéine AHASL tolérante à l'herbicide imidazolinone.

13. Procédé de maîtrise des mauvaises herbes au voisinage d'une plante de tournesol, ledit procédé comprenant l'application d'une quantité efficace d'un herbicide imidazolinone aux mauvaises herbes et à la plante de tournesol, ladite plante de tournesol étant une plante de tournesol de l'une quelconque des revendications 1-5 et 7-11, ou une plante de tournesol de la lignée MUT9, un échantillon représentatif de la graine de la lignée ayant été déposé sous le numéro de dépôt de brevet ATCC PTA-6325.

14. Procédé de la revendication 13, dans lequel ledit herbicide imidazolinone est choisi dans le groupe consistant en l'acide 2-(4-isopropyl-4-méthyl-5-oxo-2-imidazolin-2-yl)-nicotinique, l'acide 2-(4-isopropyl-4-méthyl-5-oxo-2-imidazolin-2-yl)-3-quinoléinecarboxylique, l'acide 5-éthyl-2-(4-isopropyl-4-méthyl-5-oxo-2-imidazolin-2-yl)-nicotinique, l'acide 2-(4-isopropyl-4-méthyl-5-oxo-2-imidazolin-2-yl)-5-(méthoxyméthyl)-nicotinique, l'acide 2-(4-isopropyl-4-méthyl-5-oxo-2-imidazolin-2-yl)-5-méthylnicotinique, les mélanges de 6-(4-isopropyl-4-méthyl-5-oxo-2-imidazolin-2-yl)-m-toluate de méthyle et de 2-(4-isopropyl-4-méthyl-5-oxo-2-imidazolin-2-yl)-p-toluate de méthyle, et les combinaisons de ceux-ci.

15. Molécule polynucléotidique isolée comprenant une séquence nucléotidique choisie dans le groupe consistant en :
(a) la séquence nucléotidique présentée dans SEQ ID N° 1 ;
(b) les séquences nucléotidiques codant pour la séquence d'acides aminés présentée dans SEQ ID N° 2 ;
(c) les séquences nucléotidiques ayant une identité de séquence d'au moins 75 % avec la séquence nucléotidique présentée dans SEQ ID N° 1, ladite séquence nucléotidique codant pour une protéine comprenant une isoleucine substituée au niveau de la position d'acide aminé équivalente à la position 188 de SEQ ID N° 24, ladite protéine fournissant une activité d'acétohydroxyacide-synthase (AHAS) tolérante à l'herbicide imidazolinone ;
(d) les séquences nucléotidiques codant pour une protéine ayant une identité de séquence d'au moins 75 % avec la séquence d'acides aminés présentée dans SEQ ID N° 2, ladite protéine comprenant une isoleucine substituée au niveau de la position d'acide aminé équivalente à la position 188 de SEQ ID N° 24, ladite protéine fournissant une activité d'AHAS tolérante à l'herbicide imidazolinone ; et
(e) une séquence nucléotidique qui est entièrement complémentaire de l'une quelconque des séquences présentées dans (a)-(d).

16. Molécule polynucléotidique isolée de la revendication 15, dans lequel la séquence nucléotidique est présentée dans SEQ ID N° 1.

17. Molécule polynucléotidique isolée de la revendication 15, dans lequel la séquence nucléotidique code pour la séquence d'acides aminés présentée dans SEQ ID N° 2.

18. Molécule polynucléotidique isolée de l'une quelconque des revendications 15 à 17, dans laquelle ladite protéine codée par la séquence nucléotidique de (c) ou (d) comprend en outre :
(a) une thréonine substituée au niveau de la position d'acide aminé équivalente à la position 107 de SEQ ID N° 24 ;
(b) une alanine, une thréonine, une histidine, une leucine, une arginine, une isoleucine, une glutamine ou une sérine, substituée au niveau de la position d'acide aminé équivalente à la position 182 de SEQ ID N° 24 ;
(c) un aspartate ou une valine, substitué au niveau de la position d'acide aminé équivalente à la position 190 de SEQ ID N° 24 ;
(d) une leucine substituée au niveau de la position d'acide aminé équivalente à la position 559 de SEQ ID N° 24 ; ou
(e) une asparagine, une thréonine ou une phénylalanine substituée au niveau de la position d'acide aminé équivalente à la position 638 de SEQ ID N° 24.

19. Cassette d'expression comprenant un promoteur lié de manière opérationnelle à la molécule de polynucléotide de l'une quelconque des revendications 15 à 18.

20. Cassette d'expression de la revendication 19, dans laquelle ledit promoteur est capable de commander l'expression génique dans une bactérie, une cellule fongique ou une cellule végétale.

21. Cellule hôte non humaine transformée avec la cassette d'expression de la revendication 19 ou 20.

22. Cellule hôte de la revendication 21, ladite cellule hôte étant choisie dans le groupe consistant en une bactérie, une cellule fongique et une cellule végétale.

23. Plante transformée comprenant, incorporée d'une manière stable dans son génome, une construction polynucléotidique comprenant une séquence nucléotidique liée de manière opérationnelle à un promoteur qui commande l'expression dans une cellule végétale, ladite séquence nucléotidique étant choisie dans le groupe consistant en :
(a) la séquence nucléotidique présentée dans SEQ ID N° 1 ;
(b) les séquences nucléotidiques codant pour la séquence d'acides aminés présentée dans SEQ ID N° 2 ;
(c) les séquences nucléotidiques ayant une identité de séquence d'au moins 75 % avec la séquence nucléotidique présentée dans SEQ ID N° 1, ladite séquence nucléotidique codant pour une protéine comprenant une isoleucine substituée au niveau de la position d'acide aminé équivalente à la position 188 de SEQ ID N° 24, ladite protéine fournissant une activité d'AHAS tolérante à l'herbicide imidazolinone ; et
(d) les séquences nucléotidiques codant pour une protéine ayant une identité de séquence d'au moins 75 % avec la séquence d'acides aminés présentée dans SEQ ID N° 2, ladite protéine comprenant une isoleucine substituée au niveau de la position d'acide aminé équivalente à la position 188 de SEQ ID N° 24, ladite protéine fournissant une activité d'AHAS tolérante à l'herbicide imidazolinone.

24. Plante transformée de la revendication 23, dans laquelle la séquence nucléotidique est telle que présentée dans SEQ ID N° 1.

25. Plante transformée de la revendication 23, dans laquelle la séquence nucléotidique code pour la séquence d'acides aminés présentée dans SEQ ID N° 2.

26. Plante transformée de l'une quelconque des revendications 23 à 25, dans laquelle ledit promoteur est choisi dans le groupe consistant en les promoteurs constitutifs et les promoteurs à préférence de tissu.

27. Plante transformée de l'une quelconque des revendications 23 à 26, dans laquelle ladite construction polynucléotidique comprend en outre une séquence de ciblage chloroplastique liée de manière opérationnelle.

28. Plante transformée de l'une quelconque des revendications 23 à 27, dans laquelle l'activité d'AHAS de ladite plante transformée est augmentée par rapport à celle d'une plante non transformée.

29. Plante transformée de l'une quelconque des revendications 23 à 28, dans laquelle la tolérance de ladite plante transformée à un herbicide imidazolinone est augmentée par comparaison avec une plante de tournesol de type sauvage.

30. Plante transformée de la revendication 29, dans laquelle ledit herbicide imidazolinone est choisi dans le groupe consistant en l'acide 2-(4-isopropyl-4-méthyl-5-oxo-2-imidazolin-2-yl)-nicotinique, l'acide 2-(4-isopropyl-4-méthyl-5-oxo-2-imidazolin-2-yl)-3-quinoléinecarboxylique, l'acide 5-éthyl-2-(4-isopropyl-4-méthyl-5-oxo-2-imidazolin-2-yl)-nicotinique, l'acide 2-(4-isopropyl-4-méthyl-5-oxo-2-imidazolin-2-yl)-5-(méthoxyméthyl)-nicotinique, l'acide 2-(4-isopropyl-4-méthyl-5-oxo-2-imidazolin-2-yl)-5-méthylnicotinique, les mélanges de 6-(4-isopropyl-4-méthyl-5-oxo-2-imidazolin-2-yl)-m-toluate de méthyle et de 2-(4-isopropyl-4-méthyl-5-oxo-2-imidazolin-2-yl)-p-toluate de méthyle, et les combinaisons de ceux-ci.

31. Plante transformée de l'une quelconque des revendications 23 à 30, ladite plante transformée étant une dicotylédone ou une monocotylédone.

32. Plante transformée de la revendication 31, ladite dicotylédone étant choisie dans le groupe consistant en le tournesol, le soja, le coton, *Brassica* spp., *Arabidopsis thaliana,* le tabac, la pomme de terre, la betterave à sucre, la luzerne, le carthame et l'arachide, ladite monocotylédone étant choisie dans le groupe consistant en le blé, le riz, le maïs, l'orge, le seigle, l'avoine, le triticale, le millet et le sorgho.

33. Graine de la plante transformée de l'une quelconque des revendications 23 à 32, ladite graine comprenant ladite construction polynucléotidique.

34. Procédé de production d'une plante tolérante à l'herbicide imidazolinone, le procédé comprenant la transformation d'une cellule végétale avec une construction polynucléotidique comprenant une séquence nucléotidique liée de manière opérationnelle à un promoteur qui commande l'expression dans une cellule végétale, et la régénération de la plante tolérante à l'herbicide imidazolinone à partir de ladite cellule végétale transformée, ladite séquence nucléotidique étant choisie dans le groupe consistant en :
(a) la séquence nucléotidique présentée dans SEQ ID N° 1 ;
(b) les séquences nucléotidiques codant pour la séquence d'acides aminés présentée dans SEQ ID N° 2 ;
(c) les séquences nucléotidiques ayant une identité de séquence d'au moins 75 % avec la séquence nucléotidique présentée dans SEQ ID N° 1, ladite séquence nucléotidique codant pour une protéine comprenant une isoleucine substituée au niveau de la position d'acide aminé équivalente à la position 188 de SEQ ID N° 24, ladite protéine fournissant une activité d'AHAS tolérante à l'herbicide imidazolinone ; et
(d) les séquences nucléotidiques codant pour une protéine ayant une identité de séquence d'au moins 75 % avec la séquence d'acides aminés présentée dans SEQ ID N° 2, ladite protéine comprenant une isoleucine substituée au niveau de la position d'acide aminé équivalente à la position 188 de SEQ ID N° 24, ladite protéine fournissant une activité d'AHAS tolérante à l'herbicide imidazolinone ;
ladite plante transformée ayant une tolérances accrue à un herbicide imidazolinone, par comparaison avec la tolérance d'une plante non transformée audit herbicide.

35. Procédé de la revendication 34, dans lequel la séquence nucléotidique est telle que présentée dans SEQ ID N° 1.

36. Procédé de la revendication 34, dans lequel la séquence nucléotidique code pour la séquence d'acides aminés présentée dans SEQ ID N° 2.

37. Procédé de l'une quelconque des revendications 34 à 36, dans lequel ledit promoteur est choisi dans le groupe consistant en les promoteurs constitutifs et les promoteurs à préférence de tissu.

38. Procédé de l'une quelconque des revendications 34 à 37, dans lequel ladite construction polynucléotidique comprend en outre une séquence de ciblage chloroplastique liée de manière opérationnelle.

39. Procédé de l'une quelconque des revendications 34 à 38, dans lequel l'activité d'AHAS de ladite plante transformée est augmentée par comparaison avec celle d'une plante non transformée.

40. Procédé de l'une quelconque des revendications 34 à 39, dans lequel ledit herbicide imidazolinone est choisi dans le groupe consistant en l'acide 2-(4-isopropyl-4-méthyl-5-oxo-2-imidazolin-2-yl)-nicotinique, l'acide 2-(4-isopropyl-4-méthyl-5-oxo-2-imidazolin-2-yl)-3-quinoléinecarboxylique, l'acide 5-éthyl-2-(4-isopropyl-4-méthyl-5-oxo-2-imidazolin-2-yl)-nicotinique, l'acide 2-(4-isopropyl-4-méthyl-5-oxo-2-imidazolin-2-yl)-5-(méthoxyméthyl)-nicotinique, l'acide 2-(4-isopropyl-4-méthyl-5-oxo-2-imidazolin-2-yl)-5-méthylnicotinique, les mélanges de 6-(4-isopropyl-4-méthyl-5-oxo-2-imidazolin-2-yl)-m-toluate de méthyle et de 2-(4-isopropyl-4-méthyl-5-oxo-2-imidazolin-2-yl)-p-toluate de méthyle, et les combinaisons de ceux-ci.

41. Procédé de l'une quelconque des revendications 34 à 40, dans lequel ladite plante tolérante à l'herbicide imidazolinone est une dicotylédone ou une monocotylédone.

42. Procédé de la revendication 41, dans lequel ladite dicotylédone est choisie dans le groupe consistant en le tournesol, le soja, le coton, *Brassica* spp., *Arabidopsis thaliana,* le tabac, la pomme de terre, la betterave à sucre, la luzerne, le carthame et l'arachide, et dans lequel ladite monocotylédone est choisie dans le groupe consistant en le blé, le riz, le maïs, l'orge, le seigle, l'avoine, le triticale, le millet et le sorgho.

43. Procédé de l'une quelconque des revendications 34 à 42, dans lequel ladite cellule végétale comprend une tolérance à au moins un herbicide, avant ladite étape de transformation.

44. Procédé de maîtrise de mauvaises herbes au voisinage d'une plante transformée, ledit procédé comprenant l'application d'une quantité efficace d'un herbicide imidazolinone aux mauvaises herbes et à la plante transformée, ladite plante transformée étant une plante selon l'une quelconque des revendications 23 à 32.

45. Procédé de la revendication 44, dans lequel ledit herbicide imidazolinone est choisi dans le groupe consistant en : l'acide 2-(4-isopropyl-4-méthyl-5-oxo-2-imidazolin-2-yl)-nicotinique, l'acide 2-(4-isopropyl-4-méthyl-5-oxo-2-imidazolin-2-yl)-3-quinoléinecarboxylique, l'acide 5-éthyl-2-(4-isopropyl-4-méthyl-5-oxo-2-imidazolin-2-yl)-nicotinique, l'acide 2-(4-isopropyl-4-méthyl-5-oxo-2-imidazolin-2-yl)-5-(méthoxyméthyl)-nicotinique, l'acide 2-(4-isopropyl-4-méthyl-5-oxo-2-imidazolin-2-yl)-5-méthylnicotinique,
les mélanges de 6-(4-isopropyl-4-méthyl-5-oxo-2-imidazolin-2-yl)-m-toluate de méthyle et de 2-(4-isopropyl-4-méthyl-5-oxo-2-imidazolin-2-yl)-p-toluate de méthyle, et les combinaisons de ceux-ci.

46. Polypeptide isolé comprenant une séquence d'acides aminés choisie dans le groupe consistant en :
(a) la séquence d'acides aminés présentée dans SEQ ID N° 2 ;
(b) la séquence d'acides aminés codée par la séquence nucléotidique présentée dans SEQ ID N° 1 ;
(c) les séquences d'acides aminés ayant une identité de séquence d'au moins 75 % avec la séquence d'acides aminés présentée dans SEQ ID N° 2, ledit polypeptide comprenant une isoleucine substituée au niveau de la position d'acide aminé équivalente à la position 188 de SEQ ID N° 24, ledit polypeptide fournissant une activité d'AHAS tolérante à l'herbicide imidazolinone ; et
(d) les séquences d'acides aminés codées par les séquences nucléotidiques ayant une identité de séquence d'au moins 75 % avec la séquence nucléotidique présentée dans SEQ ID N° 1, ladite séquence nucléotidique codant pour une protéine comprenant une isoleucine substituée au niveau de la position d'acide aminé équivalente à la position 188 de SEQ ID N° 24, ladite protéine fournissant une activité d'AHAS tolérante à l'herbicide imidazolinone.

47. Polypeptide isolé de la revendication 46, dans lequel la séquence d'acides aminés est codée par la séquence nucléotidique présentée dans SEQ ID N° 1.

48. Polypeptide isolé de la revendication 46, dans lequel la séquence d'acides aminés est telle que présentée dans SEQ ID N° 2.

49. Graine de l'une quelconque des revendications 6, 12 et 33, ladite graine étant traitée avec un herbicide inhibant l'AHAS.

50. Procédé pour combattre la végétation indésirable, comprenant la mise en contact de la graine de l'une quelconque des revendications 6, 12, 33 et 49 avant le semis et/ou après la pré-germination, avec un herbicide inhibant l'AHAS.


### SEQUENCE LISTING

<110> Leon, Alberto Javier
   Morata, Monica Mariel
   Olungu, Christine
<120> HERBICIDE-RESISTANT SUNFLOWER PLANTS,
   POLYNUCLEOTIDES ENCODING HERBICIDE-RESISTANT
   ACETOHYDROXYACID SYNTHASE LARGE SUBUNIT PROTEINS, AND METHODS OF USE
<130> 038867-318321
<150> US 60/735,045
   <151> 2005-11-09
<160> 26
<170> FastSEQ for Windows Version 4.0
<210> 1
   <211> 1684
   <212> DNA
   <213> Helianthus annus
<220>
   <221> CDS
   <222> (1)...(1684)
<221> misc_feature
   <222> (0) ... (0)
   <223> MUT9
<400> 1
   gtg ttg gtg gaa gct ctg gaa cgg gaa ggt gtc acc gac gtc ttc gcc      48
   Val Leu Val Glu Ala Leu Glu Arg Glu Gly Val Thr Asp Val Phe Ala
    1               5                  10                  15
   tac ccc ggc ggc gcg tca atg gag atc cac caa gct ctc acg cgc tca      96
   Tyr Pro Gly Gly Ala Ser Met Glu Ile His Gln Ala Leu Thr Arg Ser
               20                  25                  30
   aac act atc cgc aat gtc ctc cca cgt cac gaa cag ggc ggc gtg ttc     144
   Asn Thr Ile Arg Asn Val Leu Pro Arg His Glu Gln Gly Gly Val Phe
           35                  40                  45
   gcc gcc gaa ggc tac gcg cgc gcc tcc ggt ctt ccc ggc gtg tgt atc     192
   Ala Ala Glu Gly Tyr Ala Arg Ala Ser Gly Leu Pro Gly Val Cys Ile
       50                  55                  60
   gcc act tcc ggt ccc gga gct acg aac cta gtt agt ggt ctt gct gac     240
   Ala Thr Ser Gly Pro Gly Ala Thr Asn Leu Val Ser Gly Leu Ala Asp
    65                 70                  75                  80
   gcg ctg tta gac agt gtc ccc atg gtg gca atc acc ggt caa gtt ccc     288
   Ala Leu Leu Asp Ser Val Pro Met Val Ala Ile Thr Gly Gln Val Pro
                   85                  90                  95
   cgg aga atg atc gga atc gat gcg ttt caa gaa acc cca att gtt gag     336
   Arg Arg Met Ile Gly Ile Asp Ala Phe Gln Glu Thr Pro Ile Val Glu
                   100                 105                 110
   gta aca cgt tcg atc act aaa cat aat tat ctt gtg ttg gat gtt gag     384
   Val Thr Arg Ser Ile Thr Lys His Asn Tyr Leu Val Leu Asp Val Glu
               115                 120                 125
   gat att ccc aga att gtt cgt gag gct ttt tat ctt gcg agt tcg ggt     432
   Asp Ile Pro Arg Ile Val Arg Glu Ala Phe Tyr Leu Ala Ser Ser Gly
       130                 135                 140
   cga ccc ggc ccg gtt ttg ata gat gta ccg aaa gat ata cag caa cag     480
   Arg Pro Gly Pro Val Leu Ile Asp Val Pro Lys Asp Ile Gln Gln Gln
   145                 150                 155                 160
   tta gtg gtg ccg aaa tgg gat gaa ccg atg agg tta ccg ggt tat ttg     528
   Leu Val Val Pro Lys Trp Asp Glu Pro Met Arg Leu Pro Gly Tyr Leu
                   165                 170                 175
   tct aga atg ccg aag cct caa tat gat ggg cat ttg gaa cag att gtt     576
   Ser Arg Met Pro Lys Pro Gln Tyr Asp Gly His Leu Glu Gln Ile Val
               180                 185                 190
   agg ttg gtg ggg gaa gcg aag agg ccg gtt ttg tat gtg ggt ggt ggg     624
   Arg Leu Val Gly Glu Ala Lys Arg Pro Val Leu Tyr Val Gly Gly Gly
           195                 200                 205
   tgt ttg aat tcg gat gat gag ttg agg cgg ttt gtg gag ctt acg ggg     672
   Cys Leu Asn Ser Asp Asp Glu Leu Arg Arg Phe Val Glu Leu Thr Gly
       210                 215                 220
   att ccg gtt gcg agt act ttg atg ggg ctc gga gcg tac ccg gct tcg     720
   Ile Pro Val Ala Ser Thr Leu Met Gly Leu Gly Ala Tyr Pro Ala Ser
   225                 230                 235                 240
   agt gat ttg tcg ctt cat atg ctt ggg atg cat ggt acg gtt tat gcg     768
   Ser Asp Leu Ser Leu His Met Leu Gly Met His Gly Thr Val Tyr Ala
                   245                 250                 255
   aat tat gcg gtt gat aag agt gat ttg ttg ctt gcg ttt ggg gtg cgg     816
   Asn Tyr Ala Val Asp Lys Ser Asp Leu Leu Leu Ala Phe Gly Val Arg
               260                 265                 270
   ttt gat gac cgt gtg acg ggg aag ctt gag gcg ttt gct agt agg gcg     864
   Phe Asp Asp Arg Val Thr Gly Lys Leu Glu Ala Phe Ala Ser Arg Ala
           275                 280                 285
   aag att gtt cat att gat att gat ccg gct gaa att ggg aag aat aaa     912
   Lys Ile Val His Ile Asp Ile Asp Pro Ala Glu Ile Gly Lys Asn Lys
       290                 295                 300
   cag cct cat gtg tcg att tgt ggc gat att aag gtc gcg tta cag ggt     960
   Gln Pro His Val Ser Ile Cys Gly Asp Ile Lys Val Ala Leu Gln Gly
   305                 310                 315                 320
   ttg aac aag att ttg gag gaa aag aat tcg gtg act aat ctt gat ttt    1008
   Leu Asn Lys Ile Leu Glu Glu Lys Asn Ser Val Thr Asn Leu Asp Phe
                   325                 330                 335
   tcg aac tgg aga aag gaa ttg gat gaa caa aaa gtg aag ttc ccg ttg    1056
   Ser Asn Trp Arg Lys Glu Leu Asp Glu Gln Lys Val Lys Phe Pro Leu
               340                 345                 350
   agc ttt aaa acg ttt ggc gaa gcg att cct cca cag tat gct att caa     1104
   Ser Phe Lys Thr Phe Gly Glu Ala Ile Pro Pro Gln Tyr Ala Ile Gln
           355                 360                 365
   gtt ctt gat gag tta acg ggc ggg aat gca att att agc acc ggt gtc     1152
   Val Leu Asp Glu Leu Thr Gly Gly Asn Ala Ile Ile Ser Thr Gly Val
       370                 375                 380
   ggg caa cat cag atg tgg gct gct cag ttt tac aaa tac aac aaa cct     1200
   Gly Gln His Gln Met Trp Ala Ala Gln Phe Tyr Lys Tyr Asn Lys Pro
   385                 390                 395                 400
   aga caa tgg ctg acg tcg ggc ggg cta ggg gca atg ggt ttt ggg ctg     1248
   Arg Gln Trp Leu Thr Ser Gly Gly Leu Gly Ala Met Gly Phe Gly Leu
                   405                 410                 415
   ccc gct gct atc ggg gcg gcc gtt gca aga cct gat gcg gta gta gtt     1296
   Pro Ala Ala Ile Gly Ala Ala Val Ala Arg Pro Asp Ala Val Val Val
               420                 425                 430
   gac atc gac ggt gac gga agc ttt atg atg aat gtt caa gag tta gcc     1344
   Asp Ile Asp Gly Asp Gly Ser Phe Met Met Asn Val Gln Glu Leu Ala
           435                 440                 445
   aca atc cgt gtt gaa aat ctg ccg gtt aag att tta tta ctc aac aat     1392
   Thr Ile Arg Val Glu Asn Leu Pro Val Lys Ile Leu Leu Leu Asn Asn
       450                 455                 460
   cag cat ttg ggt atg gtg gtt cag tgg gag gat cgg ttt tac aag gcg     1440
   Gln His Leu Gly Met Val Val Gln Trp Glu Asp Arg Phe Tyr Lys Ala
   465                 470                 475                 480
   aat cgg gct cat acc tac tta gga aac ccg tca aaa gag tcg gaa ata     1488
   Asn Arg Ala His Thr Tyr Leu Gly Asn Pro Ser Lys Glu Ser Glu Ile
                   485                 490                 495
   ttc cct aac atg ttg aag ttt gcc gaa gcc tgt gat atc ccg gct gct     1536
   Phe Pro Asn Met Leu Lys Phe Ala Glu Ala Cys Asp Ile Pro Ala Ala
               500                 505                 510
   cga gtg acc caa aag gcg gat cta cga gca gct att cag aag atg ttg     1584
   Arg Val Thr Gln Lys Ala Asp Leu Arg Ala Ala Ile Gln Lys Met Leu
           515                 520                 525
   gat aca ccc ggg cct tac ttg ttg gat gtg att gtg ccg cat caa gaa     1632
   Asp Thr Pro Gly Pro Tyr Leu Leu Asp Val Ile Val Pro His Gln Glu
       530                 535                 540
   cac gtg ttg ccc atg atc ccg gct ggc gga ggt ttc tcg gat gtg atc     1680
   His Val Leu Pro Met Ile Pro Ala Gly Gly Gly Phe Ser Asp Val Ile
   545                 550                 555                 560
   acc g                                                               1684
   Thr
<210> 2
   <211> 561
   <212> PRT
   <213> Helianthus annus
<400> 2
   Val Leu Val Glu Ala Leu Glu Arg Glu Gly Val Thr Asp Val Phe Ala
    1               5                  10                  15
   Tyr Pro Gly Gly Ala Ser Met Glu Ile His Gln Ala Leu Thr Arg Ser
               20                  25                  30
   Asn Thr Ile Arg Asn Val Leu Pro Arg His Glu Gln Gly Gly Val Phe
           35                  40                  45
   Ala Ala Glu Gly Tyr Ala Arg Ala Ser Gly Leu Pro Gly Val Cys Ile
       50                  55                  60
   Ala Thr Ser Gly Pro Gly Ala Thr Asn Leu Val Ser Gly Leu Ala Asp
   65                  70                  75                  80
   Ala Leu Leu Asp Ser Val Pro Met Val Ala Ile Thr Gly Gln Val Pro
                   85                  90                  95
   Arg Arg Met Ile Gly Ile Asp Ala Phe Gln Glu Thr Pro Ile Val Glu
               100                 105                 110
   Val Thr Arg Ser Ile Thr Lys His Asn Tyr Leu Val Leu Asp Val Glu
           115                 120                 125
   Asp Ile Pro Arg Ile Val Arg Glu Ala Phe Tyr Leu Ala Ser Ser Gly
       130                 135                 140
   Arg Pro Gly Pro Val Leu Ile Asp Val Pro Lys Asp Ile Gln Gln Gln
   145                 150                 155                 160
   Leu Val Val Pro Lys Trp Asp Glu Pro Met Arg Leu Pro Gly Tyr Leu
                   165                 170                 175
   Ser Arg Met Pro Lys Pro Gln Tyr Asp Gly His Leu Glu Gln Ile Val
               180                 185                 190
   Arg Leu Val Gly Glu Ala Lys Arg Pro Val Leu Tyr Val Gly Gly Gly
           195                 200                 205
   Cys Leu Asn Ser Asp Asp Glu Leu Arg Arg Phe Val Glu Leu Thr Gly
       210                 215                 220
   Ile Pro Val Ala Ser Thr Leu Met Gly Leu Gly Ala Tyr Pro Ala Ser
   225                 230                 235                 240
   Ser Asp Leu Ser Leu His Met Leu Gly Met His Gly Thr Val Tyr Ala
                   245                 250                 255
   Asn Tyr Ala Val Asp Lys Ser Asp Leu Leu Leu Ala Phe Gly Val Arg
               260                 265                 270
   Phe Asp Asp Arg Val Thr Gly Lys Leu Glu Ala Phe Ala Ser Arg Ala
           275                 280                 285
   Lys Ile Val His Ile Asp Ile Asp Pro Ala Glu Ile Gly Lys Asn Lys
       290                 295                 300
   Gln Pro His Val Ser Ile Cys Gly Asp Ile Lys Val Ala Leu Gln Gly
   305                 310                 315                 320
   Leu Asn Lys Ile Leu Glu Glu Lys Asn Ser Val Thr Asn Leu Asp Phe
                   325                 330                 335
   Ser Asn Trp Arg Lys Glu Leu Asp Glu Gln Lys Val Lys Phe Pro Leu
               340                 345                 350
   Ser Phe Lys Thr Phe Gly Glu Ala Ile Pro Pro Gln Tyr Ala Ile Gln
           355                 360                 365
   Val Leu Asp Glu Leu Thr Gly Gly Asn Ala Ile Ile Ser Thr Gly Val
       370                 375                 380
   Gly Gln His Gln Met Trp Ala Ala Gln Phe Tyr Lys Tyr Asn Lys Pro
   385                 390                 395                 400
   Arg Gln Trp Leu Thr Ser Gly Gly Leu Gly Ala Met Gly Phe Gly Leu
                   405                 410                 415
   Pro Ala Ala Ile Gly Ala Ala Val Ala Arg Pro Asp Ala Val Val Val
               420                 425                 430
   Asp Ile Asp Gly Asp Gly Ser Phe Met Met Asn Val Gln Glu Leu Ala
           435                 440                 445
   Thr Ile Arg Val Glu Asn Leu Pro Val Lys Ile Leu Leu Leu Asn Asn
       450                 455                 460
   Gln His Leu Gly Met Val Val Gln Trp Glu Asp Arg Phe Tyr Lys Ala
   465                 470                 475                 480
   Asn Arg Ala His Thr Tyr Leu Gly Asn Pro Ser Lys Glu Ser Glu Ile
                   485                 490                 495
   Phe Pro Asn Met Leu Lys Phe Ala Glu Ala Cys Asp Ile Pro Ala Ala
               500                 505                 510
   Arg Val Thr Gln Lys Ala Asp Leu Arg Ala Ala Ile Gln Lys Met Leu
           515                 520                 525
   Asp Thr Pro Gly Pro Tyr Leu Leu Asp Val Ile Val Pro His Gln Glu
       530                 535                 540
   His Val Leu Pro Met Ile Pro Ala Gly Gly Gly Phe Ser Asp Val Ile
   545                 550                 555                 560
   Thr
<210> 3
   <211> 1965
   <212> DNA
   <213> Helianthus annus
<220>
   <221> CDS
   <222> (1)...(1965)
<221> misc_feature
   <222> (0) ... (0)
   <223> RHA266
<400> 3
   atg gcg gct cct ccc aac cct tcc atc tcc ttc aaa cca ccg tca ccc    48
   Met Ala Ala Pro Pro Asn Pro Ser Ile Ser Phe Lys Pro Pro Ser Pro
    1               5                  10                  15
   gcc gcc gca ctg cca cca cgc tcc gcc ttc ctc ccc cgt ttc gca tta    96
   Ala Ala Ala Leu Pro Pro Arg Ser Ala Phe Leu Pro Arg Phe Ala Leu
               20                  25                  30
   ccc atc act tcc act acc caa aaa cga cac cgt ctt cac atc tcc aat    144
   Pro Ile Thr Ser Thr Thr Gln Lys Arg His Arg Leu His Ile Ser Asn
           35                  40                  45
   gtt ctc tcc gac tcc aaa tcc acc acc acc acc acc acc act caa cca    192
   Val Leu Ser Asp Ser Lys Ser Thr Thr Thr Thr Thr Thr Thr Gln Pro
       50                  55                  60
   ccg tta cag gcg cag cct ttt gtc tcc cgg tac gcg cca gat caa ccg    240
   Pro Leu Gln Ala Gln Pro Phe Val Ser Arg Tyr Ala Pro Asp Gln Pro
   65                  70                  75                  80
   aga aaa ggc gca gac gtg ttg gtg gaa gct ctg gaa cgg gaa ggt gtc    288
   Arg Lys Gly Ala Asp Val Leu Val Glu Ala Leu Glu Arg Glu Gly Val
                   85                  90                  95
   acc gac gtc ttc gcc tac ccc ggc ggc gcg tca atg gag atc cac caa    336
   Thr Asp Val Phe Ala Tyr Pro Gly Gly Ala Ser Met Glu Ile His Gln
               100                 105                 110
   gct ctc acg cgc tca aac act atc cgc aat gtc ctc cca cgt cac gaa    384
   Ala Leu Thr Arg Ser Asn Thr Ile Arg Asn Val Leu Pro Arg His Glu
           115                 120                 125
   cag ggc ggc gtg ttc gcc gcc gaa ggc tac gcg cgc gcc tcc ggt ctt     432
   Gln Gly Gly Val Phe Ala Ala Glu Gly Tyr Ala Arg Ala Ser Gly Leu
       130                 135                 140
   ccc ggc gtg tgt atc gcc act tcc ggt ccc gga gct acg aac cta gtt     480
   Pro Gly Val Cys Ile Ala Thr Ser Gly Pro Gly Ala Thr Asn Leu Val
   145                 150                 155                 160
   agt ggt ctt gct gac gcg ctg tta gac agt gtc ccc atg gtg gca atc     528
   Ser Gly Leu Ala Asp Ala Leu Leu Asp Ser Val Pro Met Val Ala Ile
                   165                 170                 175
   acc ggt caa gtt ccc cgg aga atg atc gga acc gat gcg ttt caa gaa     576
   Thr Gly Gln Val Pro Arg Arg Met Ile Gly Thr Asp Ala Phe Gln Glu
               180                 185                 190
   acc cca att gtt gag gta aca cgt tcg atc act aaa cat aat tat ctt     624
   Thr Pro Ile Val Glu Val Thr Arg Ser Ile Thr Lys His Asn Tyr Leu
           195                 200                 205
   gtg ttg gat gtt gag gat att ccc aga att gtt cgt gag gct ttt tat     672
   Val Leu Asp Val Glu Asp Ile Pro Arg Ile Val Arg Glu Ala Phe Tyr
       210                 215                 220
   ctt gcg agt tcg ggt cga ccc ggc ccg gtt ttg ata gat gta ccg aaa     720
   Leu Ala Ser Ser Gly Arg Pro Gly Pro Val Leu Ile Asp Val Pro Lys
   225                 230                 235                 240
   gat ata cag caa cag tta gtg gtg ccg aaa tgg gat gaa ccg atg agg     768
   Asp Ile Gln Gln Gln Leu Val Val Pro Lys Trp Asp Glu Pro Met Arg
                   245                 250                 255
   tta ccg ggt tat ttg tct aga atg ccg aag cct caa tat gat ggg cat     816
   Leu Pro Gly Tyr Leu Ser Arg Met Pro Lys Pro Gln Tyr Asp Gly His
               260                 265                 270
   ttg gaa cag att gtt agg ttg gtg ggg gaa gcg aag agg ccg gtt ttg     864
   Leu Glu Gln Ile Val Arg Leu Val Gly Glu Ala Lys Arg Pro Val Leu
           275                 280                 285
   tat gtg ggt ggt ggg tgt ttg aat tcg gat gat gag ttg agg cgg ttt     912
   Tyr Val Gly Gly Gly Cys Leu Asn Ser Asp Asp Glu Leu Arg Arg Phe
       290                 295                 300
   gtg gag ctt acg ggg att ccg gtt gcg agt act ttg atg ggg ctc gga     960
   Val Glu Leu Thr Gly Ile Pro Val Ala Ser Thr Leu Met Gly Leu Gly
   305                 310                 315                 320
   gcg tac ccg gct tcg agt gat ttg tcg ctt cat atg ctt ggg atg cat     1008
   Ala Tyr Pro Ala Ser Ser Asp Leu Ser Leu His Met Leu Gly Met His
                   325                 330                 335
   ggt acg gtt tat gcg aat tat gcg gtt gat aag agt gat ttg ttg ctt     1056
   Gly Thr Val Tyr Ala Asn Tyr Ala Val Asp Lys Ser Asp Leu Leu Leu
               340                 345                 350
   gcg ttt ggg gtg cgg ttt gat gac cgt gtg acg ggg aag ctt gag gcg     1104
   Ala Phe Gly Val Arg Phe Asp Asp Arg Val Thr Gly Lys Leu Glu Ala
           355                 360                 365
   ttt gct agt agg gcg aag att gtt cat att gat att gat ccg gct gaa    1152
   Phe Ala Ser Arg Ala Lys Ile Val His Ile Asp Ile Asp Pro Ala Glu
       370                 375                 380
   att ggg aag aat aaa cag cct cat gtg tcg att tgt ggc gat att aag    1200
   Ile Gly Lys Asn Lys Gln Pro His Val Ser Ile Cys Gly Asp Ile Lys
   385                 390                 395                 400
   gtc gcg tta cag ggt ttg aac aag att ttg gag gaa aag aat tcg gtg    1248
   Val Ala Leu Gln Gly Leu Asn Lys Ile Leu Glu Glu Lys Asn Ser Val
                   405                 410                 415
   act aat ctt gat ttt tcg aac tgg aga aag gaa ttg gat gaa caa aaa    1296
   Thr Asn Leu Asp Phe Ser Asn Trp Arg Lys Glu Leu Asp Glu Gln Lys
               420                 425                 430
   gtg aag ttc ccg ttg agc ttt aaa acg ttt ggc gaa gcg att cct cca    1344
   Val Lys Phe Pro Leu Ser Phe Lys Thr Phe Gly Glu Ala Ile Pro Pro
           435                 440                 445
   cag tat gct att caa gtt ctt gat gag tta acg ggc ggg aat gca att    1392
   Gln Tyr Ala Ile Gln Val Leu Asp Glu Leu Thr Gly Gly Asn Ala Ile
       450                 455                 460
   att agc acc ggt gtc ggg caa cat cag atg tgg gct gct cag ttt tac    1440
   Ile Ser Thr Gly Val Gly Gln His Gln Met Trp Ala Ala Gln Phe Tyr
   465                 470                 475                 480
   aaa tac aac aaa cct aga caa tgg ctg acg tcg ggc ggg cta ggg gca    1488
   Lys Tyr Asn Lys Pro Arg Gln Trp Leu Thr Ser Gly Gly Leu Gly Ala
                   485                 490                 495
   atg ggt ttt ggg ctg ccc gct gct atc ggg gcg gcc gtt gca aga cct    1536
   Met Gly Phe Gly Leu Pro Ala Ala Ile Gly Ala Ala Val Ala Arg Pro
               500                 505                 510
   gat gcg gta gta gtt gac atc gac ggt gac gga agc ttt atg atg aat    1584
   Asp Ala Val Val Val Asp Ile Asp Gly Asp Gly Ser Phe Met Met Asn
           515                 520                 525
   gtt caa gag tta gcc aca atc cgt gtt gaa aat ctg ccg gtt aag att    1632
   Val Gln Glu Leu Ala Thr Ile Arg Val Glu Asn Leu Pro Val Lys Ile
       530                 535                 540
   tta tta ctc aac aat cag cat ttg ggt atg gtg gtt cag tgg gag gat    1680
   Leu Leu Leu Asn Asn Gln His Leu Gly Met Val Val Gln Trp Glu Asp
   545                 550                 555                 560
   cgg ttt tac aag gcg aat cgg gct cat acc tac tta gga aac ccg tca    1728
   Arg Phe Tyr Lys Ala Asn Arg Ala His Thr Tyr Leu Gly Asn Pro Ser
                   565                 570                 575
   aaa gag tcg gaa ata ttc cct aac atg ttg aag ttt gcc gaa gcc tgt    1776
   Lys Glu Ser Glu Ile Phe Pro Asn Met Leu Lys Phe Ala Glu Ala Cys
               580                 585                 590
   gat atc ccg gct gct cga gtg acc caa aag gcg gat cta cga gca gct    1824
   Asp Ile Pro Ala Ala Arg Val Thr Gln Lys Ala Asp Leu Arg Ala Ala
           595                 600                 605
   att cag aag atg ttg gat aca ccc ggg cct tac ttg ttg gat gtg att    1872
   Ile Gln Lys Met Leu Asp Thr Pro Gly Pro Tyr Leu Leu Asp Val Ile
       610                 615                 620
   gtg ccg cat caa gaa cac gtg ttg ccc atg atc ccg gct ggc gga ggt      1920
   Val Pro His Gln Glu His Val Leu Pro Met Ile Pro Ala Gly Gly Gly
   625                 630                 635                 640
   ttc tcg gat gtg atc acc gag ggt gat ggc aga acg aaa tat tga          1965
   Phe Ser Asp Val Ile Thr Glu Gly Asp Gly Arg Thr Lys Tyr  *
                   645                 650
<210> 4
   <211> 654
   <212> PRT
   <213> Helianthus annus
<400> 4
   Met Ala Ala Pro Pro Asn Pro Ser Ile Ser Phe Lys Pro Pro Ser Pro
    1               5                  10                  15
   Ala Ala Ala Leu Pro Pro Arg Ser Ala Phe Leu Pro Arg Phe Ala Leu
               20                  25                  30
   Pro Ile Thr Ser Thr Thr Gln Lys Arg His Arg Leu His Ile Ser Asn
           35                  40                  45
   Val Leu Ser Asp Ser Lys Ser Thr Thr Thr Thr Thr Thr Thr Gln Pro
       50                  55                  60
   Pro Leu Gln Ala Gln Pro Phe Val Ser Arg Tyr Ala Pro Asp Gln Pro
   65                  70                  75                  80
   Arg Lys Gly Ala Asp Val Leu Val Glu Ala Leu Glu Arg Glu Gly Val
                   85                  90                  95
   Thr Asp Val Phe Ala Tyr Pro Gly Gly Ala Ser Met Glu Ile His Gln
               100                 105                 110
   Ala Leu Thr Arg Ser Asn Thr Ile Arg Asn Val Leu Pro Arg His Glu
           115                 120                 125
   Gln Gly Gly Val Phe Ala Ala Glu Gly Tyr Ala Arg Ala Ser Gly Leu
       130                 135                 140
   Pro Gly Val Cys Ile Ala Thr Ser Gly Pro Gly Ala Thr Asn Leu Val
   145                 150                 155                 160
   Ser Gly Leu Ala Asp Ala Leu Leu Asp Ser Val Pro Met Val Ala Ile
                   165                 170                 175
   Thr Gly Gln Val Pro Arg Arg Met Ile Gly Thr Asp Ala Phe Gln Glu
               180                 185                 190
   Thr Pro Ile Val Glu Val Thr Arg Ser Ile Thr Lys His Asn Tyr Leu
           195                 200                 205
   Val Leu Asp Val Glu Asp Ile Pro Arg Ile Val Arg Glu Ala Phe Tyr
       210                 215                 220
   Leu Ala Ser Ser Gly Arg Pro Gly Pro Val Leu Ile Asp Val Pro Lys
   225                 230                 235                 240
   Asp Ile Gln Gln Gln Leu Val Val Pro Lys Trp Asp Glu Pro Met Arg
                   245                 250                 255
   Leu Pro Gly Tyr Leu Ser Arg Met Pro Lys Pro Gln Tyr Asp Gly His
               260                 265                 270
   Leu Glu Gln Ile Val Arg Leu Val Gly Glu Ala Lys Arg Pro Val Leu
           275                 280                 285
   Tyr Val Gly Gly Gly Cys Leu Asn Ser Asp Asp Glu Leu Arg Arg Phe
       290                 295                 300
   Val Glu Leu Thr Gly Ile Pro Val Ala Ser Thr Leu Met Gly Leu Gly
   305                 310                 315                 320
   Ala Tyr Pro Ala Ser Ser Asp Leu Ser Leu His Met Leu Gly Met His
                   325                 330                 335
   Gly Thr Val Tyr Ala Asn Tyr Ala Val Asp Lys Ser Asp Leu Leu Leu
               340                 345                 350
   Ala Phe Gly Val Arg Phe Asp Asp Arg Val Thr Gly Lys Leu Glu Ala
           355                 360                 365
   Phe Ala Ser Arg Ala Lys Ile Val His Ile Asp Ile Asp Pro Ala Glu
       370                 375                 380
   Ile Gly Lys Asn Lys Gln Pro His Val Ser Ile Cys Gly Asp Ile Lys
   385                 390                 395                 400
   Val Ala Leu Gln Gly Leu Asn Lys Ile Leu Glu Glu Lys Asn Ser Val
                   405                 410                 415
   Thr Asn Leu Asp Phe Ser Asn Trp Arg Lys Glu Leu Asp Glu Gln Lys
               420                 425                 430
   Val Lys Phe Pro Leu Ser Phe Lys Thr Phe Gly Glu Ala Ile Pro Pro
           435                 440                 445
   Gln Tyr Ala Ile Gln Val Leu Asp Glu Leu Thr Gly Gly Asn Ala Ile
       450                 455                 460
   Ile Ser Thr Gly Val Gly Gln His Gln Met Trp Ala Ala Gln Phe Tyr
   465                 470                 475                 480
   Lys Tyr Asn Lys Pro Arg Gln Trp Leu Thr Ser Gly Gly Leu Gly Ala
                   485                 490                 495
   Met Gly Phe Gly Leu Pro Ala Ala Ile Gly Ala Ala Val Ala Arg Pro
               500                 505                 510
   Asp Ala Val Val Val Asp Ile Asp Gly Asp Gly Ser Phe Met Met Asn
           515                 520                 525
   Val Gln Glu Leu Ala Thr Ile Arg Val Glu Asn Leu Pro Val Lys Ile
       530                 535                 540
   Leu Leu Leu Asn Asn Gln His Leu Gly Met Val Val Gln Trp Glu Asp
   545                 550                 555                 560
   Arg Phe Tyr Lys Ala Asn Arg Ala His Thr Tyr Leu Gly Asn Pro Ser
                   565                 570                 575
   Lys Glu Ser Glu Ile Phe Pro Asn Met Leu Lys Phe Ala Glu Ala Cys
               580                 585                 590
   Asp Ile Pro Ala Ala Arg Val Thr Gln Lys Ala Asp Leu Arg Ala Ala
           595                 600                 605
   Ile Gln Lys Met Leu Asp Thr Pro Gly Pro Tyr Leu Leu Asp Val Ile
       610                 615                 620
   Val Pro His Gln Glu His Val Leu Pro Met Ile Pro Ala Gly Gly Gly
   625                 630                 635                 640
   Phe Ser Asp Val Ile Thr Glu Gly Asp Gly Arg Thr Lys Tyr
                   645                 650
<210> 5
   <211> 2156
   <212> DNA
   <213> Xanthium sp.
<220>
   <221> CDS
   <222> (111)...(2057)
<221> misc_feature
   <222> (0) ... (0)
   <223> GenBank Accession No. U16279
<400> 5
   gaacaacagc cacatgtttc tggaccatcg tcgttcacac ctattttaat cagataaaca 60
   aagtacaaac ataacataac ataaccctag tacataacac acattcaaca atg gcg    116
                                                         Met Ala
                                                          1
   gcc atc cct cat aca aac cct tcc atc acc acc aaa cca ccc tca tct    164
   Ala Ile Pro His Thr Asn Pro Ser Ile Thr Thr Lys Pro Pro Ser Ser
           5                  10                  15
   cca cca cgt ccc acc ttc ctc gcc cgt ttc aca ttc cca ata acc tcc    212
   Pro Pro Arg Pro Thr Phe Leu Ala Arg Phe Thr Phe Pro Ile Thr Ser
       20                  25                  30
   act tcc cat aaa cga cac cgt ctc cac atc tcc aac gtc ctc tcc gac    260
   Thr Ser His Lys Arg His Arg Leu His Ile Ser Asn Val Leu Ser Asp
   35                  40                  45                  50
   tcc aaa ccc acc atc acc cat tca cca tta cca acc gaa tca ttt atc    308
   Ser Lys Pro Thr Ile Thr His Ser Pro Leu Pro Thr Glu Ser Phe Ile
                   55                  60                  65
   tcc cgt tac gct cca gac caa cca aga aaa ggc gct gat gtt ctc gtc    356
   Ser Arg Tyr Ala Pro Asp Gln Pro Arg Lys Gly Ala Asp Val Leu Val
               70                  75                  80
   gaa gct ctg gaa cgt gaa ggc gtt aca gac gtc ttc gct tac cca ggt    404
   Glu Ala Leu Glu Arg Glu Gly Val Thr Asp Val Phe Ala Tyr Pro Gly
           85                  90                  95
   ggt gcc tcc atg gag atc cac caa gct ctc acg cgc tca acc acc atc    452
   Gly Ala Ser Met Glu Ile His Gln Ala Leu Thr Arg Ser Thr Thr Ile
       100                 105                 110
   cgc aac gtt ctc cca cgt cac gaa cag ggc ggc gtc ttt gct gcc gaa    500
   Arg Asn Val Leu Pro Arg His Glu Gln Gly Gly Val Phe Ala Ala Glu
   115                 120                 125                 130
   ggc tac gca cgt gcc tcc ggt ctt ccc ggc gtc tgt att gca acc tct    548
   Gly Tyr Ala Arg Ala Ser Gly Leu Pro Gly Val Cys Ile Ala Thr Ser
                   135                 140                 145
   ggt cct gga gct acg aac cta gta agt ggt ctt gct gat gct tta tta    596
   Gly Pro Gly Ala Thr Asn Leu Val Ser Gly Leu Ala Asp Ala Leu Leu
               150                 155                 160
   gac agt gtt cca atg gtt gct att act ggt caa gtt ccc agg aga atg    644
   Asp Ser Val Pro Met Val Ala Ile Thr Gly Gln Val Pro Arg Arg Met
           165                 170                 175
   att gga aca gat gcg ttt caa gaa acc cct att gtt gag gta aca cgt    692
   Ile Gly Thr Asp Ala Phe Gln Glu Thr Pro Ile Val Glu Val Thr Arg
       180                 185                 190
   tcc att act aag cat aat tat tta gtt ttg gat gtc gag gat att ccc    740
   Ser Ile Thr Lys His Asn Tyr Leu Val Leu Asp Val Glu Asp Ile Pro
   195                 200                 205                 210
   agg att gtt agg gaa gct ttt tat ctt gcg tct tct ggt cga ccc gga    788
   Arg Ile Val Arg Glu Ala Phe Tyr Leu Ala Ser Ser Gly Arg Pro Gly
                   215                 220                 225
   ccg gtt tta att gat gta cct aag gat ata cag cag cag ttg gta gtg    836
   Pro Val Leu Ile Asp Val Pro Lys Asp Ile Gln Gln Gln Leu Val Val
               230                 235                 240
   cct aaa tgg gat gag cct att agg tta cct ggg tat ttg tct agg ttg    884
   Pro Lys Trp Asp Glu Pro Ile Arg Leu Pro Gly Tyr Leu Ser Arg Leu
           245                 250                 255
   cct aaa acg gag aat aat ggg cag ttg gaa cac att gtt agg ttg gtg    932
   Pro Lys Thr Glu Asn Asn Gly Gln Leu Glu His Ile Val Arg Leu Val
       260                 265                 270
   agt gag gcc aag agg ccg gtt ttg tat gtg ggg ggt ggg tgt ttg aat    980
   Ser Glu Ala Lys Arg Pro Val Leu Tyr Val Gly Gly Gly Cys Leu Asn
   275                 280                 285                 290
   tcg gga gat gag ttg agg cgg ttt gtg gag ctt acg ggg ata ccg gtt   1028
   Ser Gly Asp Glu Leu Arg Arg Phe Val Glu Leu Thr Gly Ile Pro Val
                   295                 300                 305
   gcg agt acg ttg atg ggg ctt gga gcg tac cct gct tct agt gat ttg   1076
   Ala Ser Thr Leu Met Gly Leu Gly Ala Tyr Pro Ala Ser Ser Asp Leu
               310                 315                 320
   tcg ctg cat atg ctt ggg atg cat ggg acg gtt tat gcg aat tat gcg   1124
   Ser Leu His Met Leu Gly Met His Gly Thr Val Tyr Ala Asn Tyr Ala
           325                 330                 335
   gtt gat aag agt gat ttg ttg ctt gcg ttt ggg gta agg ttt gat gac   1172
   Val Asp Lys Ser Asp Leu Leu Leu Ala Phe Gly Val Arg Phe Asp Asp
       340                 345                 350
   cgt gtg acg ggg aag ctt gag gct ttt gct agc aga gct aag att gtt   1220
   Arg Val Thr Gly Lys Leu Glu Ala Phe Ala Ser Arg Ala Lys Ile Val
   355                 360                 365                 370
   cat att gat att gat tct gcg gaa att ggg aag aat aag cag cct cat   1268
   His Ile Asp Ile Asp Ser Ala Glu Ile Gly Lys Asn Lys Gln Pro His
                   375                 380                 385
   gtg tcg att tgt ggt gat atc aag gtc gcg tta cag ggt ctg aac aag   1316
   Val Ser Ile Cys Gly Asp Ile Lys Val Ala Leu Gln Gly Leu Asn Lys
               390                 395                 400
   att ttg gag gta aag aat tcg gtg act aat ctt gat ttc tcg aac tgg   1364
   Ile Leu Glu Val Lys Asn Ser Val Thr Asn Leu Asp Phe Ser Asn Trp
           405                 410                 415
   agg aag gaa ttg gat gag caa aag gtt aag tat ccg ttg agt ttt aaa   1412
   Arg Lys Glu Leu Asp Glu Gln Lys Val Lys Tyr Pro Leu Ser Phe Lys
       420                 425                 430
   aca ttt ggc gaa gct att cct ccg cag tat gcc att caa gtg ctt gat   1460
   Thr Phe Gly Glu Ala Ile Pro Pro Gln Tyr Ala Ile Gln Val Leu Asp
   435                 440                 445                 450
   gag tta acg ggt ggg aat gcg att att agc act ggg gtc ggg cag cat   1508
   Glu Leu Thr Gly Gly Asn Ala Ile Ile Ser Thr Gly Val Gly Gln His
                   455                 460                 465
   cag atg tgg gct gct cag ttt tac aaa tac aac aag cct aga caa tgg   1556
   Gln Met Trp Ala Ala Gln Phe Tyr Lys Tyr Asn Lys Pro Arg Gln Trp
               470                 475                 480
   ctg acg tca ggt gga cta ggc gcg atg ggt ttt ggg ttg ccc gct gct   1604
   Leu Thr Ser Gly Gly Leu Gly Ala Met Gly Phe Gly Leu Pro Ala Ala
           485                 490                 495
   atc ggg gcg gct gtt gca aga cct gat gcg gta gta gtt gat atc gat      1652
   Ile Gly Ala Ala Val Ala Arg Pro Asp Ala Val Val Val Asp Ile Asp
       500                 505                 510
   ggt gat gga agc ttt ata atg agc gtt caa gag tta gcc aca atc cgt      1700
   Gly Asp Gly Ser Phe Ile Met Ser Val Gln Glu Leu Ala Thr Ile Arg
   515                 520                 525                 530
   gtt gaa aat ctt cct gtt aag att ttg tta ctt aac aat cag cat ttg      1748
   Val Glu Asn Leu Pro Val Lys Ile Leu Leu Leu Asn Asn Gln His Leu
                   535                 540                 545
   ggt atg gtg gtt cag ttg gag gat cgg ttt tac aag gcg aat cgg gct      1796
   Gly Met Val Val Gln Leu Glu Asp Arg Phe Tyr Lys Ala Asn Arg Ala
               550                 555                 560
   cat acc tac tta gga aat ccg tca aaa gag tct gaa ata ttc cct aac      1844
   His Thr Tyr Leu Gly Asn Pro Ser Lys Glu Ser Glu Ile Phe Pro Asn
           565                 570                 575
   atg ttg aag ttt gct gaa gcg tgt gat atc cca gct gcc cga gtg acc      1892
   Met Leu Lys Phe Ala Glu Ala Cys Asp Ile Pro Ala Ala Arg Val Thr
       580                 585                 590
   cgg aag gca gat cta cga gca gct att cag aag atg ttg gat aca ccg      1940
   Arg Lys Ala Asp Leu Arg Ala Ala Ile Gln Lys Met Leu Asp Thr Pro
   595                 600                 605                 610
   ggg cct tac ttg ttg gat gtg atc gtg ccc cat caa gaa cat gtg ttg      1988
   Gly Pro Tyr Leu Leu Asp Val Ile Val Pro His Gln Glu His Val Leu
                   615                 620                 625
   ccc atg atc ccg gct ggt gga ggt ttc atg gat gtg atc acc gaa ggc      2036
   Pro Met Ile Pro Ala Gly Gly Gly Phe Met Asp Val Ile Thr Glu Gly
               630                 635                 640
   gac ggc aga atg aaa tat tga gcttcaatgt cacatatagt gtgttctgta        2087
   Asp Gly Arg Met Lys Tyr  *
           645
   agcagtttgt cggttatgaa gttaaatgtt ttgttgtgta atttcgttcc tggttaaaaa 2147
   atcaagctt                                                         2156
<210> 6
   <211> 648
   <212> PRT
   <213> Helianthus annus
<400> 6
   Met Ala Ala Ile Pro His Thr Asn Pro Ser Ile Thr Thr Lys Pro Pro
    1               5                   10                  15
   Ser Ser Pro Pro Arg Pro Thr Phe Leu Ala Arg Phe Thr Phe Pro Ile
               20                  25                  30
   Thr Ser Thr Ser His Lys Arg His Arg Leu His Ile Ser Asn Val Leu
           35                  40                  45
   Ser Asp Ser Lys Pro Thr Ile Thr His Ser Pro Leu Pro Thr Glu Ser
       50                  55                  60
   Phe Ile Ser Arg Tyr Ala Pro Asp Gln Pro Arg Lys Gly Ala Asp Val
   65                  70                  75                  80
   Leu Val Glu Ala Leu Glu Arg Glu Gly Val Thr Asp Val Phe Ala Tyr
                   85                  90                  95
   Pro Gly Gly Ala Ser Met Glu Ile His Gln Ala Leu Thr Arg Ser Thr
               100                 105                 110
   Thr Ile Arg Asn Val Leu Pro Arg His Glu Gln Gly Gly Val Phe Ala
           115                 120                 125
   Ala Glu Gly Tyr Ala Arg Ala Ser Gly Leu Pro Gly Val Cys Ile Ala
       130                 135                 140
   Thr Ser Gly Pro Gly Ala Thr Asn Leu Val Ser Gly Leu Ala Asp Ala
   145                 150                 155                 160
   Leu Leu Asp Ser Val Pro Met Val Ala Ile Thr Gly Gln Val Pro Arg
                   165                 170                 175
   Arg Met Ile Gly Thr Asp Ala Phe Gln Glu Thr Pro Ile Val Glu Val
               180                 185                 190
   Thr Arg Ser Ile Thr Lys His Asn Tyr Leu Val Leu Asp Val Glu Asp
           195                 200                 205
   Ile Pro Arg Ile Val Arg Glu Ala Phe Tyr Leu Ala Ser Ser Gly Arg
       210                 215                 220
   Pro Gly Pro Val Leu Ile Asp Val Pro Lys Asp Ile Gln Gln Gln Leu
   225                 230                 235                 240
   Val Val Pro Lys Trp Asp Glu Pro Ile Arg Leu Pro Gly Tyr Leu Ser
                   245                 250                 255
   Arg Leu Pro Lys Thr Glu Asn Asn Gly Gln Leu Glu His Ile Val Arg
               260                 265                 270
   Leu Val Ser Glu Ala Lys Arg Pro Val Leu Tyr Val Gly Gly Gly Cys
           275                 280                 285
   Leu Asn Ser Gly Asp Glu Leu Arg Arg Phe Val Glu Leu Thr Gly Ile
       290                 295                 300
   Pro Val Ala Ser Thr Leu Met Gly Leu Gly Ala Tyr Pro Ala Ser Ser
   305                 310                 315                 320
   Asp Leu Ser Leu His Met Leu Gly Met His Gly Thr Val Tyr Ala Asn
                   325                 330                 335
   Tyr Ala Val Asp Lys Ser Asp Leu Leu Leu Ala Phe Gly Val Arg Phe
               340                 345                 350
   Asp Asp Arg Val Thr Gly Lys Leu Glu Ala Phe Ala Ser Arg Ala Lys
           355                 360                 365
   Ile Val His Ile Asp Ile Asp Ser Ala Glu Ile Gly Lys Asn Lys Gln
       370                 375                 380
   Pro His Val Ser Ile Cys Gly Asp Ile Lys Val Ala Leu Gln Gly Leu
   385                 390                 395                 400
   Asn Lys Ile Leu Glu Val Lys Asn Ser Val Thr Asn Leu Asp Phe Ser
                   405                 410                 415
   Asn Trp Arg Lys Glu Leu Asp Glu Gln Lys Val Lys Tyr Pro Leu Ser
               420                 425                 430
   Phe Lys Thr Phe Gly Glu Ala Ile Pro Pro Gln Tyr Ala Ile Gln Val
           435                 440                 445
   Leu Asp Glu Leu Thr Gly Gly Asn Ala Ile Ile Ser Thr Gly Val Gly
       450                 455                 460
   Gln His Gln Met Trp Ala Ala Gln Phe Tyr Lys Tyr Asn Lys Pro Arg
   465                 470                 475                 480
   Gln Trp Leu Thr Ser Gly Gly Leu Gly Ala Met Gly Phe Gly Leu Pro
                   485                 490                 495
   Ala Ala Ile Gly Ala Ala Val Ala Arg Pro Asp Ala Val Val Val Asp
               500                 505                 510
   Ile Asp Gly Asp Gly Ser Phe Ile Met Ser Val Gln Glu Leu Ala Thr
           515                 520                 525
   Ile Arg Val Glu Asn Leu Pro Val Lys Ile Leu Leu Leu Asn Asn Gln
       530                 535                 540
   His Leu Gly Met Val Val Gln Leu Glu Asp Arg Phe Tyr Lys Ala Asn
   545                 550                 555                 560
   Arg Ala His Thr Tyr Leu Gly Asn Pro Ser Lys Glu Ser Glu Ile Phe
                       565                     570                     575
   Pro Asn Met Leu Lys Phe Ala Glu Ala Cys Asp Ile Pro Ala Ala Arg
                   580                     585                     590
   Val Thr Arg Lys Ala Asp Leu Arg Ala Ala Ile Gln Lys Met Leu Asp
               595                     600                     605
   Thr Pro Gly Pro Tyr Leu Leu Asp Val Ile Val Pro His Gln Glu His
       610                     615                     620
   Val Leu Pro Met Ile Pro Ala Gly Gly Gly Phe Met Asp Val Ile Thr
   625                     630                     635                     640
   Glu Gly Asp Gly Arg Met Lys Tyr
                   645
<210> 7
   <211> 2156
   <212> DNA
   <213> Xanthium sp.
<220>
   <221> CDS
   <222> (111) ... (2057)
<221> misc_feature
   <222> (0) ... (0)
   <223> GenBank Accession No. U16280
<400> 7
   gaacaacagc cacatgtttc tggaccatcg tcgttcacac ctattttaat cagataaaca 60
   aagtacaaac ataacataac ataaccctag tacataacac acattcaaca atg gcg    116
                                                         Met Ala
                                                          1
   gcc atc cct cat aca aac cct tcc atc acc acc aaa cca ccc tca tct   164
   Ala Ile Pro His Thr Asn Pro Ser Ile Thr Thr Lys Pro Pro Ser Ser
           5                  10                  15
   cca cca cgt ccc acc ttc ctc gcc cgt ttc aca ttc cca ata acc tcc   212
   Pro Pro Arg Pro Thr Phe Leu Ala Arg Phe Thr Phe Pro Ile Thr Ser
       20                  25                  30
   act tcc cat aaa cga cac cgt ctc cac atc tcc aac gtc ctc tcc gac   260
   Thr Ser His Lys Arg His Arg Leu His Ile Ser Asn Val Leu Ser Asp
   35                  40                  45                  50
   tcc aaa ccc acc atc acc cat tca cca tta cca acc aaa tca ttt atc   308
   Ser Lys Pro Thr Ile Thr His Ser Pro Leu Pro Thr Lys Ser Phe Ile
                   55                  60                  65
   tcc cgt tac gct cca gac caa cca aga aaa ggc gct gat gtt ctc gtc   356
   Ser Arg Tyr Ala Pro Asp Gln Pro Arg Lys Gly Ala Asp Val Leu Val
               70                  75                  80
   gaa gct ctg gaa cgt gaa ggc gtt aca gac gtc ttc gct tac cca ggt   404
   Glu Ala Leu Glu Arg Glu Gly Val Thr Asp Val Phe Ala Tyr Pro Gly
           85                  90                  95
   ggt gcc tcc atg gag atc cac caa gct ctc acg cgc tca acc acc atc   452
   Gly Ala Ser Met Glu Ile His Gln Ala Leu Thr Arg Ser Thr Thr Ile
       100                 105                 110
   cgc aac gtt ctc cca cgt cac gaa cag ggc ggc gtc ttt gct gcc gaa   500
   Arg Asn Val Leu Pro Arg His Glu Gln Gly Gly Val Phe Ala Ala Glu
   115                 120                 125                 130
   ggc tac gca cgt gcc tcc ggt ctt ccc ggc gtc tgt att gca acc tct      548
   Gly Tyr Ala Arg Ala Ser Gly Leu Pro Gly Val Cys Ile Ala Thr Ser
                   135                 140                 145
   ggt cct gga gct acg aac cta gta agt ggt ctt gct gat gct tta tta      596
   Gly Pro Gly Ala Thr Asn Leu Val Ser Gly Leu Ala Asp Ala Leu Leu
               150                 155                 160
   gac agt gtt cca atg gtt gct att act ggt caa gtt ccc agg aga atg      644
   Asp Ser Val Pro Met Val Ala Ile Thr Gly Gln Val Pro Arg Arg Met
           165                 170                 175
   att gga aca gat gcg ttt caa gaa acc cct att gtt gag gta aca cgt      692
   Ile Gly Thr Asp Ala Phe Gln Glu Thr Pro Ile Val Glu Val Thr Arg
       180                 185                 190
   tcc att act aag cat aat tat tta gtt ttg gat gtc gag gat att ccc      740
   Ser Ile Thr Lys His Asn Tyr Leu Val Leu Asp Val Glu Asp Ile Pro
   195                 200                 205                 210
   agg att gtt agg gaa gct ttt tat ctt gcg tct tct ggt cga ccc gga      788
   Arg Ile Val Arg Glu Ala Phe Tyr Leu Ala Ser Ser Gly Arg Pro Gly
                   215                 220                 225
   ccg gtt tta att gat gta cct aag gat ata cag cag cag ttg gta gtg      836
   Pro Val Leu Ile Asp Val Pro Lys Asp Ile Gln Gln Gln Leu Val Val
               230                 235                 240
   cct aaa tgg gat gag cct att agg tta cct ggg tat ttg tct agg ttc      884
   Pro Lys Trp Asp Glu Pro Ile Arg Leu Pro Gly Tyr Leu Ser Arg Phe
           245                 250                 255
   cct aaa acg gag aat aat ggg cag ttg gaa cag att gtt agg ttg gtg      932
   Pro Lys Thr Glu Asn Asn Gly Gln Leu Glu Gln Ile Val Arg Leu Val
       260                 265                 270
   agt gag gcc aag agg ccg gtt ttg tat gtg ggg ggt ggg tgt ttg aat      980
   Ser Glu Ala Lys Arg Pro Val Leu Tyr Val Gly Gly Gly Cys Leu Asn
   275                 280                 285                 290
   tcg gga gat gag ttg agg cgg ttt gtg gag ctt acg ggg ata ccg gtt      1028
   Ser Gly Asp Glu Leu Arg Arg Phe Val Glu Leu Thr Gly Ile Pro Val
                   295                 300                 305
   gcg agt acg ttg atg ggg ctt gga gcg tac cct gct tct agt gat ttg      1076
   Ala Ser Thr Leu Met Gly Leu Gly Ala Tyr Pro Ala Ser Ser Asp Leu
               310                 315                 320
   tcg ctg cat atg ctt ggg atg cat ggg acc gtt tat gcg aat tat gcg      1124
   Ser Leu His Met Leu Gly Met His Gly Thr Val Tyr Ala Asn Tyr Ala
           325                 330                 335
   gtt gat aag agt gat ttg ttg ctt gcg ttt ggg gta agg ttt gat gac      1172
   Val Asp Lys Ser Asp Leu Leu Leu Ala Phe Gly Val Arg Phe Asp Asp
       340                 345                 350
   cgt gtg acg ggg aag ctt gag gct ttt gct agc aga gct aag att gtt      1220
   Arg Val Thr Gly Lys Leu Glu Ala Phe Ala Ser Arg Ala Lys Ile Val
   355                 360                 365                 370
   cat att gat att gat tct gcg gaa att ggg aag aat aag cag cct cat    1268
   His Ile Asp Ile Asp Ser Ala Glu Ile Gly Lys Asn Lys Gln Pro His
                   375                 380                 385
   gtg tcg att tgt ggt gat att aag gtc gcg tta cag ggt ctg aac aag    1316
   Val Ser Ile Cys Gly Asp Ile Lys Val Ala Leu Gln Gly Leu Asn Lys
               390                 395                 400
   att ttg gag gta aag aat tcg gtg act aat ctt gat ttc tcg aac tgg    1364
   Ile Leu Glu Val Lys Asn Ser Val Thr Asn Leu Asp Phe Ser Asn Trp
           405                 410                 415
   agg aag gaa ttg gat gag caa aag gtt aag tat ccg ttg agt ttt aaa    1412
   Arg Lys Glu Leu Asp Glu Gln Lys Val Lys Tyr Pro Leu Ser Phe Lys
       420                 425                 430
   aca ttt ggc gaa gct att cct ccg cag tat gcc att caa gtg ctt gat    1460
   Thr Phe Gly Glu Ala Ile Pro Pro Gln Tyr Ala Ile Gln Val Leu Asp
   435                 440                 445                 450
   gag tta acg ggt ggg aat gcg att att agc act ggg gtc ggg cag cat    1508
   Glu Leu Thr Gly Gly Asn Ala Ile Ile Ser Thr Gly Val Gly Gln His
                   455                 460                 465
   cag atg tgg gct gct cag ttt tac aaa tac aac aag cct aga caa tgg    1556
   Gln Met Trp Ala Ala Gln Phe Tyr Lys Tyr Asn Lys Pro Arg Gln Trp
               470                 475                 480
   ctg acg tca ggt gga cta ggg gcg atg ggt ttt ggg ttg ccc gct gct    1604
   Leu Thr Ser Gly Gly Leu Gly Ala Met Gly Phe Gly Leu Pro Ala Ala
           485                 490                 495
   atc ggg gcc gct gtt gca aga cct gat gcg gta gta gtt gat atc gat    1652
   Ile Gly Ala Ala Val Ala Arg Pro Asp Ala Val Val Val Asp Ile Asp
       500                 505                 510
   ggt gat gga agc ttt ata atg aac gtt caa gag tta gcc aca atc cgt    1700
   Gly Asp Gly Ser Phe Ile Met Asn Val Gln Glu Leu Ala Thr Ile Arg
   515                 520                 525                 530
   gtt gaa aat ctt cct gtt aag att ttg tta ctt aac aat cag cat ttg    1748
   Val Glu Asn Leu Pro Val Lys Ile Leu Leu Leu Asn Asn Gln His Leu
                   535                 540                 545
   ggt atg gtg gtt cag tgg gag gat cgg ttt tac aag gcg aat cgg gct    1796
   Gly Met Val Val Gln Trp Glu Asp Arg Phe Tyr Lys Ala Asn Arg Ala
               550                 555                 560
   cat acc tac tta gga aat ccg tca aaa gag tct gaa ata ttc cct aac    1844
   His Thr Tyr Leu Gly Asn Pro Ser Lys Glu Ser Glu Ile Phe Pro Asn
           565                 570                 575
   atg ttg aag ttt gct gaa gcg tgt gat atc cca gct gcc cga gtg acc    1892
   Met Leu Lys Phe Ala Glu Ala Cys Asp Ile Pro Ala Ala Arg Val Thr
       580                 585                 590
   cgg aag gca gat cta cga gca gct att cag aag atg ttg gat aca ccg    1940
   Arg Lys Ala Asp Leu Arg Ala Ala Ile Gln Lys Met Leu Asp Thr Pro
   595                 600                 605                 610
   ggg cct tac ttg ttg gat gtg atc gtg ccc cat caa gaa cat gtg ttg   1988
   Gly Pro Tyr Leu Leu Asp Val Ile Val Pro His Gln Glu His Val Leu
                   615                 620                 625
   ccc atg atc ccg gct ggt gga ggt ttc atg gat gtg atc acc gaa ggc   2036
   Pro Met Ile Pro Ala Gly Gly Gly Phe Met Asp Val Ile Thr Glu Gly
               630                 635                 640
   gac ggc aga atg aaa tat tga gcttcaatgt cacatatagt gtgttctgta      2087
   Asp Gly Arg Met Lys Tyr  *
           645
   agcagtttgt cggttatgaa gttaaatgtt ttgttgtgta atttcgttcc tggttaaaaa 2147
   atcaagctt                                                         2156
<210> 8
   <211> 648
   <212> PRT
   <213> Xanthium sp.
<400> 8
   Met Ala Ala Ile Pro His Thr Asn Pro Ser Ile Thr Thr Lys Pro Pro
    1               5                  10                  15
   Ser Ser Pro Pro Arg Pro Thr Phe Leu Ala Arg Phe Thr Phe Pro Ile
               20                  25                  30
   Thr Ser Thr Ser His Lys Arg His Arg Leu His Ile Ser Asn Val Leu
           35                  40                  45
   Ser Asp Ser Lys Pro Thr Ile Thr His Ser Pro Leu Pro Thr Lys Ser
       50                  55                  60
   Phe Ile Ser Arg Tyr Ala Pro Asp Gln Pro Arg Lys Gly Ala Asp Val
   65                  70                  75                  80
   Leu Val Glu Ala Leu Glu Arg Glu Gly Val Thr Asp Val Phe Ala Tyr
                   85                  90                  95
   Pro Gly Gly Ala Ser Met Glu Ile His Gln Ala Leu Thr Arg Ser Thr
               100                 105                 110
   Thr Ile Arg Asn Val Leu Pro Arg His Glu Gln Gly Gly Val Phe Ala
           115                 120                 125
   Ala Glu Gly Tyr Ala Arg Ala Ser Gly Leu Pro Gly Val Cys Ile Ala
       130                 135                 140
   Thr Ser Gly Pro Gly Ala Thr Asn Leu Val Ser Gly Leu Ala Asp Ala
   145                 150                 155                 160
   Leu Leu Asp Ser Val Pro Met Val Ala Ile Thr Gly Gln Val Pro Arg
                   165                 170                 175
   Arg Met Ile Gly Thr Asp Ala Phe Gln Glu Thr Pro Ile Val Glu Val
               180                 185                 190
   Thr Arg Ser Ile Thr Lys His Asn Tyr Leu Val Leu Asp Val Glu Asp
           195                 200                 205
   Ile Pro Arg Ile Val Arg Glu Ala Phe Tyr Leu Ala Ser Ser Gly Arg
       210                 215                 220
   Pro Gly Pro Val Leu Ile Asp Val Pro Lys Asp Ile Gln Gln Gln Leu
   225                 230                 235                 240
   Val Val Pro Lys Trp Asp Glu Pro Ile Arg Leu Pro Gly Tyr Leu Ser
                   245                 250                 255
   Arg Phe Pro Lys Thr Glu Asn Asn Gly Gln Leu Glu Gln Ile Val Arg
               260                 265                 270
   Leu Val Ser Glu Ala Lys Arg Pro Val Leu Tyr Val Gly Gly Gly Cys
           275                 280                 285
   Leu Asn Ser Gly Asp Glu Leu Arg Arg Phe Val Glu Leu Thr Gly Ile
       290                 295                 300
   Pro Val Ala Ser Thr Leu Met Gly Leu Gly Ala Tyr Pro Ala Ser Ser
   305                 310                 315                 320
   Asp Leu Ser Leu His Met Leu Gly Met His Gly Thr Val Tyr Ala Asn
                   325                 330                 335
   Tyr Ala Val Asp Lys Ser Asp Leu Leu Leu Ala Phe Gly Val Arg Phe
               340                 345                 350
   Asp Asp Arg Val Thr Gly Lys Leu Glu Ala Phe Ala Ser Arg Ala Lys
           355                 360                 365
   Ile Val His Ile Asp Ile Asp Ser Ala Glu Ile Gly Lys Asn Lys Gln
       370                 375                 380
   Pro His Val Ser Ile Cys Gly Asp Ile Lys Val Ala Leu Gln Gly Leu
   385                 390                 395                 400
   Asn Lys Ile Leu Glu Val Lys Asn Ser Val Thr Asn Leu Asp Phe Ser
                   405                 410                 415
   Asn Trp Arg Lys Glu Leu Asp Glu Gln Lys Val Lys Tyr Pro Leu Ser
               420                 425                 430
   Phe Lys Thr Phe Gly Glu Ala Ile Pro Pro Gln Tyr Ala Ile Gln Val
           435                 440                 445
   Leu Asp Glu Leu Thr Gly Gly Asn Ala Ile Ile Ser Thr Gly Val Gly
       450                 455                 460
   Gln His Gln Met Trp Ala Ala Gln Phe Tyr Lys Tyr Asn Lys Pro Arg
   465                 470                 475                 480
   Gln Trp Leu Thr Ser Gly Gly Leu Gly Ala Met Gly Phe Gly Leu Pro
                   485                 490                 495
   Ala Ala Ile Gly Ala Ala Val Ala Arg Pro Asp Ala Val Val Val Asp
               500                 505                 510
   Ile Asp Gly Asp Gly Ser Phe Ile Met Asn Val Gln Glu Leu Ala Thr
           515                 520                 525
   Ile Arg Val Glu Asn Leu Pro Val Lys Ile Leu Leu Leu Asn Asn Gln
       530                 535                 540
   His Leu Gly Met Val Val Gln Trp Glu Asp Arg Phe Tyr Lys Ala Asn
   545                 550                 555                 560
   Arg Ala His Thr Tyr Leu Gly Asn Pro Ser Lys Glu Ser Glu Ile Phe
                   565                 570                 575
   Pro Asn Met Leu Lys Phe Ala Glu Ala Cys Asp Ile Pro Ala Ala Arg
               580                 585                 590
   Val Thr Arg Lys Ala Asp Leu Arg Ala Ala Ile Gln Lys Met Leu Asp
           595                 600                 605
   Thr Pro Gly Pro Tyr Leu Leu Asp Val Ile Val Pro His Gln Glu His
       610                 615                 620
   Val Leu Pro Met Ile Pro Ala Gly Gly Gly Phe Met Asp Val Ile Thr
   625                 630                 635                 640
   Glu Gly Asp Gly Arg Met Lys Tyr
                   645
<210> 9
   <211> 2270
   <212> DNA
   <213> Arabidopsis thaliana
<220>
   <221> CDS
   <222> (40)...(2052)
<221> misc_feature
   <222> (0) ... (0)
   <223> GenBank Accession No. NM_114714
<400> 9
   ctctgtttct ctatctcttg ctcatctttc tcctgaacc atg gcg gcg gca aca     54
                                              Met Ala Ala Ala Thr
                                                     1               5
   aca aca aca aca aca tct tct tcg atc tcc ttc tcc acc aaa cca tct     102
   Thr Thr Thr Thr Thr Ser Ser Ser Ile Ser Phe Ser Thr Lys Pro Ser
                   10                  15                  20
   cct tcc tcc tcc aaa tca cca tta cca atc tcc aga ttc tcc ctc cca     150
   Pro Ser Ser Ser Lys Ser Pro Leu Pro Ile Ser Arg Phe Ser Leu Pro
               25                  30                  35
   ttc tcc cta aac ccc aac aaa tca tcc tcc tcc tcc cgc cgc cgc ggt     198
   Phe Ser Leu Asn Pro Asn Lys Ser Ser Ser Ser Ser Arg Arg Arg Gly
           40                  45                  50
   atc aaa tcc agc tct ccc tcc tcc atc tcc gcc gtg ctc aac aca acc     246
   Ile Lys Ser Ser Ser Pro Ser Ser Ile Ser Ala Val Leu Asn Thr Thr
       55                  60                  65
   acc aat gtc aca acc act ccc tct cca acc aaa cct acc aaa ccc gaa     294
   Thr Asn Val Thr Thr Thr Pro Ser Pro Thr Lys Pro Thr Lys Pro Glu
   70                  75                  80                  85
   aca ttc atc tcc cga ttc gct cca gat caa ccc cgc aaa ggc gct gat     342
   Thr Phe Ile Ser Arg Phe Ala Pro Asp Gln Pro Arg Lys Gly Ala Asp
                   90                  95                  100
   atc ctc gtc gaa gct tta gaa cgt caa ggc gta gaa acc gta ttc gct     390
   Ile Leu Val Glu Ala Leu Glu Arg Gln Gly Val Glu Thr Val Phe Ala
               105                 110                 115
   tac cct gga ggt gca tca atg gag att cac caa gcc tta acc cgc tct     438
   Tyr Pro Gly Gly Ala Ser Met Glu Ile His Gln Ala Leu Thr Arg Ser
           120                 125                 130
   tcc tca atc cgt aac gtc ctt cct cgt cac gaa caa gga ggt gta ttc     486
   Ser Ser Ile Arg Asn Val Leu Pro Arg His Glu Gln Gly Gly Val Phe
       135                 140                 145
   gca gca gaa gga tac gct cga tcc tca ggt aaa cca ggt atc tgt ata     534
   Ala Ala Glu Gly Tyr Ala Arg Ser Ser Gly Lys Pro Gly Ile Cys Ile
   150                 155                 160                 165
   gcc act tca ggt ccc gga gct aca aat ctc gtt agc gga tta gcc gat     582
   Ala Thr Ser Gly Pro Gly Ala Thr Asn Leu Val Ser Gly Leu Ala Asp
                   170                 175                 180
   gcg ttg tta gat agt gtt cct ctt gta gca atc aca gga caa gtc cct     630
   Ala Leu Leu Asp Ser Val Pro Leu Val Ala Ile Thr Gly Gln Val Pro
               185                 190                 195
   cgt cgt atg att ggt aca gat gcg ttt caa gag act ccg att gtt gag     678
   Arg Arg Met Ile Gly Thr Asp Ala Phe Gln Glu Thr Pro Ile Val Glu
           200                 205                 210
   gta acg cgt tcg att acg aag cat aac tat ctt gtg atg gat gtt gaa     726
   Val Thr Arg Ser Ile Thr Lys His Asn Tyr Leu Val Met Asp Val Glu
       215                 220                 225
   gat atc cct agg att att gag gaa gct ttc ttt tta gct act tct ggt     774
   Asp Ile Pro Arg Ile Ile Glu Glu Ala Phe Phe Leu Ala Thr Ser Gly
   230                 235                 240                 245
   aga cct gga cct gtt ttg gtt gat gtt cct aaa gat att caa caa cag      822
   Arg Pro Gly Pro Val Leu Val Asp Val Pro Lys Asp Ile Gln Gln Gln
                   250                 255                 260
   ctt gcg att cct aat tgg gaa cag gct atg aga tta cct ggt tat atg      870
   Leu Ala Ile Pro Asn Trp Glu Gln Ala Met Arg Leu Pro Gly Tyr Met
               265                 270                 275
   tct agg atg cct aaa cct ccg gaa gat tct cat ttg gag cag att gtt      918
   Ser Arg Met Pro Lys Pro Pro Glu Asp Ser His Leu Glu Gln Ile Val
           280                 285                 290
   agg ttg att tct gag tct aag aag cct gtg ttg tat gtt ggt ggt ggt      966
   Arg Leu Ile Ser Glu Ser Lys Lys Pro Val Leu Tyr Val Gly Gly Gly
       295                 300                 305
   tgt ttg aat tct agc gat gaa ttg ggt agg ttt gtt gag ctt acg ggg      1014
   Cys Leu Asn Ser Ser Asp Glu Leu Gly Arg Phe Val Glu Leu Thr Gly
   310                 315                 320                 325
   atc cct gtt gcg agt acg ttg atg ggg ctg gga tct tat cct tgt gat      1062
   Ile Pro Val Ala Ser Thr Leu Met Gly Leu Gly Ser Tyr Pro Cys Asp
                   330                 335                 340
   gat gag ttg tcg tta cat atg ctt gga atg cat ggg act gtg tat gca      1110
   Asp Glu Leu Ser Leu His Met Leu Gly Met His Gly Thr Val Tyr Ala
               345                 350                 355
   aat tac gct gtg gag cat agt gat ttg ttg ttg gcg ttt ggg gta agg      1158
   Asn Tyr Ala Val Glu His Ser Asp Leu Leu Leu Ala Phe Gly Val Arg
           360                 365                 370
   ttt gat gat cgt gtc acg ggt aag ctt gag gct ttt gct agt agg gct      1206
   Phe Asp Asp Arg Val Thr Gly Lys Leu Glu Ala Phe Ala Ser Arg Ala
       375                 380                 385
   aag att gtt cat att gat att gac tcg gct gag att ggg aag aat aag      1254
   Lys Ile Val His Ile Asp Ile Asp Ser Ala Glu Ile Gly Lys Asn Lys
   390                 395                 400                 405
   act cct cat gtg tct gtg tgt ggt gat gtt aag ctg gct ttg caa ggg      1302
   Thr Pro His Val Ser Val Cys Gly Asp Val Lys Leu Ala Leu Gln Gly
                   410                 415                 420
   atg aat aag gtt ctt gag aac cga gcg gag gag ctt aag ctt gat ttt      1350
   Met Asn Lys Val Leu Glu Asn Arg Ala Glu Glu Leu Lys Leu Asp Phe
               425                 430                 435
   gga gtt tgg agg aat gag ttg aac gta cag aaa cag aag ttt ccg ttg      1398
   Gly Val Trp Arg Asn Glu Leu Asn Val Gln Lys Gln Lys Phe Pro Leu
           440                 445                 450
   agc ttt aag acg ttt ggg gaa gct att cct cca cag tat gcg att aag      1446
   Ser Phe Lys Thr Phe Gly Glu Ala Ile Pro Pro Gln Tyr Ala Ile Lys
       455                 460                 465
   gtc ctt gat gag ttg act gat gga aaa gcc ata ata agt act ggt gtc      1494
   Val Leu Asp Glu Leu Thr Asp Gly Lys Ala Ile Ile Ser Thr Gly Val
   470                 475                 480                 485
   ggg caa cat caa atg tgg gcg gcg cag ttc tac aat tac aag aaa cca    1542
   Gly Gln His Gln Met Trp Ala Ala Gln Phe Tyr Asn Tyr Lys Lys Pro
                   490                 495                 500
   agg cag tgg cta tca tca gga ggc ctt gga gct atg gga ttt gga ctt    1590
   Arg Gln Trp Leu Ser Ser Gly Gly Leu Gly Ala Met Gly Phe Gly Leu
               505                 510                 515
   cct gct gcg att gga gcg tct gtt gct aac cct gat gcg ata gtt gtg    1638
   Pro Ala Ala Ile Gly Ala Ser Val Ala Asn Pro Asp Ala Ile Val Val
           520                 525                 530
   gat att gac gga gat gga agc ttt ata atg aat gtg caa gag cta gcc    1686
   Asp Ile Asp Gly Asp Gly Ser Phe Ile Met Asn Val Gln Glu Leu Ala
       535                 540                 545
   act att cgt gta gag aat ctt cca gtg aag gta ctt tta tta aac aac    1734
   Thr Ile Arg Val Glu Asn Leu Pro Val Lys Val Leu Leu Leu Asn Asn
   550                 555                 560                 565
   cag cat ctt ggc atg gtt atg caa tgg gaa gat cgg ttc tac aaa gct    1782
   Gln His Leu Gly Met Val Met Gln Trp Glu Asp Arg Phe Tyr Lys Ala
                   570                 575                 580
   aac cga gct cac aca ttt ctc ggg gat ccg gct cag gag gac gag ata    1830
   Asn Arg Ala His Thr Phe Leu Gly Asp Pro Ala Gln Glu Asp Glu Ile
               585                 590                 595
   ttc ccg aac atg ttg ctg ttt gca gca gct tgc ggg att cca gcg gcg    1878
   Phe Pro Asn Met Leu Leu Phe Ala Ala Ala Cys Gly Ile Pro Ala Ala
           600                 605                 610
   agg gtg aca aag aaa gca gat ctc cga gaa gct att cag aca atg ctg    1926
   Arg Val Thr Lys Lys Ala Asp Leu Arg Glu Ala Ile Gln Thr Met Leu
       615                 620                 625
   gat aca cca gga cct tac ctg ttg gat gtg att tgt ccg cac caa gaa    1974
   Asp Thr Pro Gly Pro Tyr Leu Leu Asp Val Ile Cys Pro His Gln Glu
   630                 635                 640                 645
   cat gtg ttg ccg atg atc ccg agt ggt ggc act ttc aac gat gtc ata    2022
   His Val Leu Pro Met Ile Pro Ser Gly Gly Thr Phe Asn Asp Val Ile
                   650                 655                 660
   acg gaa gga gat ggc cgg att aaa tac tga gagatgaaac cggtgattat      2072
   Thr Glu Gly Asp Gly Arg Ile Lys Tyr  *
               665                 670
   cagaaccttt tatggtcttt gtatgcatat ggtaaaaaaa cttagtttgc aatttcctgt 2132
   ttgttttggt aatttgagtt tcttttagtt gttgatctgc ctgctttttg gtttacgtca 2192
   gactactact gctgttgttg tttggtttcc tttctttcat tttataaata aataatccgg 2252
   ttcggtttac tccttgtg                                               2270
<210> 10
   <211> 670
   <212> PRT
   <213> Nicotiana tabacum
<400> 10
   Met Ala Ala Ala Thr Thr Thr Thr Thr Thr Ser Ser Ser Ile Ser Phe
   1               5                   10                  15
   Ser Thr Lys Pro Ser Pro Ser Ser Ser Lys Ser Pro Leu Pro Ile Ser
               20                  25                  30
   Arg Phe Ser Leu Pro Phe Ser Leu Asn Pro Asn Lys Ser Ser Ser Ser
           35                  40                  45
   Ser Arg Arg Arg Gly Ile Lys Ser Ser Ser Pro Ser Ser Ile Ser Ala
       50                  55                  60
   Val Leu Asn Thr Thr Thr Asn Val Thr Thr Thr Pro Ser Pro Thr Lys
   65                  70                  75                  80
   Pro Thr Lys Pro Glu Thr Phe Ile Ser Arg Phe Ala Pro Asp Gln Pro
                   85                  90                  95
   Arg Lys Gly Ala Asp Ile Leu Val Glu Ala Leu Glu Arg Gln Gly Val
               100                 105                 110
   Glu Thr Val Phe Ala Tyr Pro Gly Gly Ala Ser Met Glu Ile His Gln
           115                 120                 125
   Ala Leu Thr Arg Ser Ser Ser Ile Arg Asn Val Leu Pro Arg His Glu
       130                 135                 140
   Gln Gly Gly Val Phe Ala Ala Glu Gly Tyr Ala Arg Ser Ser Gly Lys
   145                 150                 155                 160
   Pro Gly Ile Cys Ile Ala Thr Ser Gly Pro Gly Ala Thr Asn Leu Val
                   165                 170                 175
   Ser Gly Leu Ala Asp Ala Leu Leu Asp Ser Val Pro Leu Val Ala Ile
               180                 185                 190
   Thr Gly Gln Val Pro Arg Arg Met Ile Gly Thr Asp Ala Phe Gln Glu
           195                 200                 205
   Thr Pro Ile Val Glu Val Thr Arg Ser Ile Thr Lys His Asn Tyr Leu
       210                 215                 220
   Val Met Asp Val Glu Asp Ile Pro Arg Ile Ile Glu Glu Ala Phe Phe
   225                 230                 235                 240
   Leu Ala Thr Ser Gly Arg Pro Gly Pro Val Leu Val Asp Val Pro Lys
                   245                 250                 255
   Asp Ile Gln Gln Gln Leu Ala Ile Pro Asn Trp Glu Gln Ala Met Arg
               260                 265                 270
   Leu Pro Gly Tyr Met Ser Arg Met Pro Lys Pro Pro Glu Asp Ser His
           275                 280                 285
   Leu Glu Gln Ile Val Arg Leu Ile Ser Glu Ser Lys Lys Pro Val Leu
       290                 295                 300
   Tyr Val Gly Gly Gly Cys Leu Asn Ser Ser Asp Glu Leu Gly Arg Phe
   305                 310                 315                 320
   Val Glu Leu Thr Gly Ile Pro Val Ala Ser Thr Leu Met Gly Leu Gly
                   325                 330                 335
   Ser Tyr Pro Cys Asp Asp Glu Leu Ser Leu His Met Leu Gly Met His
               340                 345                 350
   Gly Thr Val Tyr Ala Asn Tyr Ala Val Glu His Ser Asp Leu Leu Leu
           355                 360                 365
   Ala Phe Gly Val Arg Phe Asp Asp Arg Val Thr Gly Lys Leu Glu Ala
       370                 375                 380
   Phe Ala Ser Arg Ala Lys Ile Val His Ile Asp Ile Asp Ser Ala Glu
   385                 390                 395                 400
   Ile Gly Lys Asn Lys Thr Pro His Val Ser Val Cys Gly Asp Val Lys
                   405                 410                 415
   Leu Ala Leu Gln Gly Met Asn Lys Val Leu Glu Asn Arg Ala Glu Glu
               420                 425                 430
   Leu Lys Leu Asp Phe Gly Val Trp Arg Asn Glu Leu Asn Val Gln Lys
           435                 440                 445
   Gln Lys Phe Pro Leu Ser Phe Lys Thr Phe Gly Glu Ala Ile Pro Pro
       450                 455                 460
   Gln Tyr Ala Ile Lys Val Leu Asp Glu Leu Thr Asp Gly Lys Ala Ile
   465                 470                 475                 480
   Ile Ser Thr Gly Val Gly Gln His Gln Met Trp Ala Ala Gln Phe Tyr
                   485                 490                 495
   Asn Tyr Lys Lys Pro Arg Gln Trp Leu Ser Ser Gly Gly Leu Gly Ala
                   500                 505                 510
   Met Gly Phe Gly Leu Pro Ala Ala Ile Gly Ala Ser Val Ala Asn Pro
               515                 520                 525
   Asp Ala Ile Val Val Asp Ile Asp Gly Asp Gly Ser Phe Ile Met Asn
       530                 535                 540
   Val Gln Glu Leu Ala Thr Ile Arg Val Glu Asn Leu Pro Val Lys Val
   545                 550                 555                 560
   Leu Leu Leu Asn Asn Gln His Leu Gly Met Val Met Gln Trp Glu Asp
                   565                 570                 575
   Arg Phe Tyr Lys Ala Asn Arg Ala His Thr Phe Leu Gly Asp Pro Ala
               580                 585                 590
   Gln Glu Asp Glu Ile Phe Pro Asn Met Leu Leu Phe Ala Ala Ala Cys
           595                 600                 605
   Gly Ile Pro Ala Ala Arg Val Thr Lys Lys Ala Asp Leu Arg Glu Ala
       610                 615                 620
   Ile Gln Thr Met Leu Asp Thr Pro Gly Pro Tyr Leu Leu Asp Val Ile
   625                 630                 635                 640
   Cys Pro His Gln Glu His Val Leu Pro Met Ile Pro Ser Gly Gly Thr
                   645                 650                 655
   Phe Asn Asp Val Ile Thr Glu Gly Asp Gly Arg Ile Lys Tyr
               660                 665                 670
<210> 11
   <211> 20
   <212> DNA
   <213> Artificial Sequence
<220>
   <223> PCR Primer C2359-FI
<400> 11
   agatcaaccg agaaaaggcg 20
<210> 12
   <211> 22
   <212> DNA
   <213> Artificial Sequence
<220>
   <223> PCR Primer ALSRI
<400> 12
   cgaaaaatca agattagtca cc 22
<210> 13
   <211> 21
   <212> DNA
   <213> Artificial Sequence
<220>
   <223> PCR Primer SUNALS1-F1
<400> 13
   gaggaaaaga attcggtgac t 21
<210> 14
   <211> 20
   <212> DNA
   <213> Artificial Sequence
<220>
   <223> PCR Primer ALS1-8R
<400> 14
   cggtgatcac atccgagaaa 20
<210> 15
   <211> 20
   <212> DNA
   <213> Artificial Sequence
<220>
   <223> PCR Primer ALS-2R
<400> 15
   catcggttcc gatcattctc 20
<210> 16
   <211> 19
   <212> DNA
   <213> Artificial Sequence
<220>
   <223> PCR Primer ALS-2F
<400> 16
   gtgtcaccga cgtcttcgc 19
<210> 17
   <211> 22
   <212> DNA
   <213> Artificial Sequence
<220>
   <223> PCR Primer ALS-3R
<400> 17
   ctagacaaat aacccggtaa cc 22
<210> 18
   <211> 19
   <212> DNA
   <213> Artificial Sequence
<220>
   <223> PCR Primer ALS-3F
<400> 18
   gcgctgttag acagtgtcc 19
<210> 19
   <211> 19
   <212> DNA
   <213> Artificial Sequence
<220>
   <223> PCR Primer ALS-4F
<400> 19
   acagcaacag ttagtggtg 19
<210> 20
   <211> 20
   <212> DNA
   <213> Artificial Sequence
<220>
   <223> PCR Primer ALS-6R
<400> 20
   cggcagattt tcaacacgga 20
<210> 21
   <211> 19
   <212> DNA
   <213> Artificial Sequence
<220>
   <223> PCR Primer ALS-7F
<400> 21
   agacaatggc tgacgtcgg 19
<210> 22
   <211> 20
   <212> DNA
   <213> Artificial Sequence
<220>
   <223> PCR Primer ALS-8F
<400> 22
   tatggtggtt cagtgggagg 20
<210> 23
   <211> 1968
   <212> DNA
   <213> Helianthus annus
<220>
   <221> CDS
   <222> (1) ... (1965)
<221> misc_feature
   <222> (0) ... (0)
   <223> GenBank Accession No. AY541451
<400> 23
   atg gcg gct cct ccc aac cct tcc atc tcc ttc aaa cca ccg tca ccc    48
   Met Ala Ala Pro Pro Asn Pro Ser Ile Ser Phe Lys Pro Pro Ser Pro
    1               5                  10                  15
   gcc gcc gca ctg cca cca cgc tcc gcc ttc ctc ccc cgt ttc gca tta    96
   Ala Ala Ala Leu Pro Pro Arg Ser Ala Phe Leu Pro Arg Phe Ala Leu
               20                  25                  30
   ccc atc act tcc act acc caa aaa cga cac cgt ctt cac atc tcc aat   144
   Pro Ile Thr Ser Thr Thr Gln Lys Arg His Arg Leu His Ile Ser Asn
           35                  40                  45
   gtt ctc tcc gac tcc aaa tcc acc acc acc acc acc acc acc act caa   192
   Val Leu Ser Asp Ser Lys Ser Thr Thr Thr Thr Thr Thr Thr Thr Gln
       50                  55                  60
   cga ccg tta ccg gtg cag cct ttt gtc tcc cgt tac gcg cca gat caa   240
   Arg Pro Leu Pro Val Gln Pro Phe Val Ser Arg Tyr Ala Pro Asp Gln
    65                  70                  75                  80
   ccg aga aaa ggc gca gac gtg ttg gtg gaa gct ctg gaa cgg gaa ggt   288
   Pro Arg Lys Gly Ala Asp Val Leu Val Glu Ala Leu Glu Arg Glu Gly
                   85                  90                  95
   gtc acc gac gtc ttc gcc tac ccc ggc ggc gcg tca atg gag atc cac   336
   Val Thr Asp Val Phe Ala Tyr Pro Gly Gly Ala Ser Met Glu Ile His
               100                 105                 110
   caa gct ctc acg cgc tca agc act atc cgc aat gtg ctc ccc cgt cac   384
   Gln Ala Leu Thr Arg Ser Ser Thr Ile Arg Asn Val Leu Pro Arg His
           115                 120                 125
   gaa cag ggc ggc gtg ttc gcc gcc gaa ggc tac gcg cgc gcc tcc ggt   432
   Glu Gln Gly Gly Val Phe Ala Ala Glu Gly Tyr Ala Arg Ala Ser Gly
       130                 135                 140
   ctt ccc ggc gtg tgt atc gcc act tcc ggt ccc gga gct acg aac cta   480
   Leu Pro Gly Val Cys Ile Ala Thr Ser Gly Pro Gly Ala Thr Asn Leu
   145                 150                 155                 160
   gtt agt ggt ctt gct gac gcg ctg tta gac agt gtc ccc atg gtg gca   528
   Val Ser Gly Leu Ala Asp Ala Leu Leu Asp Ser Val Pro Met Val Ala
                   165                 170                 175
   atc acc ggt caa gtt ccc cgg aga atg atc gga acc gat gcg ttt caa   576
   Ile Thr Gly Gln Val Pro Arg Arg Met Ile Gly Thr Asp Ala Phe Gln
               180                 185                 190
   gaa acc cca att gtt gag gta aca cgt tcg atc act aaa cat aat tat   624
   Glu Thr Pro Ile Val Glu Val Thr Arg Ser Ile Thr Lys His Asn Tyr
           195                 200                 205
   ctt gtg ttg gat gtt gag gat att ccc aga att gtt cgt gag gct ttt   672
   Leu Val Leu Asp Val Glu Asp Ile Pro Arg Ile Val Arg Glu Ala Phe
       210                 215                 220
   tat ctt gcg agt tcg ggt cga ccc ggc ccg gtt ttg ata gat gta ccg   720
   Tyr Leu Ala Ser Ser Gly Arg Pro Gly Pro Val Leu Ile Asp Val Pro
   225                 230                 235                 240
   aaa gat ata cag caa cag tta gtg gtg ccg aaa tgg gat gaa ccg atg     768
   Lys Asp Ile Gln Gln Gln Leu Val Val Pro Lys Trp Asp Glu Pro Met
                   245                 250                 255
   agg tta ccg ggt tat ttg tct aga atg ccg aag cct caa tat gat ggg     816
   Arg Leu Pro Gly Tyr Leu Ser Arg Met Pro Lys Pro Gln Tyr Asp Gly
               260                 265                 270
   cat ttg gaa cag att gtt agg ttg gtg ggg gaa gcg aag agg ccg gtt     864
   His Leu Glu Gln Ile Val Arg Leu Val Gly Glu Ala Lys Arg Pro Val
           275                 280                 285
   ttg tat gtg ggt ggt ggg tgt ttg aat tcg gat gat gag ttg agg cgg     912
   Leu Tyr Val Gly Gly Gly Cys Leu Asn Ser Asp Asp Glu Leu Arg Arg
       290                 295                 300
   ttt gtg gag ctt acg ggg att ccg gtt gcg agt act ttg atg ggg ctc     960
   Phe Val Glu Leu Thr Gly Ile Pro Val Ala Ser Thr Leu Met Gly Leu
   305                 310                 315                 320
   gga gcg tac cct gct tcg agt gat ttg tcg ctt cat atg ctt ggg atg     1008
   Gly Ala Tyr Pro Ala Ser Ser Asp Leu Ser Leu His Met Leu Gly Met
                   325                 330                 335
   cat ggt acg gtt tat gcg aat tat gcg gtt gat aag agt gat ttg ttg     1056
   His Gly Thr Val Tyr Ala Asn Tyr Ala Val Asp Lys Ser Asp Leu Leu
               340                 345                 350
   ctt gcg ttt ggg gtg cgg ttt gat gat cgt gtg acg ggg aag ctt gag     1104
   Leu Ala Phe Gly Val Arg Phe Asp Asp Arg Val Thr Gly Lys Leu Glu
           355                 360                 365
   gcg ttt gct agt agg gcg aag att gtt cat att gat att gat cct gct     1152
   Ala Phe Ala Ser Arg Ala Lys Ile Val His Ile Asp Ile Asp Pro Ala
       370                 375                 380
   gaa att ggg aag aat aag cag cct cat gtg tcg att tgt ggt gat att     1200
   Glu Ile Gly Lys Asn Lys Gln Pro His Val Ser Ile Cys Gly Asp Ile
   385                 390                 395                 400
   aag gtc gcg tta cag ggt ttg aac aag att ttg gag gaa aag aat tcg     1248
   Lys Val Ala Leu Gln Gly Leu Asn Lys Ile Leu Glu Glu Lys Asn Ser
                   405                 410                 415
   gtg act aat ctt gat ttt tcg acc tgg aga aag gaa ttg gat gaa caa     1296
   Val Thr Asn Leu Asp Phe Ser Thr Trp Arg Lys Glu Leu Asp Glu Gln
               420                 425                 430
   aaa atg aag ttc ccg ttg agc ttt aaa acg ttt ggc gaa gcg att cct     1344
   Lys Met Lys Phe Pro Leu Ser Phe Lys Thr Phe Gly Glu Ala Ile Pro
           435                 440                 445
   cca cag tat gct att caa gtt ctt gat gag tta acg ggc ggg aat gca     1392
   Pro Gln Tyr Ala Ile Gln Val Leu Asp Glu Leu Thr Gly Gly Asn Ala
       450                 455                 460
   att att agc acc ggt gtc ggg caa cat cag atg tgg gct gct cag ttt     1440
   Ile Ile Ser Thr Gly Val Gly Gln His Gln Met Trp Ala Ala Gln Phe
   465                 470                 475                 480
   tac aaa tac aac aaa cct aga caa tgg ctg acg tcg ggc ggg cta ggg     1488
   Tyr Lys Tyr Asn Lys Pro Arg Gln Trp Leu Thr Ser Gly Gly Leu Gly
                   485                 490                 495
   gca atg ggt ttc ggc ctg ccc gct gct atc ggg gcg gcc gtt gca aga     1536
   Ala Met Gly Phe Gly Leu Pro Ala Ala Ile Gly Ala Ala Val Ala Arg
               500                 505                 510
   cct gat gcg gta gta gtt gac atc gac ggt gac gga agc ttt atg atg     1584
   Pro Asp Ala Val Val Val Asp Ile Asp Gly Asp Gly Ser Phe Met Met
           515                 520                 525
   aat gtt caa gag tta gcc aca atc cgt gtt gaa aat ctg ccg gtt aag     1632
   Asn Val Gln Glu Leu Ala Thr Ile Arg Val Glu Asn Leu Pro Val Lys
       530                 535                 540
   att tta tta ctt aac aac cag cat ttg ggt atg gtg gtt cag tgg gag     1680
   Ile Leu Leu Leu Asn Asn Gln His Leu Gly Met Val Val Gln Trp Glu
   545                 550                 555                 560
   gat cgg ttt tac aag gcg aat cgg gct cat acc tac tta gga aac ccg     1728
   Asp Arg Phe Tyr Lys Ala Asn Arg Ala His Thr Tyr Leu Gly Asn Pro
                   565                 570                 575
   tca aaa gag tcg gaa ata ttc cct aac atg gtg aag ttt gct gaa gcc     1776
   Ser Lys Glu Ser Glu Ile Phe Pro Asn Met Val Lys Phe Ala Glu Ala
               580                 585                 590
   tgt gat atc ccg gct gct cga gtg acc caa aag gcg gat cta cga gca     1824
   Cys Asp Ile Pro Ala Ala Arg Val Thr Gln Lys Ala Asp Leu Arg Ala
           595                 600                 605
   gct att cag aag atg ttg gat aca ccc ggg cct tac ttg ttg gat gtg     1872
   Ala Ile Gln Lys Met Leu Asp Thr Pro Gly Pro Tyr Leu Leu Asp Val
       610                 615                 620
   att gtg ccg cat caa gaa cac gtg ttg ccc atg atc ccg gct ggc gga     1920
   Ile Val Pro His Gln Glu His Val Leu Pro Met Ile Pro Ala Gly Gly
   625                 630                 635                 640
   ggt ttc tcg gat gtg atc acc gag ggt gat ggc aga acg aaa tat         1965
   Gly Phe Ser Asp Val Ile Thr Glu Gly Asp Gly Arg Thr Lys Tyr
                   645                 650                 655
   tga                                                                 1968
<210> 24
   <211> 655
   <212> PRT
   <213> Helianthus annus
<400> 24
   Met Ala Ala Pro Pro Asn Pro Ser Ile Ser Phe Lys Pro Pro Ser Pro
    1               5                  10                  15
   Ala Ala Ala Leu Pro Pro Arg Ser Ala Phe Leu Pro Arg Phe Ala Leu
               20                  25                  30
   Pro Ile Thr Ser Thr Thr Gln Lys Arg His Arg Leu His Ile Ser Asn
           35                  40                  45
   Val Leu Ser Asp Ser Lys Ser Thr Thr Thr Thr Thr Thr Thr Thr Gln
       50                  55                  60
   Arg Pro Leu Pro Val Gln Pro Phe Val Ser Arg Tyr Ala Pro Asp Gln
   65                  70                  75                  80
   Pro Arg Lys Gly Ala Asp Val Leu Val Glu Ala Leu Glu Arg Glu Gly
                   85                  90                  95
   Val Thr Asp Val Phe Ala Tyr Pro Gly Gly Ala Ser Met Glu Ile His
               100                 105                 110
   Gln Ala Leu Thr Arg Ser Ser Thr Ile Arg Asn Val Leu Pro Arg His
           115                 120                 125
   Glu Gln Gly Gly Val Phe Ala Ala Glu Gly Tyr Ala Arg Ala Ser Gly
       130                 135                 140
   Leu Pro Gly Val Cys Ile Ala Thr Ser Gly Pro Gly Ala Thr Asn Leu
   145                 150                 155                 160
   Val Ser Gly Leu Ala Asp Ala Leu Leu Asp Ser Val Pro Met Val Ala
                   165                 170                 175
   Ile Thr Gly Gln Val Pro Arg Arg Met Ile Gly Thr Asp Ala Phe Gln
               180                 185                 190
   Glu Thr Pro Ile Val Glu Val Thr Arg Ser Ile Thr Lys His Asn Tyr
           195                 200                 205
   Leu Val Leu Asp Val Glu Asp Ile Pro Arg Ile Val Arg Glu Ala Phe
       210                 215                 220
   Tyr Leu Ala Ser Ser Gly Arg Pro Gly Pro Val Leu Ile Asp Val Pro
   225                 230                 235                 240
   Lys Asp Ile Gln Gln Gln Leu Val Val Pro Lys Trp Asp Glu Pro Met
                   245                 250                 255
   Arg Leu Pro Gly Tyr Leu Ser Arg Met Pro Lys Pro Gln Tyr Asp Gly
               260                 265                 270
   His Leu Glu Gln Ile Val Arg Leu Val Gly Glu Ala Lys Arg Pro Val
           275                 280                 285
   Leu Tyr Val Gly Gly Gly Cys Leu Asn Ser Asp Asp Glu Leu Arg Arg
       290                 295                 300
   Phe Val Glu Leu Thr Gly Ile Pro Val Ala Ser Thr Leu Met Gly Leu
   305                 310                 315                 320
   Gly Ala Tyr Pro Ala Ser Ser Asp Leu Ser Leu His Met Leu Gly Met
                   325                 330                 335
   His Gly Thr Val Tyr Ala Asn Tyr Ala Val Asp Lys Ser Asp Leu Leu
               340                 345                 350
   Leu Ala Phe Gly Val Arg Phe Asp Asp Arg Val Thr Gly Lys Leu Glu
           355                 360                 365
   Ala Phe Ala Ser Arg Ala Lys Ile Val His Ile Asp Ile Asp Pro Ala
       370                 375                 380
   Glu Ile Gly Lys Asn Lys Gln Pro His Val Ser Ile Cys Gly Asp Ile
   385                 390                 395                 400
   Lys Val Ala Leu Gln Gly Leu Asn Lys Ile Leu Glu Glu Lys Asn Ser
                   405                 410                 415
   Val Thr Asn Leu Asp Phe Ser Thr Trp Arg Lys Glu Leu Asp Glu Gln
               420                 425                 430
   Lys Met Lys Phe Pro Leu Ser Phe Lys Thr Phe Gly Glu Ala Ile Pro
           435                 440                 445
   Pro Gln Tyr Ala Ile Gln Val Leu Asp Glu Leu Thr Gly Gly Asn Ala
       450                 455                 460
   Ile Ile Ser Thr Gly Val Gly Gln His Gln Met Trp Ala Ala Gln Phe
   465                 470                 475                 480
   Tyr Lys Tyr Asn Lys Pro Arg Gln Trp Leu Thr Ser Gly Gly Leu Gly
                   485                 490                 495
   Ala Met Gly Phe Gly Leu Pro Ala Ala Ile Gly Ala Ala Val Ala Arg
               500                 505                 510
   Pro Asp Ala Val Val Val Asp Ile Asp Gly Asp Gly Ser Phe Met Met
           515                 520                 525
   Asn Val Gln Glu Leu Ala Thr Ile Arg Val Glu Asn Leu Pro Val Lys
       530                 535                 540
   Ile Leu Leu Leu Asn Asn Gln His Leu Gly Met Val Val Gln Trp Glu
   545                 550                 555                 560
   Asp Arg Phe Tyr Lys Ala Asn Arg Ala His Thr Tyr Leu Gly Asn Pro
                   565                 570                 575
   Ser Lys Glu Ser Glu Ile Phe Pro Asn Met Val Lys Phe Ala Glu Ala
               580                 585                 590
   Cys Asp Ile Pro Ala Ala Arg Val Thr Gln Lys Ala Asp Leu Arg Ala
           595                 600                 605
   Ala Ile Gln Lys Met Leu Asp Thr Pro Gly Pro Tyr Leu Leu Asp Val
       610                 615                 620
   Ile Val Pro His Gln Glu His Val Leu Pro Met Ile Pro Ala Gly Gly
   625                 630                 635                 640
   Gly Phe Ser Asp Val Ile Thr Glu Gly Asp Gly Arg Thr Lys Tyr
                   645                 650                 655
<210> 25
   <211> 2664
   <212> DNA
   <213> Zea mays
<220>
   <221> CDS
   <222> (666)...(2582)
<221> misc_feature
   <222> (0) ... (0)
   <223> GenBank Accession number X63553
<400> 25
   gatccccgat gggatttatc tccgtgggga acggggatgg agaagaaatg tctctcgcaa 60
   gagttcgtgg gaatccccgc gggaaatttt tttgtcgcgg ggacagatac ggatacggcg 120
   agccaaaggg tgccatccct aacaccgaca ccgggcctga cgctgtcgcc gcagctgctc 180
   atcttgtggt tgtgggctgt gcccggtttc cctgttgcgg attgcgggtg gcagcctggc 240
   aggtgggtgc gacccgtttg gattcccttg tctgggcccc ttgtgtcagt accgtctgta 300
   ctccgatgac atgcaccgtc gtccacagtc aagtccaaaa tctcccctct tttttaacgg 360
   aacagttcaa aacctccttg acgcacgctg tcgtgtacca gcactcggtg gacaccacgt 420
   ttgtaatgca ggccgacacg tcggtcccac gtcgacaggc cccaccgtcc ggtctgtagc 480
   gtgtacgtat tcgggcgacg gacgtgtcgt cgtcgtcttg cgagtcccat tcccatcacc 540
   atctgagcca cacatcctct gaacaaaagc agggaggcct ctacgcacat cccccttgct 600
   cccactccgt gtccgtggca cccaccccaa accctcgcgc cgcctccgag acagccgccg 660
   caacc atg gcc acc gcc gcc gcc gcg tct acc gcg ctc act ggc gcc act 710
         Met Ala Thr Ala Ala Ala Ala Ser Thr Ala Leu Thr Gly Ala Thr
         1               5                   10                  15
   acc gct gcg ccc aag gcg agg cgc cgg gcg cac ctc ctg gcc acc cgc   758
   Thr Ala Ala Pro Lys Ala Arg Arg Arg Ala His Leu Leu Ala Thr Arg
                   20                  25                  30
   cgc gcc ctc gcc gcg ccc atc agg tgc tca gcg gcg tca ccc gcc atg   806
   Arg Ala Leu Ala Ala Pro Ile Arg Cys Ser Ala Ala Ser Pro Ala Met
               35                  40                  45
   ccg atg gct ccc ccg gcc acc ccg ctc cgg ccg tgg ggc ccc acc gat   854
   Pro Met Ala Pro Pro Ala Thr Pro Leu Arg Pro Trp Gly Pro Thr Asp
           50                  55                  60
   ccc cgc aag ggc gcc gac atc ctc gtc gag tcc ctc gag cgc tgc ggc   902
   Pro Arg Lys Gly Ala Asp Ile Leu Val Glu Ser Leu Glu Arg Cys Gly
       65                  70                  75
   gtc cgc gac gtc ttc gcc tac ccc ggc ggc gcg tcc atg gag atc cac    950
   Val Arg Asp Val Phe Ala Tyr Pro Gly Gly Ala Ser Met Glu Ile His
    80                 85                  90                  95
   cag gca ctc acc cgc tcc ccc gtc atc gcc aac cac ctc ttc cgc cac    998
   Gln Ala Leu Thr Arg Ser Pro Val Ile Ala Asn His Leu Phe Arg His
                   100                 105                 110
   gag caa ggg gag gcc ttt gcg gcc tcc ggc tac gcg cgc tcc tcg ggc   1046
   Glu Gln Gly Glu Ala Phe Ala Ala Ser Gly Tyr Ala Arg Ser Ser Gly
               115                 120                 125
   cgc gtc ggc gtc tgc atc gcc acc tcc ggc ccc ggc gcc acc aac ctt   1094
   Arg Val Gly Val Cys Ile Ala Thr Ser Gly Pro Gly Ala Thr Asn Leu
           130                 135                 140
   gtc tcc gcg ctc gcc gac gcg ctg ctc gat tcc gtc ccc atg gtc gcc   1142
   Val Ser Ala Leu Ala Asp Ala Leu Leu Asp Ser Val Pro Met Val Ala
       145                 150                 155
   atc acg gga cag gtg ccg cga cgc atg att ggc acc gac gcc ttc cag   1190
   Ile Thr Gly Gln Val Pro Arg Arg Met Ile Gly Thr Asp Ala Phe Gln
   160                 165                 170                 175
   gag acg ccc atc gtc gag gtc acc cgc tcc atc acc aag cac aac tac   1238
   Glu Thr Pro Ile Val Glu Val Thr Arg Ser Ile Thr Lys His Asn Tyr
                   180                 185                 190
   ctg gtc ctc gac gtc gac gac atc ccc cgc gtc gtg cag gag gct ttc   1286
   Leu Val Leu Asp Val Asp Asp Ile Pro Arg Val Val Gln Glu Ala Phe
               195                 200                 205
   ttc ctc gcc tcc tct ggt cga ccg ggg ccg gtg ctt gtc gac atc ccc   1334
   Phe Leu Ala Ser Ser Gly Arg Pro Gly Pro Val Leu Val Asp Ile Pro
           210                 215                 220
   aag gac atc cag cag cag atg gcg gtg cct gtc tgg gac aag ccc atg   1382
   Lys Asp Ile Gln Gln Gln Met Ala Val Pro Val Trp Asp Lys Pro Met
       225                 230                 235
   agt ctg cct ggg tac att gcg cgc ctt ccc aag ccc cct gcg act gag   1430
   Ser Leu Pro Gly Tyr Ile Ala Arg Leu Pro Lys Pro Pro Ala Thr Glu
   240                 245                 250                 255
   ttg ctt gag cag gtg ctg cgt ctt gtt ggt gaa tcc cgg cgc cct gtt   1478
   Leu Leu Glu Gln Val Leu Arg Leu Val Gly Glu Ser Arg Arg Pro Val
                   260                 265                 270
   ctt tat gtt ggc ggt ggc tgc gca gca tct ggt gag gag ttg cga cgc   1526
   Leu Tyr Val Gly Gly Gly Cys Ala Ala Ser Gly Glu Glu Leu Arg Arg
               275                 280                 285
   ttt gtg gag ctg act gga atc ccg gtc aca act act ctt atg ggc ctc   1574
   Phe Val Glu Leu Thr Gly Ile Pro Val Thr Thr Thr Leu Met Gly Leu
           290                 295                 300
   ggc aac ttc ccc agc gac gac cca ctg tct ctg cgc atg cta ggt atg   1622
   Gly Asn Phe Pro Ser Asp Asp Pro Leu Ser Leu Arg Met Leu Gly Met
       305                 310                 315
   cat ggc acg gtg tat gca aat tat gca gtg gat aag gcc gat ctg ttg   1670
   His Gly Thr Val Tyr Ala Asn Tyr Ala Val Asp Lys Ala Asp Leu Leu
   320                 325                 330                 335
   ctt gca ctt ggt gtg cgg ttt gat gat cgt gtg aca ggg aag att gag     1718
   Leu Ala Leu Gly Val Arg Phe Asp Asp Arg Val Thr Gly Lys Ile Glu
                   340                 345                 350
   gct ttt gca agc agg gct aag att gtg cac gtt gat att gat ccg gct     1766
   Ala Phe Ala Ser Arg Ala Lys Ile Val His Val Asp Ile Asp Pro Ala
               355                 360                 365
   gag att ggc aag aac aag cag cca cat gtg tcc atc tgt gca gat gtt     1814
   Glu Ile Gly Lys Asn Lys Gln Pro His Val Ser Ile Cys Ala Asp Val
           370                 375                 380
   aag ctt gct ttg cag ggc atg aat gct ctt ctt gaa gga agc aca tca     1862
   Lys Leu Ala Leu Gln Gly Met Asn Ala Leu Leu Glu Gly Ser Thr Ser
       385                 390                 395
   aag aag agc ttt gac ttt ggc tca tgg aac gat gag ttg gat cag cag     1910
   Lys Lys Ser Phe Asp Phe Gly Ser Trp Asn Asp Glu Leu Asp Gln Gln
   400                 405                 410                 415
   aag agg gaa ttc ccc ctt ggg tat aaa aca tct aat gag gag atc cag     1958
   Lys Arg Glu Phe Pro Leu Gly Tyr Lys Thr Ser Asn Glu Glu Ile Gln
                   420                 425                 430
   cca caa tat gct att cag gtt ctt gat gag ctg acg aaa ggc gag gcc     2006
   Pro Gln Tyr Ala Ile Gln Val Leu Asp Glu Leu Thr Lys Gly Glu Ala
               435                 440                 445
   atc atc ggc aca ggt gtt ggg cag cac cag atg tgg gcg gca cag tac     2054
   Ile Ile Gly Thr Gly Val Gly Gln His Gln Met Trp Ala Ala Gln Tyr
           450                 455                 460
   tac act tac aag cgg cca agg cag tgg ttg tct tca gct ggt ctt ggg     2102
   Tyr Thr Tyr Lys Arg Pro Arg Gln Trp Leu Ser Ser Ala Gly Leu Gly
       465                 470                 475
   gct atg gga ttt ggt ttg ccg gct gct gct ggt gct tct gtg gcc aac     2150
   Ala Met Gly Phe Gly Leu Pro Ala Ala Ala Gly Ala Ser Val Ala Asn
   480                 485                 490                 495
   cca ggt gtt act gtt gtt gac atc gat gga gat ggt agc ttt ctc atg     2198
   Pro Gly Val Thr Val Val Asp Ile Asp Gly Asp Gly Ser Phe Leu Met
                   500                 505                 510
   aac gtt cag gag cta gct atg atc cga att gag aac ctc ccg gtg aag     2246
   Asn Val Gln Glu Leu Ala Met Ile Arg Ile Glu Asn Leu Pro Val Lys
               515                 520                 525
   gtc ttt gtg cta aac aac cag cac ctg ggg atg gtg gtg cag tgg gag     2294
   Val Phe Val Leu Asn Asn Gln His Leu Gly Met Val Val Gln Trp Glu
           530                 535                 540
   gac agg ttc tat aag gcc aac aga gcg cac aca tac ttg gga aac cca     2342
   Asp Arg Phe Tyr Lys Ala Asn Arg Ala His Thr Tyr Leu Gly Asn Pro
       545                 550                 555
   gag aat gaa agt gag ata tat cca gat ttc gtg acg atc gcc aaa ggg     2390
   Glu Asn Glu Ser Glu Ile Tyr Pro Asp Phe Val Thr Ile Ala Lys Gly
   560                 565                 570                 575
   ttc aac att cca gcg gtc cgt gtg aca aag aag aac gaa gtc cgc gca    2438
   Phe Asn Ile Pro Ala Val Arg Val Thr Lys Lys Asn Glu Val Arg Ala
                   580                 585                 590
   gcg ata aag aag atg ctc gag act cca ggg ccg tac ctc ttg gat ata    2486
   Ala Ile Lys Lys Met Leu Glu Thr Pro Gly Pro Tyr Leu Leu Asp Ile
               595                 600                 605
   atc gtc cca cac cag gag cat gtg ttg cct atg atc cct agt ggt ggg    2534
   Ile Val Pro His Gln Glu His Val Leu Pro Met Ile Pro Ser Gly Gly
           610                 615                 620
   gct ttc aag gat atg atc ctg gat ggt gat ggc agg act gtg tac tga    2582
   Ala Phe Lys Asp Met Ile Leu Asp Gly Asp Gly Arg Thr Val Tyr  *
       625                 630                 635
   tctaaaatcc agcaagcaac tgatctaaaa tccagcaagc accgcctccc tgctagtaca 2642
   aggtgatatg tttatctgtg tg                                          2664
<210> 26
   <211> 638
   <212> PRT
   <213> Zea mays
<400> 26
   Met Ala Thr Ala Ala Ala Ala Ser Thr Ala Leu Thr Gly Ala Thr Thr
    1               5                  10                  15
   Ala Ala Pro Lys Ala Arg Arg Arg Ala His Leu Leu Ala Thr Arg Arg
               20                  25                  30
   Ala Leu Ala Ala Pro Ile Arg Cys Ser Ala Ala Ser Pro Ala Met Pro
           35                  40                  45
   Met Ala Pro Pro Ala Thr Pro Leu Arg Pro Trp Gly Pro Thr Asp Pro
       50                  55                  60
   Arg Lys Gly Ala Asp Ile Leu Val Glu Ser Leu Glu Arg Cys Gly Val
   65                  70                  75                  80
   Arg Asp Val Phe Ala Tyr Pro Gly Gly Ala Ser Met Glu Ile His Gln
                   85                  90                  95
   Ala Leu Thr Arg Ser Pro Val Ile Ala Asn His Leu Phe Arg His Glu
               100                 105                 110
   Gln Gly Glu Ala Phe Ala Ala Ser Gly Tyr Ala Arg Ser Ser Gly Arg
           115                 120                 125
   Val Gly Val Cys Ile Ala Thr Ser Gly Pro Gly Ala Thr Asn Leu Val
       130                 135                 140
   Ser Ala Leu Ala Asp Ala Leu Leu Asp Ser Val Pro Met Val Ala Ile
   145                 150                 155                 160
   Thr Gly Gln Val Pro Arg Arg Met Ile Gly Thr Asp Ala Phe Gln Glu
                   165                 170                 175
   Thr Pro Ile Val Glu Val Thr Arg Ser Ile Thr Lys His Asn Tyr Leu
               180                 185                 190
   Val Leu Asp Val Asp Asp Ile Pro Arg Val Val Gln Glu Ala Phe Phe
           195                 200                 205
   Leu Ala Ser Ser Gly Arg Pro Gly Pro Val Leu Val Asp Ile Pro Lys
       210                 215                 220
   Asp Ile Gln Gln Gln Met Ala Val Pro Val Trp Asp Lys Pro Met Ser
   225                 230                 235                 240
   Leu Pro Gly Tyr Ile Ala Arg Leu Pro Lys Pro Pro Ala Thr Glu Leu
                   245                 250                 255
   Leu Glu Gln Val Leu Arg Leu Val Gly Glu Ser Arg Arg Pro Val Leu
               260                 265                 270
   Tyr Val Gly Gly Gly Cys Ala Ala Ser Gly Glu Glu Leu Arg Arg Phe
           275                 280                 285
   Val Glu Leu Thr Gly Ile Pro Val Thr Thr Thr Leu Met Gly Leu Gly
       290                 295                 300
   Asn Phe Pro Ser Asp Asp Pro Leu Ser Leu Arg Met Leu Gly Met His
   305                 310                 315                 320
   Gly Thr Val Tyr Ala Asn Tyr Ala Val Asp Lys Ala Asp Leu Leu Leu
                   325                 330                 335
   Ala Leu Gly Val Arg Phe Asp Asp Arg Val Thr Gly Lys Ile Glu Ala
               340                 345                 350
   Phe Ala Ser Arg Ala Lys Ile Val His Val Asp Ile Asp Pro Ala Glu
           355                 360                 365
   Ile Gly Lys Asn Lys Gln Pro His Val Ser Ile Cys Ala Asp Val Lys
       370                 375                 380
   Leu Ala Leu Gln Gly Met Asn Ala Leu Leu Glu Gly Ser Thr Ser Lys
   385                 390                 395                 400
   Lys Ser Phe Asp Phe Gly Ser Trp Asn Asp Glu Leu Asp Gln Gln Lys
                   405                 410                 415
   Arg Glu Phe Pro Leu Gly Tyr Lys Thr Ser Asn Glu Glu Ile Gln Pro
               420                 425                 430
   Gln Tyr Ala Ile Gln Val Leu Asp Glu Leu Thr Lys Gly Glu Ala Ile
           435                 440                 445
   Ile Gly Thr Gly Val Gly Gln His Gln Met Trp Ala Ala Gln Tyr Tyr
       450                 455                 460
   Thr Tyr Lys Arg Pro Arg Gln Trp Leu Ser Ser Ala Gly Leu Gly Ala
   465                 470                 475                 480
   Met Gly Phe Gly Leu Pro Ala Ala Ala Gly Ala Ser Val Ala Asn Pro
                   485                 490                 495
   Gly Val Thr Val Val Asp Ile Asp Gly Asp Gly Ser Phe Leu Met Asn
               500                 505                 510
   Val Gln Glu Leu Ala Met Ile Arg Ile Glu Asn Leu Pro Val Lys Val
           515                 520                 525
   Phe Val Leu Asn Asn Gln His Leu Gly Met Val Val Gln Trp Glu Asp
       530                 535                 540
   Arg Phe Tyr Lys Ala Asn Arg Ala His Thr Tyr Leu Gly Asn Pro Glu
   545                 550                 555                 560
   Asn Glu Ser Glu Ile Tyr Pro Asp Phe Val Thr Ile Ala Lys Gly Phe
                   565                 570                 575
   Asn Ile Pro Ala Val Arg Val Thr Lys Lys Asn Glu Val Arg Ala Ala
               580                 585                 590
   Ile Lys Lys Met Leu Glu Thr Pro Gly Pro Tyr Leu Leu Asp Ile Ile
           595                 600                 605
   Val Pro His Gln Glu His Val Leu Pro Met Ile Pro Ser Gly Gly Ala
       610                 615                 620
   Phe Lys Asp Met Ile Leu Asp Gly Asp Gly Arg Thr Val Tyr
   625                 630                 635